# EUROPEAN PATENT APPLICATION

(11) **EP 4 488 272 A1**
(43) Date of publication of application: **08.01.2025**
(21) Application number: 23759238.1
(22) Date of filing: 23.02.2023
(51) Int. Cl.: C07D 403/12, C07D 401/12, C07D 401/14, C07D 407/14, C07D 487/00, A61K 31/397, A61K 31/4035, A61K 31/506, A61P 35/00, A61P 35/02, A61P 35/04

(54) **COMPOUND AS FAK INHIBITOR AND USE THEREOF**

(30) Priority: 28.02.2022 CN 202210190680; 06.07.2022 CN 202210800802
(71) Applicant: Wigen Biomedicine Technology (Shanghai) Co., Ltd., Shanghai 201203 (CN)
(72) Inventor: XIE, Yuli, Shanghai 201203 (CN); LIU, Wenzhong, Shanghai 201203 (CN); QIAN, Lihui, Shanghai 201203 (CN)
(74) Representative: Willett, Christopher David
(86) International application number: PCT/CN2023/077893
(87) International publication number: WO 2023/160614

(57) **Abstract**

Disclosed in the present invention are a compound as a FAK inhibitor and use thereof. Specifically, the present invention relates to a compound of general formula (1), a preparation method therefor, and use of the compound of general formula (1) and an isomer thereof, a crystalline form thereof, a pharmaceutically acceptable salt thereof, a hydrate thereof, or a solvate thereof as a FAK inhibitor. The compound and the isomer thereof, the crystalline form thereof, the pharmaceutically acceptable salt thereof, the hydrate thereof, or the solvate thereof of the present invention can be used for preparing a medicament for treating or preventing a related disease mediated by FAK.

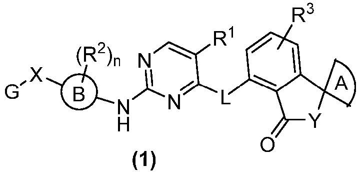

## Description

The present application claims priority to Chinese Patent Application No. 202210190680.2 filed on February 28, 2022 and Chinese Patent Application No. 202210800802.5 filed on July 6, 2022, which are incorporated herein by reference in their entirety.

### TECHNICAL FIELD

The present invention relates to the field of pharmaceutical chemistry, and particularly relates to a class of compounds with an inhibitory effect on a focal adhesion kinase (FAK kinase), a pharmaceutically acceptable salt thereof, and a pharmaceutical composition thereof, as well as use of the compound, the pharmaceutically acceptable salt thereof, and the pharmaceutical composition thereof in the treatment or prevention of a related disease mediated by FAK.

### BACKGROUND

Focal adhesion kinase (FAK) is a member of the non-receptor tyrosine kinase family, located at the cell-to-cell junction. After the extracellular matrix (ECM) binds to integrin receptors on the cell membrane surface, FAK is activated, thereby up-regulating the downstream signaling pathway of the integrin receptors. Autophosphorylation of FAK Tyr397 is a biomarker of FAK kinase activity. Studies have shown that FAK plays an important role in cell survival, growth, adhesion, migration, and invasion (McLean et al., 2005, Nat Rev cancer, 5: 505-515). The expression and activity of FAK are up-regulated by cancer cells, thereby promoting the proliferation and invasion of the cancer cells and increasing tumor metastasis *in vivo.*

The mRNA level of FAK is highly expressed in about 37% of ovarian cancer cells and 26% of breast cancer cells. FAK inhibitors inhibit the proliferation and invasion of tumor cells. When the function of FAK kinase is hindered, the metastasis of breast cancer is greatly reduced. In addition to promoting the function of cancer cells, FAK mediates the activation of various signaling downstream of angiogenic factors, and is involved in the proliferation, migration, and differentiation of vascular endothelial cells. The specific absence of FAK in the vascular endothelial cells indicates that FAK maintains vascular stability during development. Thus, FAK inhibitors can be used for antagonizing pathological angiogenesis. Therefore, FAK inhibitors can directly and indirectly antagonize the development and progression of tumors by inhibiting the function of tumor cells and anti-angiogenesis, thereby being used for treating tumors. In addition to cancer, FAK inhibitors can also be used in non-oncological indications associated with pathological vascular proliferation such as retinal diseases.

Currently, no FAK inhibitor drug is available on the market. A series of pyrimidine compounds serving as FAK inhibitors were reported in the patent WO2010058032A2, wherein BI853520, as a highly selective FAK inhibitor, is in clinical phase I at present.

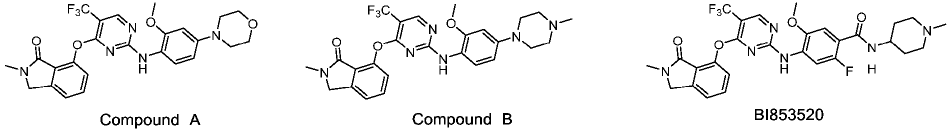

### SUMMARY

The present invention provides a compound of general formula (1) or an isomer thereof, a crystalline form thereof, a pharmaceutically acceptable salt thereof, a hydrate thereof, or a solvate thereof: 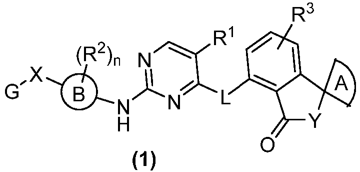 wherein in general formula (1):
ring A is (C3-C6) cycloalkyl or (3-6 membered) heterocycloalkyl, wherein the (C3-C6) cycloalkyl or (3-6 membered) heterocycloalkyl may be each independently and optionally substituted with 1, 2, 3, or 4 R^{a};
Y is -O-, -NR^{a}-, or -CR^{a}R^{b}-;
L is -CH₂-, -O-, or -S-;
ring B is selected from (3-18 membered) heterocycloalkyl, (C3-C18) cycloalkyl, (6-18 membered) aryl, or (5-18 membered) heteroaryl, wherein the (3-18 membered) heterocycloalkyl,
(C3-C18) cycloalkyl, (6-18 membered) aryl, or (5-18 membered) heteroaryl may be each independently and optionally substituted with n R²;
X is a chemical bond, 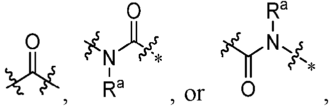 with * representing attachment to ring B;
G is -H, -D, halogen, hydroxy, amino, cyano, nitro, -OR^{a}, -NR^{a}R^{b}, -C(O)R^{a}, -CO₂R^{a}, -CONR^{a}R^{b}, (C1-C8) alkyl, (C1-C8) alkoxy, (C1-C8) haloalkyl, (C2-C8) alkenyl, (C2-C8) alkynyl, (C3-C15) cycloalkyl, (3-15 membered) heterocycloalkyl, (C6-C14) aryl, or (5-14 membered) heteroaryl, wherein the (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, or (5-14 membered) heteroaryl may be each independently and optionally substituted with 1, 2, 3, or 4 R^{c};
R¹ is -H, -D, halogen, hydroxy, amino, cyano, nitro, -OR^{a}, -NR^{a}R^{b}, -C(O)R^{a}, -CO₂R^{a}, -CONR^{a}R^{b}, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, or (5-14 membered) heteroaryl, wherein the (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, or (5-14 membered) heteroaryl may be each independently and optionally substituted with 1, 2, 3, or 4 R^{c};
R² is -H, -D, halogen, hydroxy, amino, cyano, nitro, -OR^{a}, -NR^{a}R^{b}, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, or (5-14 membered) heteroaryl, wherein the (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, or (5-14 membered) heteroaryl may be each independently and optionally substituted with 1, 2, 3, or 4 R^{c}; or when 2 R² are attached to the same atom, the 2 R² may form one oxo group;
R³ is -H, -D, halogen, hydroxy, amino, cyano, nitro, -OR^{3a}, -NR^{3a}R^{3b}, -C(O)R^{3a}, -CO₂R^{3a}, - S(O)ₚR^{3a}, -S(O)ₚNR^{3a}R^{3b}, -CONR^{3a}R^{3b}, -C(=NR^{3a})-NR^{3b}R^{3c}, -NR^{3a}COR^{3b}, -NR^{3a}CONR^{3b}R^{3c}, - NR^{3a}CO₂R^{3b}, -NR^{3a}S(O)ₚNR^{3b}R^{3c}, -NR^{3a}S(O)ₚR^{3b}, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C1-C8) alkoxy, -(C1-C8) alkylene-(C3-C14) cycloalkyl, -(C1-C8) alkylene-(3-14 membered) heterocycloalkyl, -(C1-C8) alkylene-(C6-C14) aryl, or -(C1-C8) alkylene-(5-14 membered) heteroaryl;
R^{a} and R^{b} are each independently -H, -D, halogen, hydroxy, amino, cyano, nitro, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C3-C14) cycloalkyl, -(C1-C8) alkylene-(3-14 membered) heterocycloalkyl, -(C1-C8) alkylene-(C6-C14) aryl, or -(C1-C8) alkylene-(5-14 membered) heteroaryl, wherein the (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C3-C14) cycloalkyl, -(C1-C8) alkylene-(3-14 membered) heterocycloalkyl, -(C1-C8) alkylene-(C6-C14) aryl, or -(C1-C8) alkylene-(5-14 membered) heteroaryl may be each independently and optionally substituted with 1, 2, 3, or 4 R^{c}; or R^{a} and R^{b}, together with the atom to which they are attached, form one (C3-C6) cycloalkyl or (3-6 membered) heterocycloalkyl group, and the (C3-C6) cycloalkyl or (3-6 membered) heterocycloalkyl group may be each independently and optionally substituted with 1, 2, 3, or 4 R^{c};
R^{c} is -H, -D, halogen, hydroxy, amino, cyano, nitro, -OR^{xa}, -NR^{xa}R^{xb}, -(CH₂)ₘOR^{xa}, - (CH₂)ₘNR^{xa}R^{xb}, -C(O)R^{xa}, -CO₂R^{xa}, -(CH₂)ₘS (O)ₚR^{xa}, -S(O)ₚNR^{xa}R^{xb}, -(CH₂)ₘCONR^{xa}R^{xb}, - C(=NR^{xa})-NR^{xb}R^{xc}, -NR^{xa}COR^{xb}, -NR^{xa}CONR^{xb}R^{xc}, -NR^{xa}CO₂R^{xb}, -NR^{xa}S(O)ₚNR^{xb}R^{xc}, - NR^{xa}S(O)ₚR^{xb}, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C1-C8) alkoxy, -(C1-C8) alkylene-(C3-C14)cycloalkyl, -(C1-C8) alkylene-(3-14 membered) heterocycloalkyl, -(C1-C8) alkylene-(C6-C14) aryl, or -(C1-C8) alkylene-(5-14 membered) heteroaryl, wherein the (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, or (5-14 membered) heteroaryl may be each independently and optionally substituted with 1, 2, 3, or 4 R^{3c}; or when 2 R^{c} are attached to the same atom, the 2 R^{c} may form one oxo group;
R^{xa}, R^{xb}, and R^{xc} are each independently -H, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C3-C14) cycloalkyl, -(C1-C8) alkylene-(3-14 membered) heterocycloalkyl, -(C1-C8) alkylene-(C6-C14) aryl, or -(C1-C8) alkylene-(5-14 membered) heteroaryl;
R^{3a}, R^{3b}, and R^{3c} are each independently -H, -D, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C3-C14) cycloalkyl, -(C1-C8) alkylene-(3-14 membered) heterocycloalkyl, -(C1-C8) alkylene-(C6-C14) aryl, or -(C1-C8) alkylene-(5-14 membered) heteroaryl;
p is an integer of 0, 1, or 2;
m is an integer of 0, 1, 2, or 3;
n is an integer of 0, 1, 2, or 3.

In another preferred embodiment, in general formula (1), L is -CH₂- or -O-.

In another preferred embodiment, in general formula (1), ring A is (C3-C6) cycloalkyl or (3-6 membered) heterocycloalkyl, wherein the (C3-C6) cycloalkyl or (3-6 membered) heterocycloalkyl may be each independently and optionally substituted with 1, 2, 3, or 4 -H, -D, -F, -Cl, -Br, -I, -OH, -NH₂, -CN, -NO₂, -OCH₃, -OCH₂CH₃, -OCF₃, -CH₃, -CD₃, -CH₂CH₃, - CH₂F, -CHF₂, -CF₃, -(CH₂)₂OCH₃, -CH₂SO₂CH₃, -CH₂CONH₂, -CH₂CON(CH₃)₂, 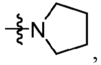 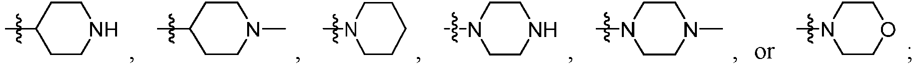 the substituent is preferably -H, -D, -F, -OCH₃, -OCF₃, -CH₃, -CD₃, -CH₂F, -CHF₂, or -CF₃; the substituent is more preferably -H, -D, or -F; the substituent is more preferably -H; the substituent is more preferably -D; the substituent is more preferably -F.

In another preferred embodiment, in general formula (1), ring A is preferably 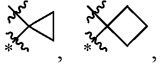 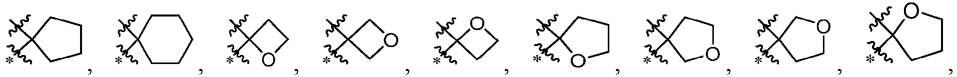 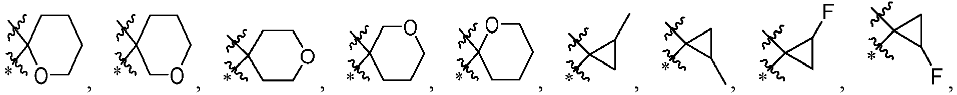 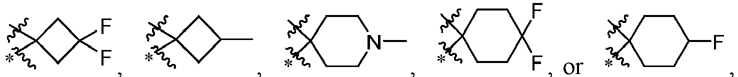 wherein * represents a ring attached to Y; A is more preferably 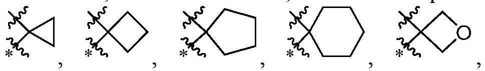 or 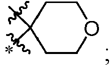 A is more preferably 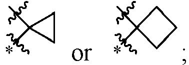 A is more preferably 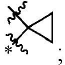 A is more preferably

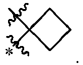

In another preferred embodiment, in general formula (1), ring B is selected from (6-12 membered) aryl or (5-14 membered) heteroaryl, wherein the (6-12 membered) aryl or (5-14 membered) heteroaryl may be optionally substituted with n R².

In another preferred embodiment, in general formula (1), ring B is 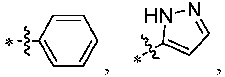 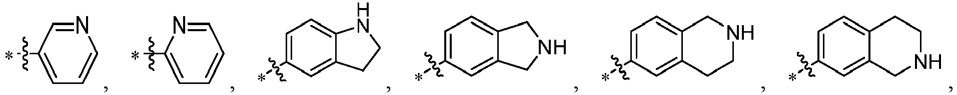 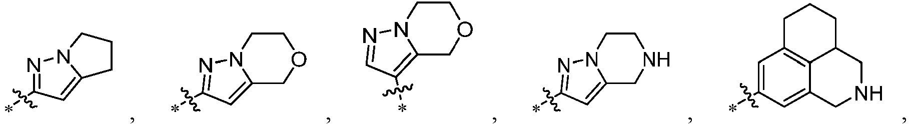 or 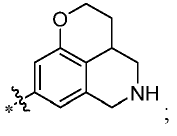 ring B is preferably 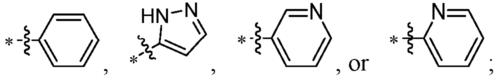 ring B is more preferably 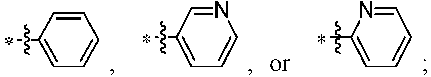 ring B is more preferably 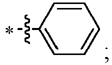 wherein "*" represents attachment to

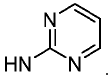

In another preferred embodiment, in general formula (1), G is -H, -D, (C1-C6) alkyl, (C1-C6) alkoxy, (C1-C6) haloalkyl, or (5-15 membered) heterocycloalkyl, wherein the (C1-C6) alkyl, (C1-C6) alkoxy, (C1-C6) haloalkyl, or (5-15 membered) heterocycloalkyl may be optionally substituted with 1, 2, 3, or 4 -H, -D, -F, -Cl, -Br, -I, -OH, -NH₂, -CN, -NO₂, -OCH₃, -NHCH₃, - N(CH₃)₂, -C(O)NH₂, -OCH₂CH₃, -OCF₃, -CH₃, -CD₃, -CH₂CH₃, -CH₂F, -CHF₂, -CF₃, - (CH₂)₂OCH₃, -CH₂SO₂CH₃, -CH₂CONH₂, -CH₂CON(CH₃)₂, 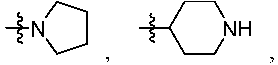 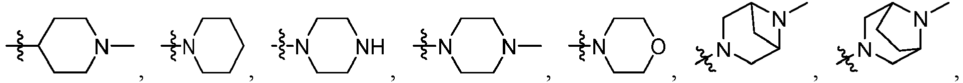 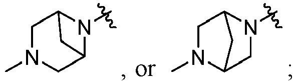 or two substituents attached to the same atom may form one oxo group.

In another preferred embodiment, in general formula (1), G is -H, (C1-C3) alkyl, or (6-12 membered) heterocycloalkyl, wherein the (C1-C3) alkyl or (6-12 membered) heterocycloalkyl may be optionally substituted with 1, 2, 3, or 4 -H, -D, -F, -CH₃, -CD₃, -CH₂CH₃, -OCH₃, - N(CH₃)₂, -(CH₂)₂OCH₃, -CH₂SO₂CH₃, -CH₂CONH₂, -CH₂CON(CH₃)₂, 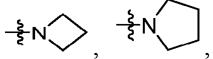 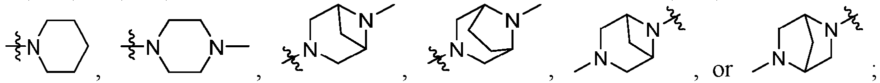 or two substituents attached to the same atom may form one oxo group.

In another preferred embodiment, in general formula (1), G is selected from -H, -CH₃, -CD₃, - CH₂CH₃, -CH₂F, -CHF₂, -CF₃, -CH₂N(CH₃)₂, 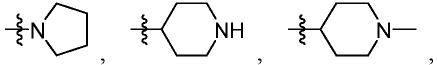 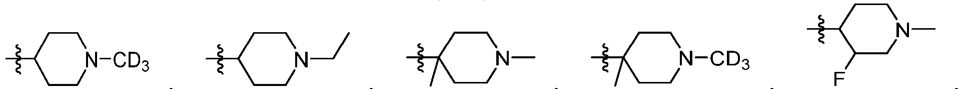 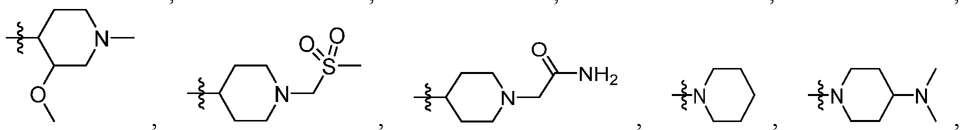 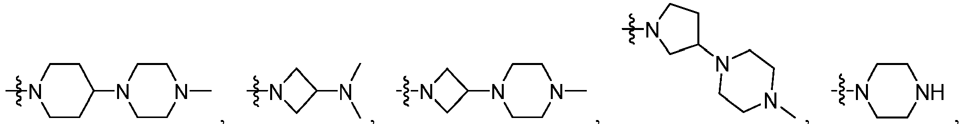 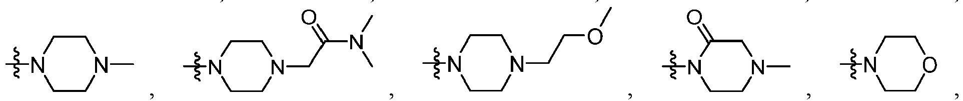 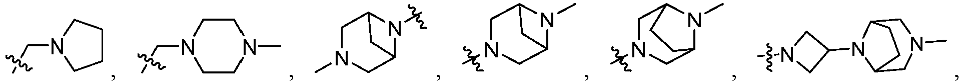 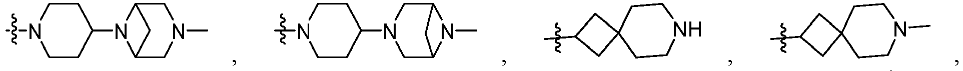 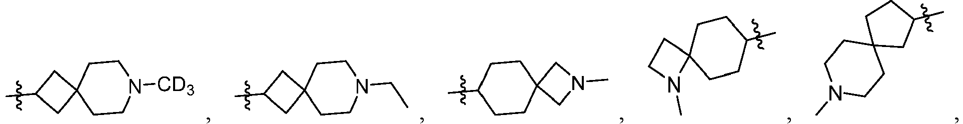 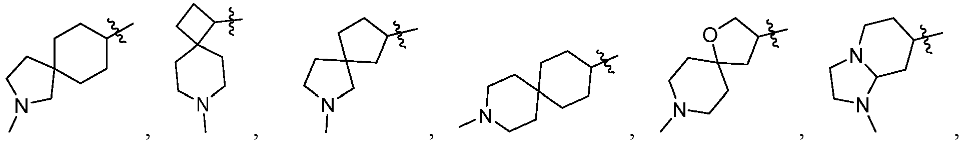 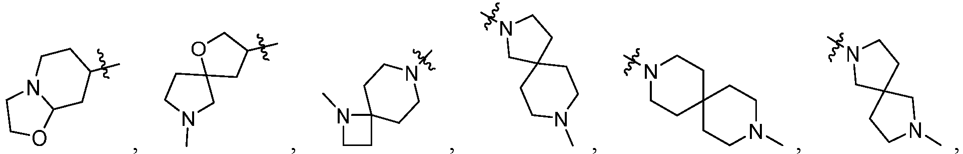 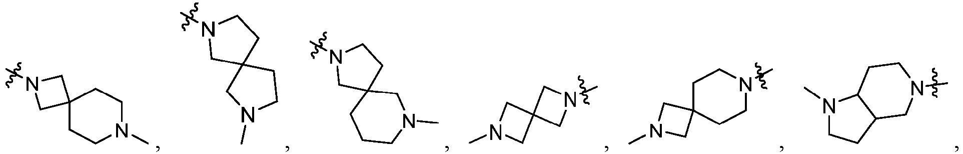 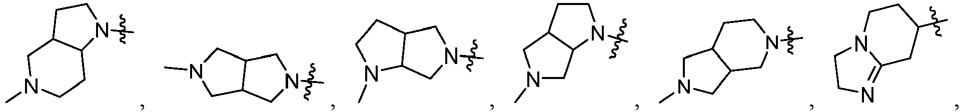 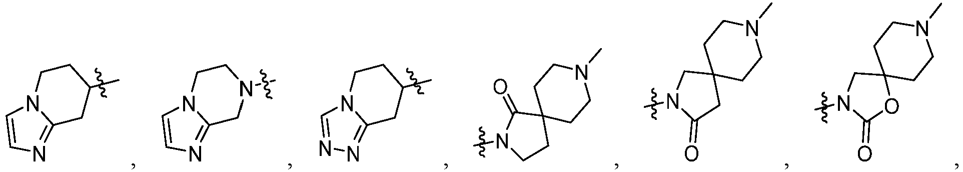 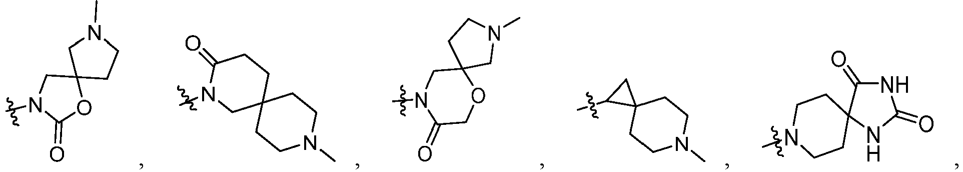 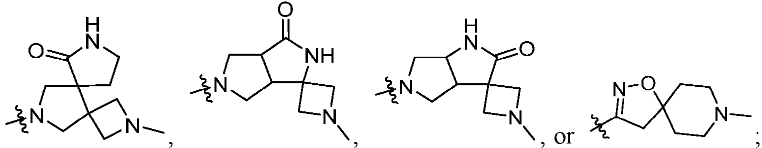 G is preferably -H,

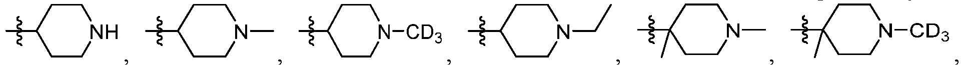

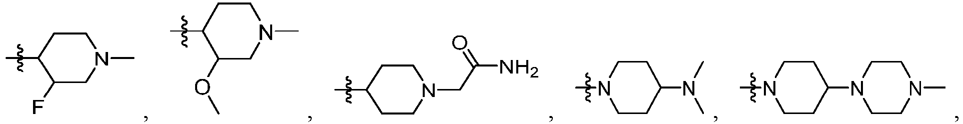

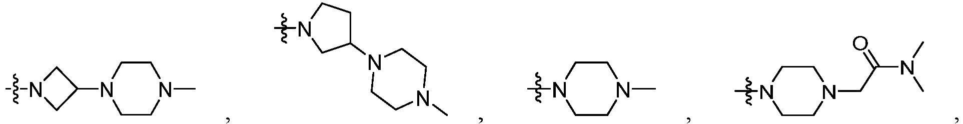

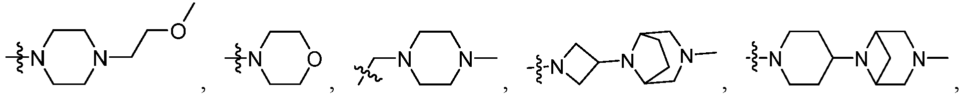

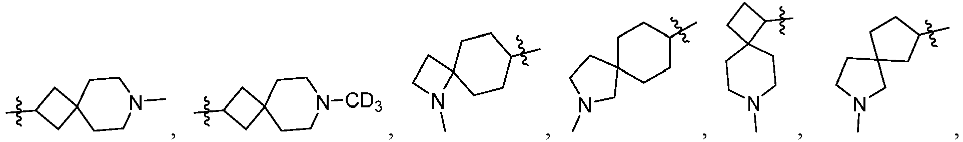

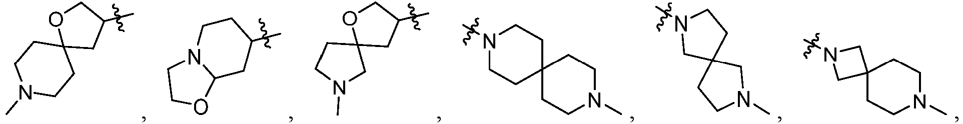

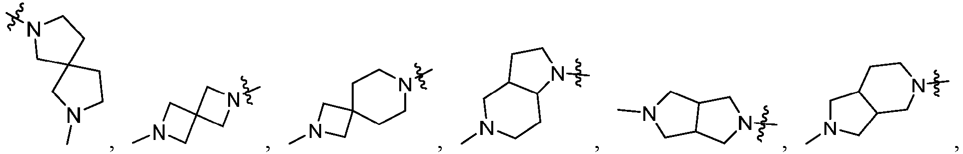

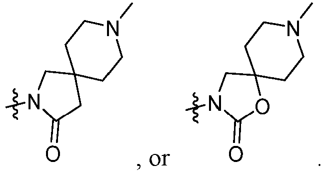

In another preferred embodiment, in general formula (1), X is selected from a chemical bond, 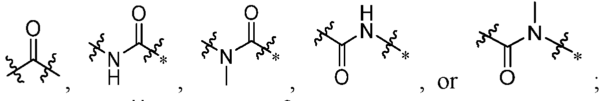 X is preferably a chemical bond, 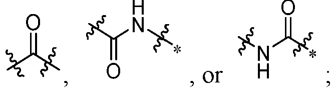 X is more preferably a chemical bond, 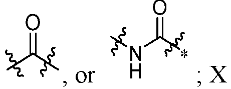 is more preferably 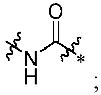 X is more preferably 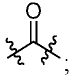 X is more preferably a chemical bond; wherein * represents attachment to ring B.

In another preferred embodiment, in general formula (1), R¹ is selected from -H, -D, -F, -Cl, -Br, -I, -OH, -NH₂, -CN, -NO₂, -OCH₃, -NHCH₃, -N(CH₃)₂, -C(O)NH₂, -OCH₂CH₃, -OCF₃, -CH₃, - CD₃, -CH₂CH₃, -CH₂F, -CHF₂, CF₃, or 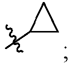 R¹ is preferably -F, -Cl, -Br, -CN, -NO₂, -CF₃, - C(O)NH₂, or 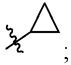 R¹ is preferably -F, -Cl, -Br, -CN, or -CF₃; R¹ is more preferably -F; R¹ is more preferably -Cl; R¹ is more preferably -Br; R¹ is more preferably -CN; R¹ is more preferably -CF₃.

In another preferred embodiment, in general formula (1), R² is -H, -D, -F, -Cl, -Br, -I, -OH, - NH₂, -CN, -NO₂, -OCF₃, -NHCH₃, -N(CH₃)₂, -NHCH₂CH₃, -N(CH₂CH₃)₂, (C1-C3) alkyl, (C1-C3) alkoxy, (C1-C3) haloalkyl, (C2-C4) alkenyl, (C2-C4) alkynyl, (C3-C6) cycloalkyl, (3-6 membered) heterocycloalkyl, (C6-C10) aryl, or (5-10 membered) heteroaryl, wherein the (C1-C3) alkyl, (C1-C3) alkoxy, (C1-C3) haloalkyl, (C2-C4) alkenyl, (C2-C4) alkynyl, (C3-C6) cycloalkyl, (3-6 membered) heterocycloalkyl, (C6-C10) aryl, or (5-10 membered) heteroaryl may be each independently and optionally substituted with 1, 2, 3, or 4 -H, -D, -F, -Cl, -Br, -I, - OH, -NH₂, -CN, -NO₂, -OCH₃, -NHCH₃, -N(CH₃)₂, -C(O)NH₂, -OCH₂CH₃, -OCF₃, -CH₃, -CD₃, -CH₂CH₃, -CH₂F, -CHF₂, or -CF₃; or when 2 R² are attached to the same atom, the 2 R² may form one oxo group.

In another preferred embodiment, in general formula (1), R² is selected from -H, -D, -F, -Cl, -Br, -I, -OH, -NH₂, -CN, -NO₂, -OCH₃, -NHCH₃, -N(CH₃)₂, -C(O)NH₂, -OCH₂CH₃, -OCF₃, -CH₃, - CD₃ -CH₂CH₃, -CH(CH₃)₂, -CH₂F, -CHF₂, -CF₃, 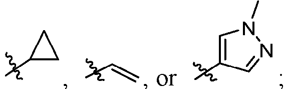 R² is preferably -H, -D, -F, -Cl, -OH, -OCH₃, -OCF₃, -CH₃, -CD₃, -CH₂CH₃, -CH(CH₃)₂, -CF₃, 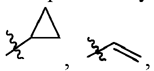 or 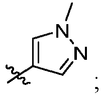 R² is more preferably -H, -F, -Cl, -OH, -OCH₃, -OCF₃, -CH₂CH₃, -CH(CH₃)₂,

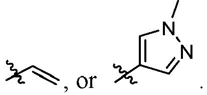

In another preferred embodiment, in general formula (1), R³ is selected from -H, -D, -F, -Cl, -Br, -I, -OH, -NH₂, -CN, -NO₂, -OCH₃, -NHCH₃, -N(CH₃)₂, -C(O)NH₂, -OCH₂CH₃, -OCF₃, -CH₃, - CD₃, -CH₂CH₃, -CH₂F, CHF₂, or -CF₃; R³ is preferably selected from -H, -D, -F, -CH₃, -CD₃, or -CF₃; R³ is more preferably selected from -H, -D, or -F; R³ is more preferably selected from -H; R³ is more preferably selected from -D; R³ is more preferably selected from -F.

In some embodiments of the present invention, the present invention provides a compound of general formula (2) or an isomer thereof, a crystalline form thereof, a pharmaceutically acceptable salt thereof, a hydrate thereof, or a solvate thereof: 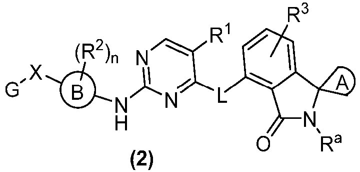 wherein A, L, B, X, G, R¹, R², n, and R³ are as defined above; R^{a} is selected from H, D, (C1-C3) alkyl, (C1-C3) haloalkyl, (C2-C4) alkenyl, (C2-C4) alkynyl, and (C3-C6) cycloalkyl, and is exemplified in the detailed description.

In some embodiments of the present invention, the present invention provides a compound of general formula (3) or an isomer thereof, a crystalline form thereof, a pharmaceutically acceptable salt thereof, a hydrate thereof, or a solvate thereof: 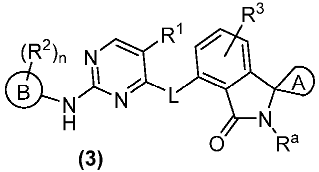 wherein A, L, B, R¹, R², n, and R³ are as defined above; R^{a} is selected from H, D, (C1-C3) alkyl, (C1-C3) haloalkyl, (C2-C4) alkenyl, (C2-C4) alkynyl, (C3-C6) cycloalkyl, and is exemplified in the detailed description.

In some embodiments of the present invention, the present invention provides a compound of general formula (4) or an isomer thereof, a crystalline form thereof, a pharmaceutically acceptable salt thereof, a hydrate thereof, or a solvate thereof: 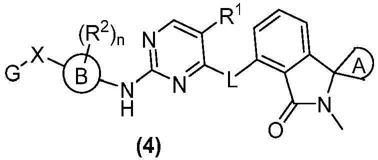 wherein A, L, B, X, G, R¹, and R² are as defined above, and are exemplified in the detailed description.

In some embodiments of the present invention, the present invention provides a compound of general formula (5) or an isomer thereof, a crystalline form thereof, a pharmaceutically acceptable salt thereof, a hydrate thereof, or a solvate thereof: 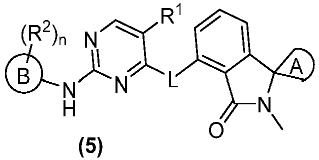 wherein A, L, B, R¹, R², and n are as defined above, and are exemplified in the detailed description.

In some embodiments of the present invention, the present invention provides compounds of general formula (5a) to general formula (5i) or isomers thereof, crystalline forms thereof, pharmaceutically acceptable salts thereof, hydrates thereof, or solvates thereof: 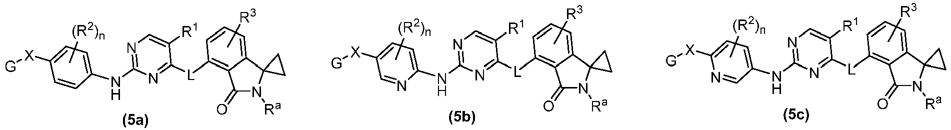 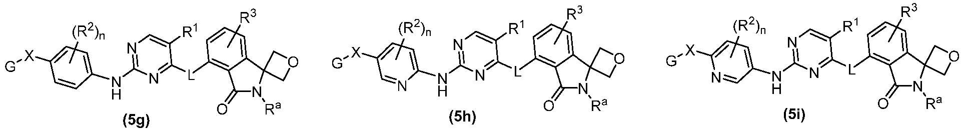 wherein L, X, G, R¹, R², n, and R³ are as defined above; R^{a} is selected from H, D, (C1-C3) alkyl, (C1-C3) haloalkyl, (C2-C4) alkenyl, (C2-C4) alkynyl, and (C3-C6) cycloalkyl, and is exemplified in the detailed description.

In some embodiments of the present invention, the present invention provides compounds of general formula (6a) to general formula (6i) or isomers thereof, crystalline forms thereof, pharmaceutically acceptable salts thereof, hydrates thereof, or solvates thereof: 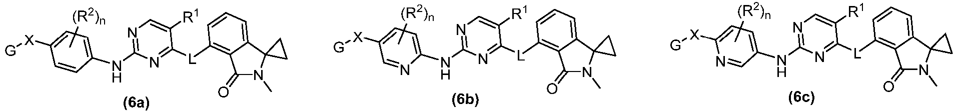 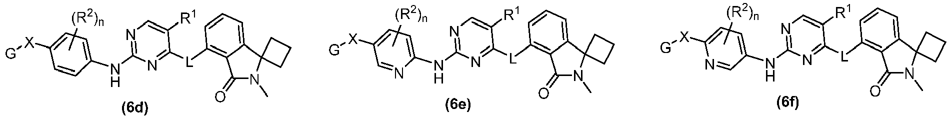 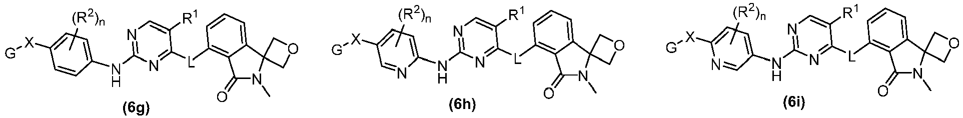 wherein L, X, G, R¹, R², and n are as defined above, and are exemplified in the detailed description.

In some embodiments of the present invention, the present invention provides compounds of general formula (7a) to general formula (7r) or isomers thereof, crystalline forms thereof, pharmaceutically acceptable salts thereof, hydrates thereof, or solvates thereof: 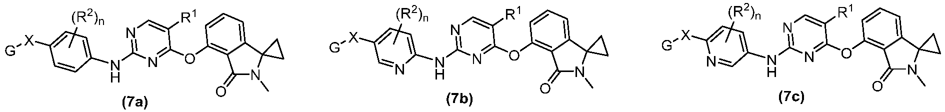 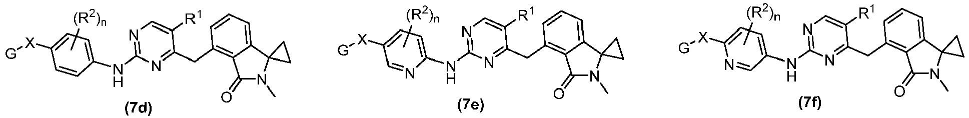 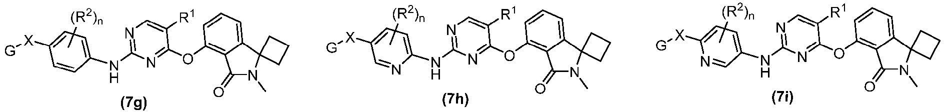 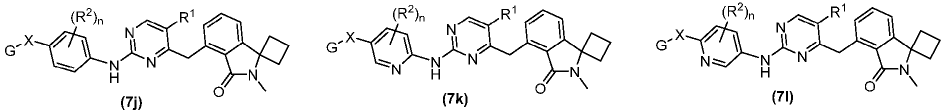 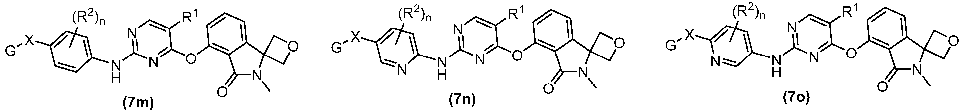 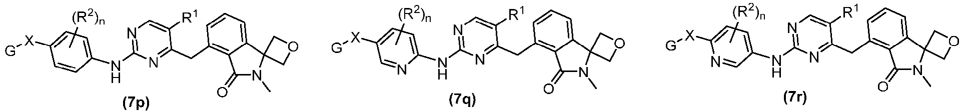 wherein X, G, R¹, R², and n are as defined above, and are exemplified in the detailed description.

In another specific embodiment of the present invention, the compound of general formula (1) has one of the following structures:

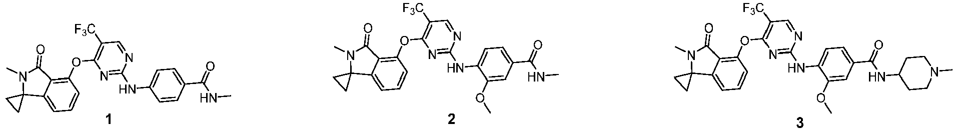

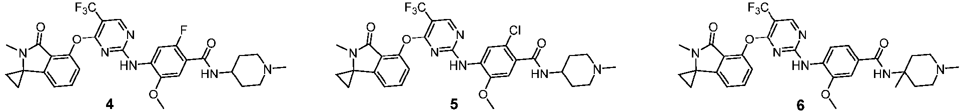

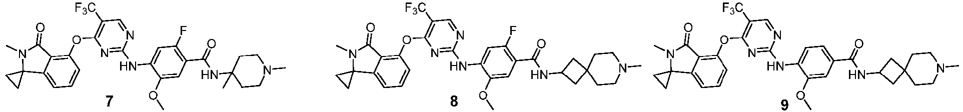

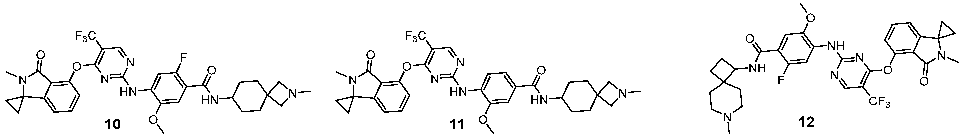

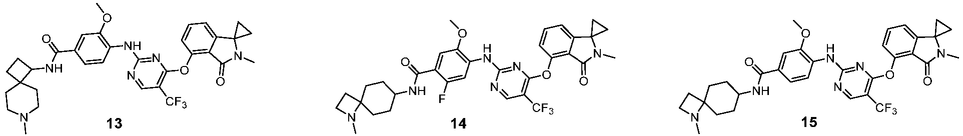

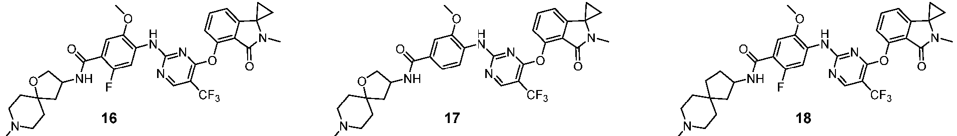

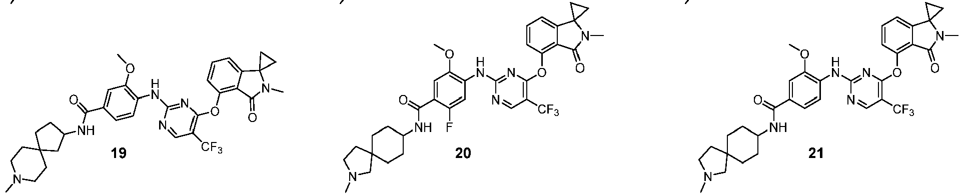

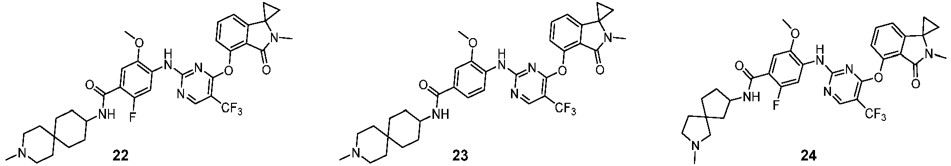

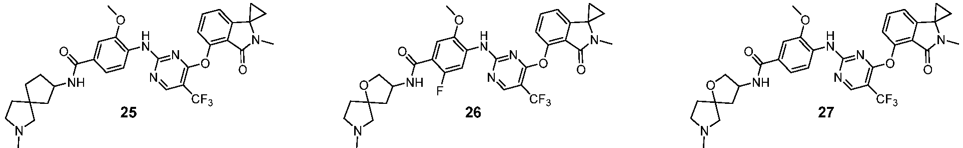

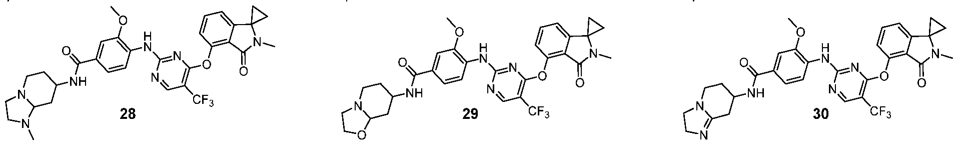

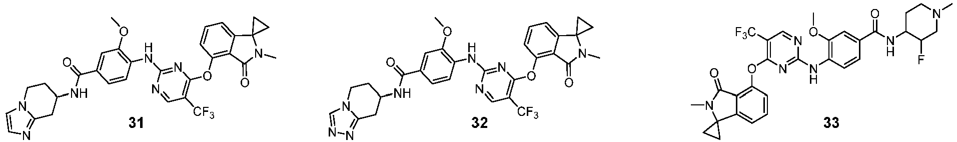

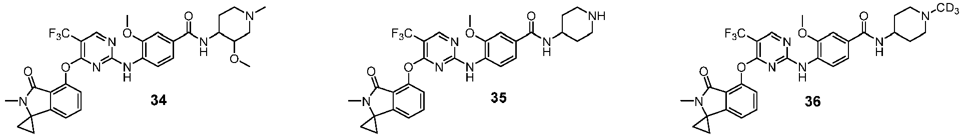

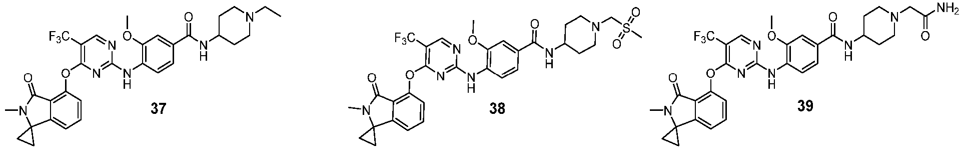

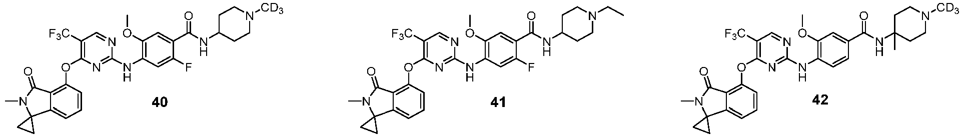

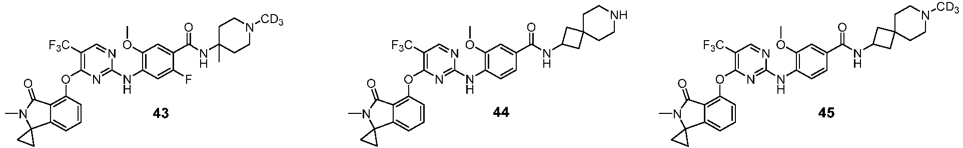

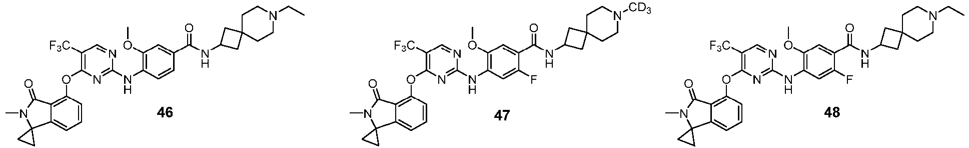

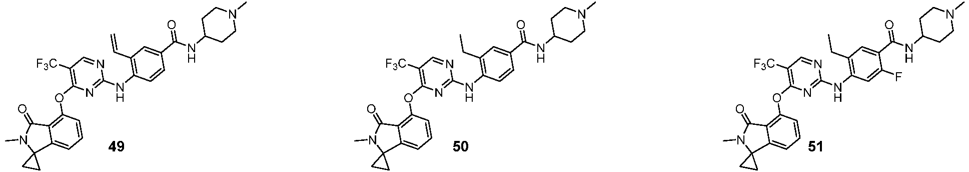

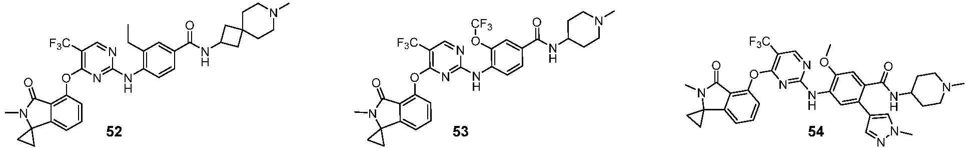

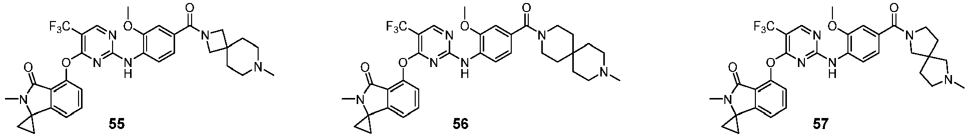

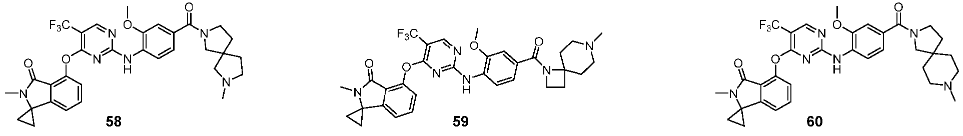

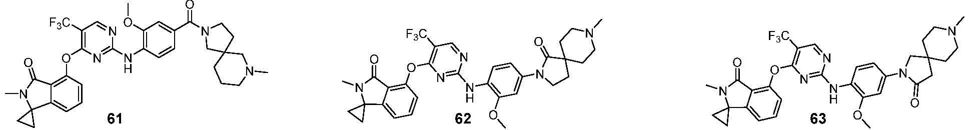

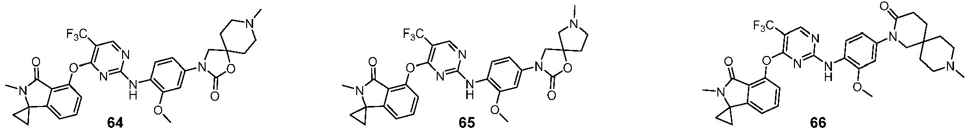

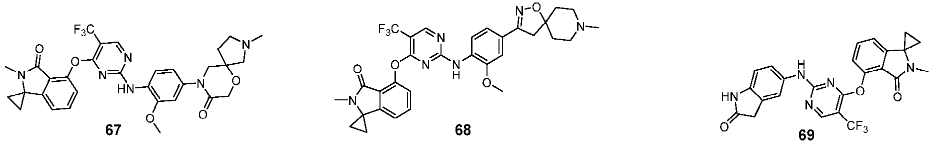

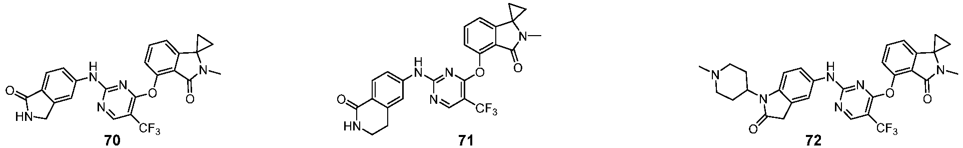

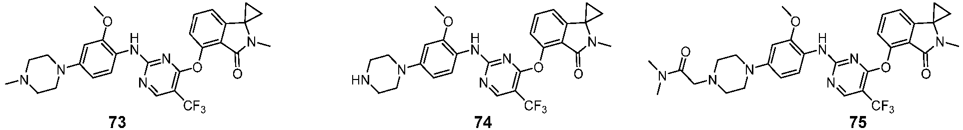

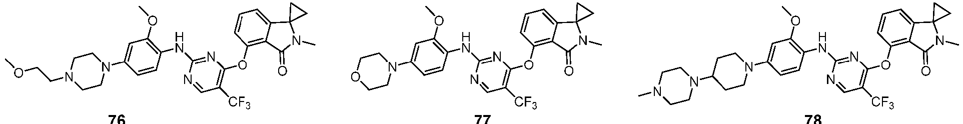

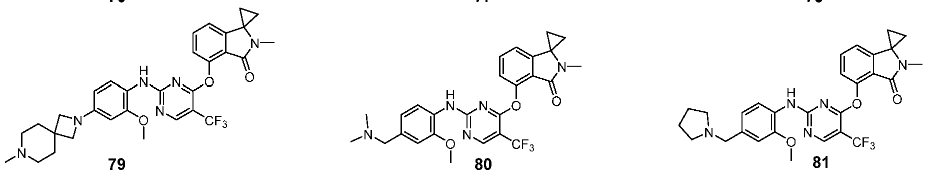

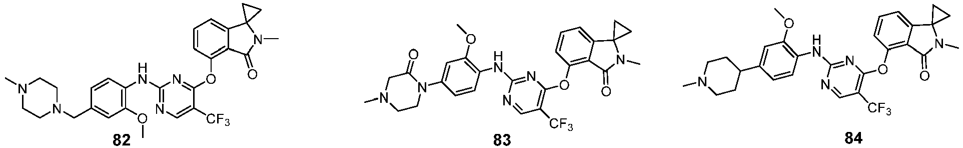

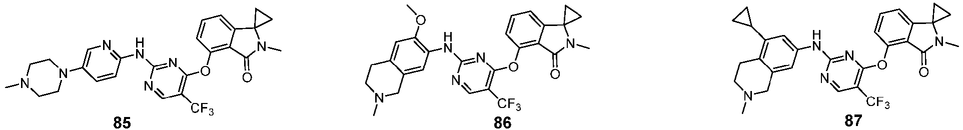

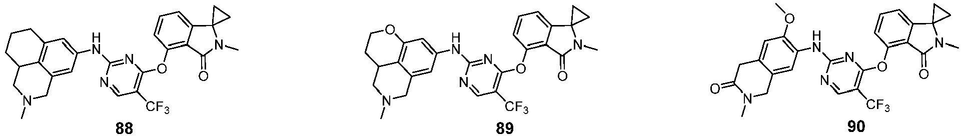

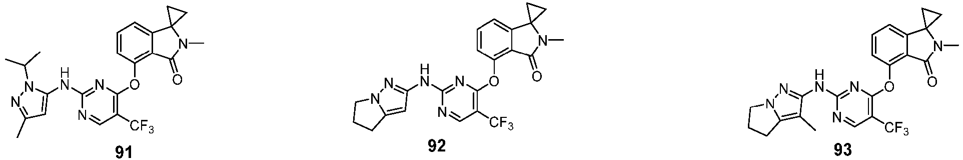

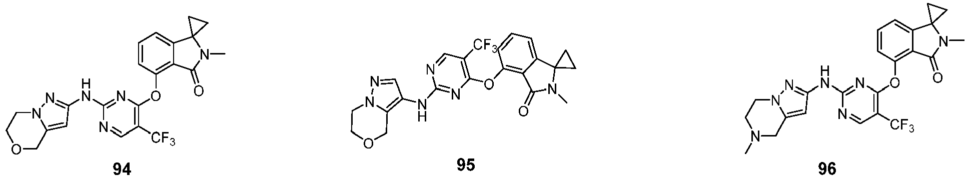

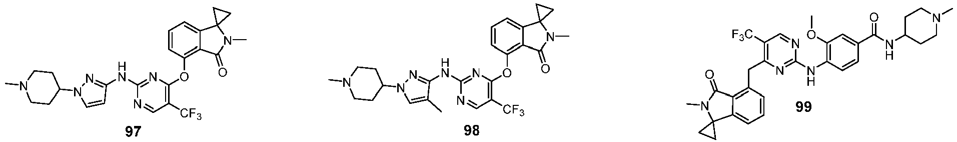

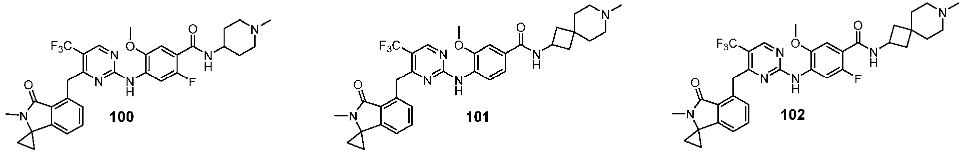

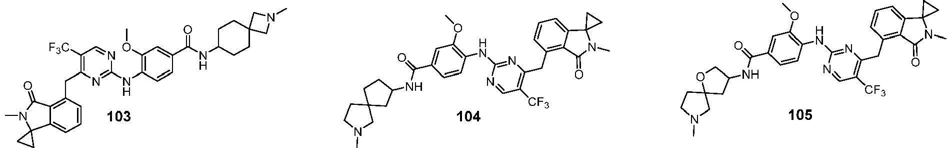

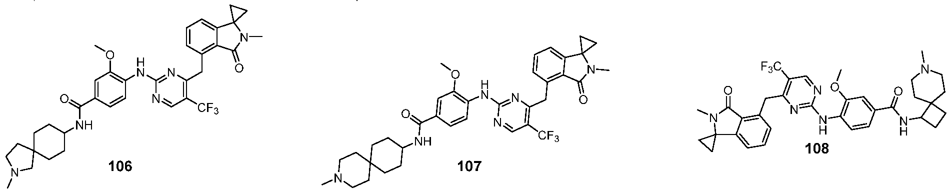

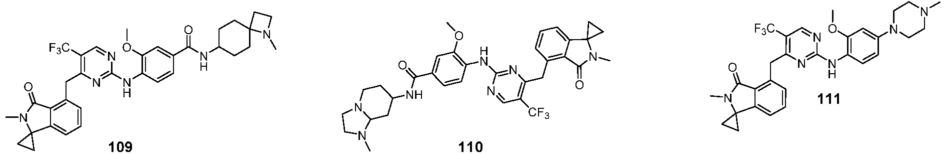

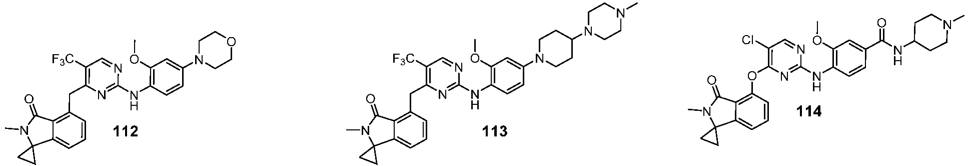

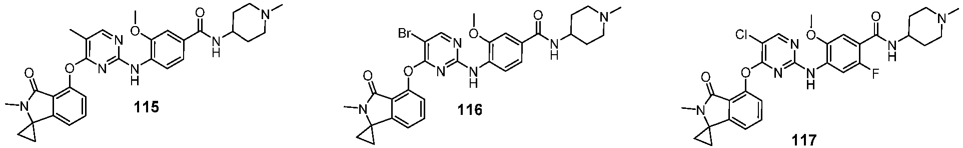

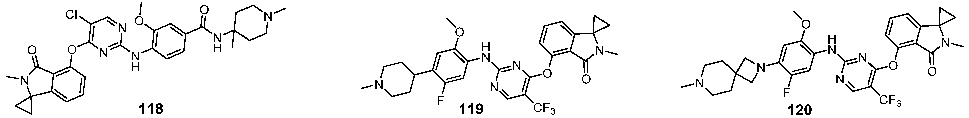

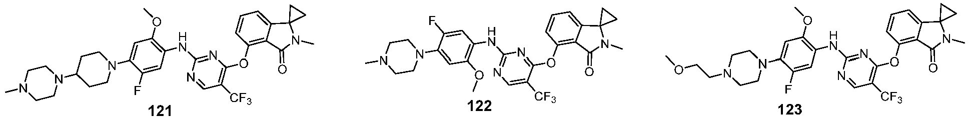

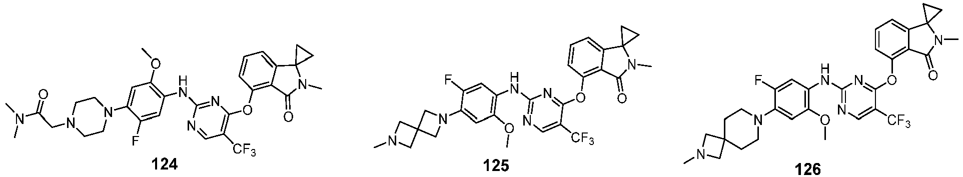

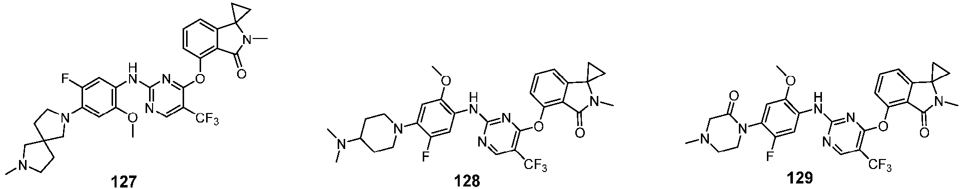

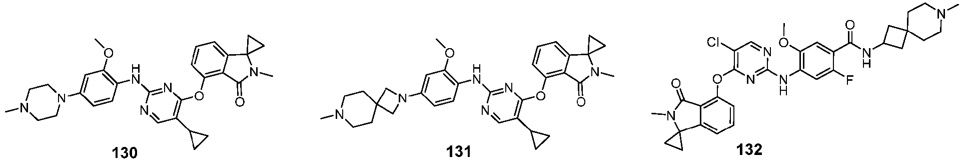

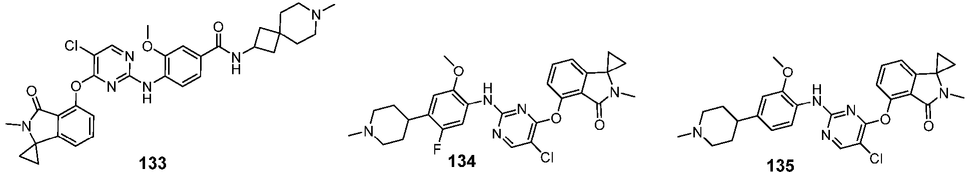

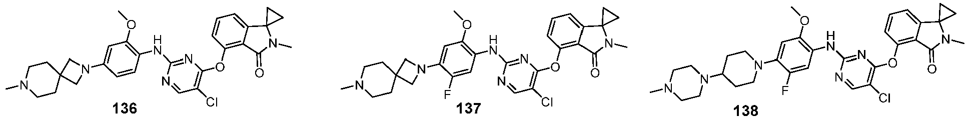

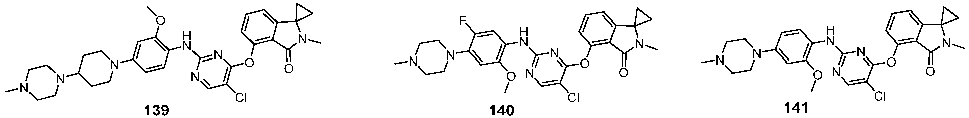

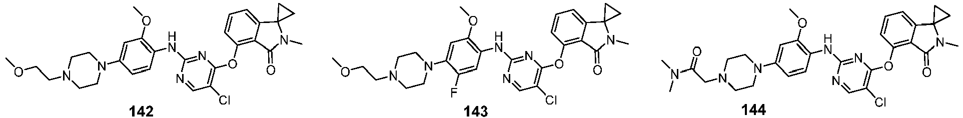

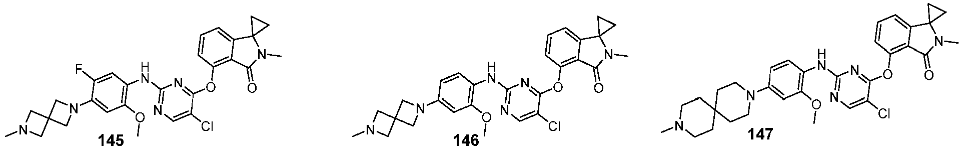

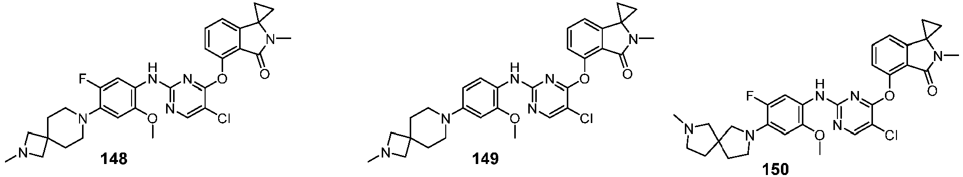

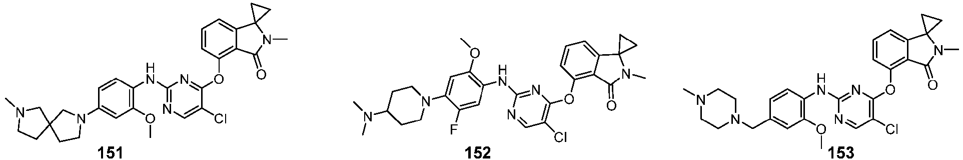

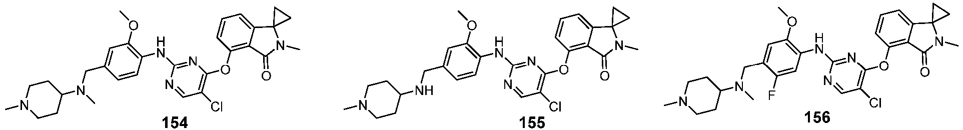

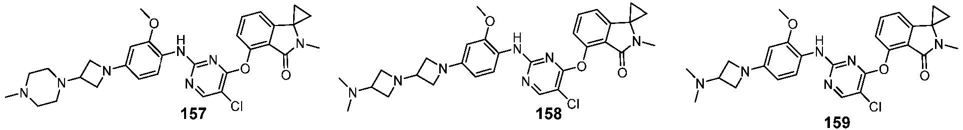

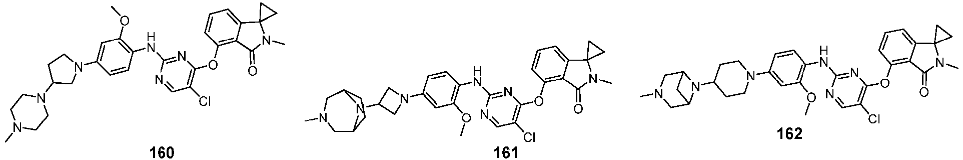

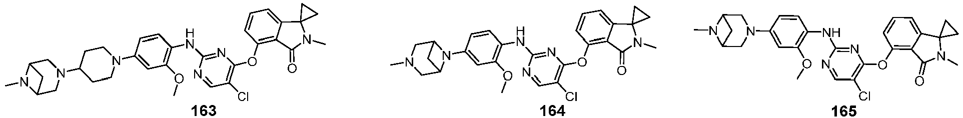

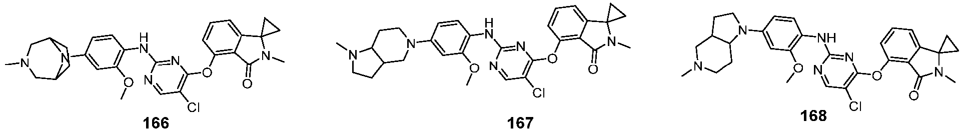

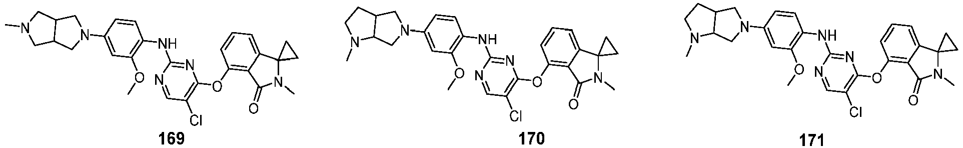

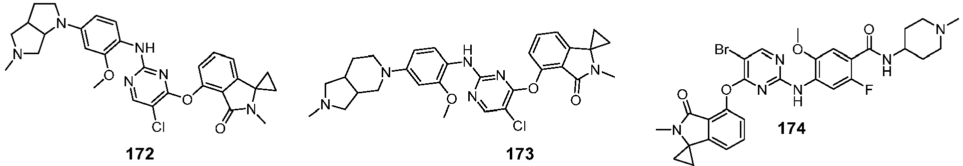

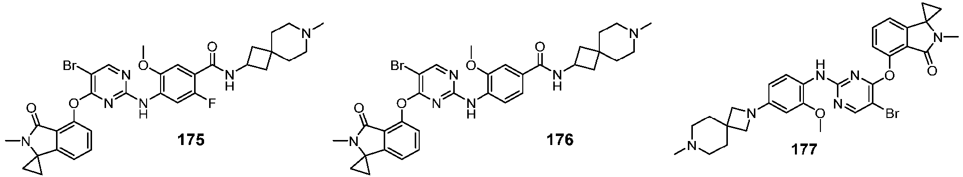

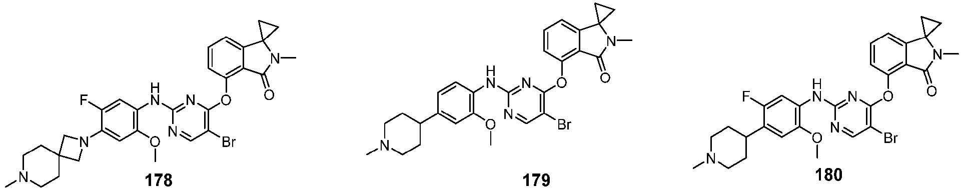

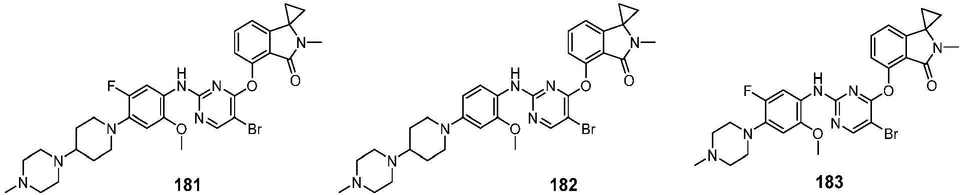

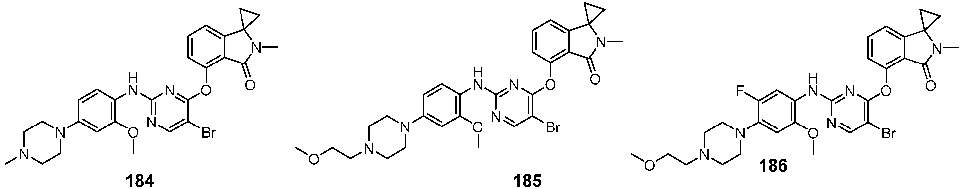

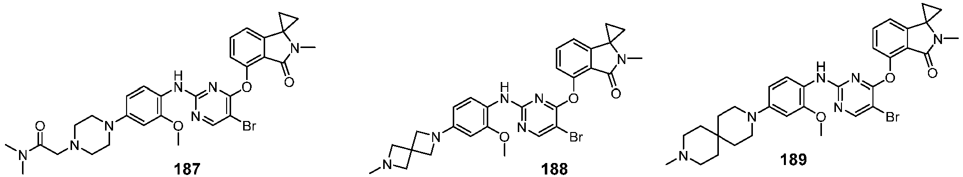

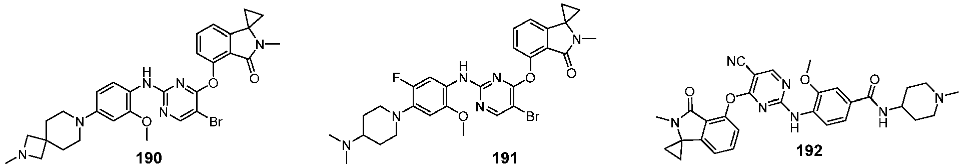

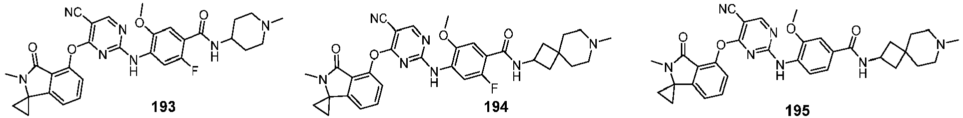

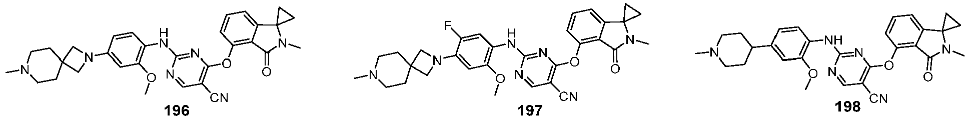

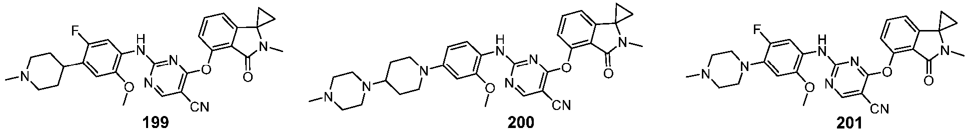

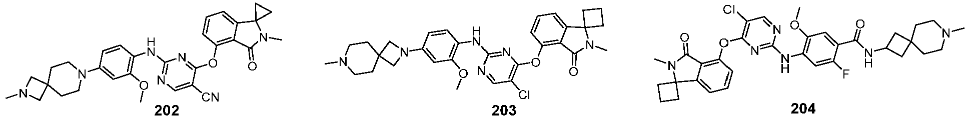

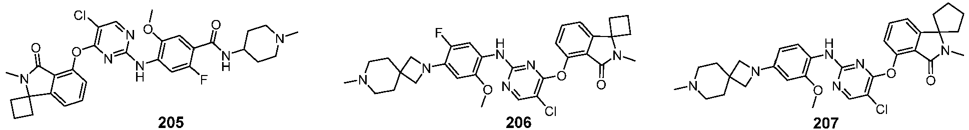

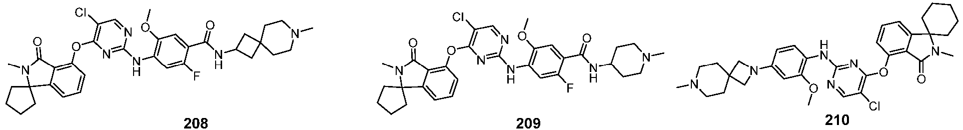

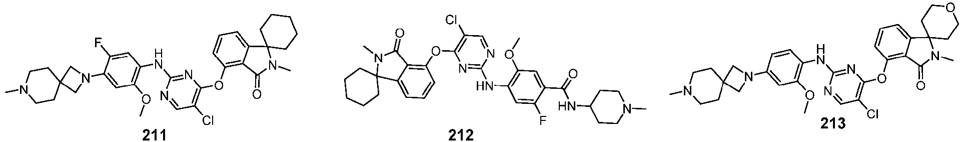

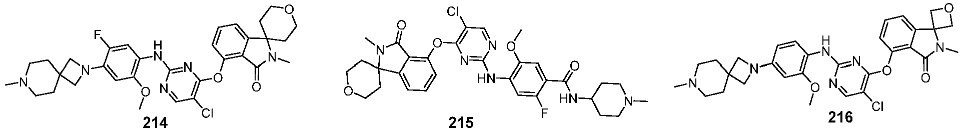

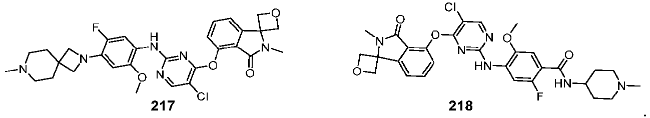

The present invention is further intended to provide a pharmaceutical composition comprising a pharmaceutically acceptable carrier, diluent, and/or excipient, as well as the compound of general formula (1) or the isomer thereof, the crystalline form thereof, the pharmaceutically acceptable salt thereof, the hydrate thereof, or the solvate thereof of the present invention as an active ingredient.

The present invention is still further intended to provide use of the compound of general formula (1) or the isomer thereof, the crystalline form thereof, the pharmaceutically acceptable salt thereof, the hydrate thereof, or the solvate thereof of the present invention or the pharmaceutical composition described above in the preparation of a medicament for treating, regulating, or preventing a disease related to FAK protein kinase. The disease is preferably cancer, and the cancer is a hematologic cancer and a solid tumor, preferably ovarian cancer, melanoma, breast cancer, head and neck cancer, pancreatic cancer, non-small cell lung cancer, colon cancer, prostate cancer, oral cancer, esophageal cancer, mesothelioma, myeloma, leukemia, and cancer metastases of these cancers.

The present invention is even further intended to provide a method for treating, regulating, or preventing a related disease mediated by FAK protein kinase, the method comprising administering to a subject a therapeutically effective amount of the compound of general formula (1) or the isomer thereof, the crystalline form thereof, the pharmaceutically acceptable salt thereof, the hydrate thereof, or the solvate thereof of the present invention, or the pharmaceutical composition described above.

It should be understood that both the aforementioned general description and the following detailed description of the present invention are exemplary and explanatory, and are intended to provide further explanation of the present invention claimed.

### Synthesis of Compounds

Methods for preparing the compound of general formula (1) of the present invention are specifically described below, but these specific methods do not limit the present invention in any way.

The compound of general formula (1) described above may be synthesized using standard synthetic techniques or well-known techniques in combination with the methods described herein. In addition, the solvents, temperatures, and other reaction conditions mentioned herein may vary. Starting materials for the synthesis of the compounds may be obtained synthetically or commercially. The compounds described herein and other related compounds with different substituents may be synthesized using well-known techniques and starting materials, including the methods found in March, ADVANCED ORGaNIC CHEMISTRY, 4th Ed., (Wiley 1992); Carey and Sundberg, ADVANCED ORGANIC CHEMISTRY, 4th Ed., Vols. A and B (Plenum 2000, 2001); and Green and Wuts, PROTECTIVE GROUPS IN ORGANIC SYNTHESIS, 3rd Ed., (Wiley 1999). General methods for preparing the compounds may be altered by using appropriate reagents and conditions for introducing different groups into the molecular formulas provided herein.

In one aspect, the compounds described herein are prepared according to methods well known in the art. However, the conditions of the methods, such as reactants, solvents, bases, the amount of the compound used, reaction temperature, and time required for the reaction are not limited to the following explanation. The compounds of the present invention may also be conveniently prepared by optionally combining various synthetic methods described herein or known in the art, and such combinations may be easily determined by those skilled in the art to which the present invention pertains. In one aspect, the present invention further provides a method for preparing the compound of general formula (1), wherein the compound of general formula (1) may be prepared using general reaction schemes 1-4 below:

### General Reaction Scheme 1

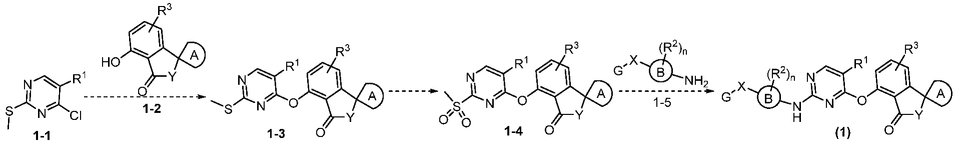

Embodiments of the compound of general formula (1) may be prepared according to general reaction scheme 1, wherein R¹, R², R³, X, L, Y, G, A, B, and n are as defined above. As shown in general reaction scheme 1, compound 1-1 reacts with compound 1-2 under alkaline conditions to give compound 1-3, compound 1-3 is oxidized to give compound 1-4, and compound 1-4 reacts with compound 1-5 under appropriate conditions to give the target compound (1).

### General Reaction Scheme 2

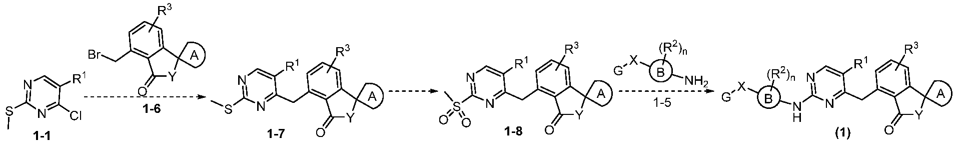

Embodiments of the compound of general formula (1) may be prepared according to general reaction scheme 2, wherein R¹, R², R³, X, L, Y, G, A, B, and n are as defined above. As shown in general reaction scheme 2, compound 1-1 undergoes a coupling reaction with compound 1-6 to give compound 1-7, compound 1-7 is oxidized to give compound 1-8, and compound 1-8 reacts with compound 1-5 under appropriate conditions to give the target compound (1).

### General Reaction Scheme 3

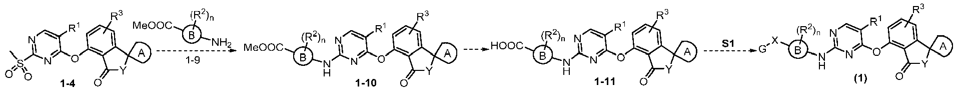

Embodiments of the compound of general formula (1) may be prepared according to general reaction scheme 3, wherein R¹, R², R³, X, L, Y, G, A, B, and n are as defined above. As shown in general reaction scheme 3, compound 1-4 reacts with compound 1-9 under appropriate conditions to give compound 1-10, compound 1-10 is hydrolyzed to give compound 1-11, and compound 1-11 undergoes a condensation reaction with fragment S1 to give the target compound (1).

### General Reaction Scheme 4

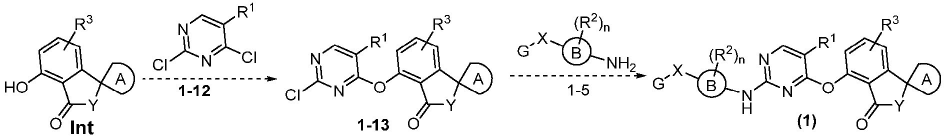

Embodiments of the compound of general formula (1) may be prepared according to general reaction scheme 4, wherein R¹, R², R³, X, L, Y, G, A, B, and n are as defined above. As shown in general reaction scheme 4, compound Int reacts with compound 1-12 under appropriate conditions to give compound 1-13, and compound 1-13 reacts with compound 1-5 under appropriate conditions to give the target compound (1).

### Further Forms of Compounds

"Pharmaceutically acceptable" herein refers to a substance, such as a carrier or diluent, which will not lead to loss of biological activity or properties of a compound and is relatively non-toxic. For example, when an individual is given a substance, the substance will not cause undesired biological effects or interact with any component contained therein in a deleterious manner.

The term "pharmaceutically acceptable salt" refers to a form of a compound that does not cause significant irritation to the organism receiving the administration or eliminate the biological activity and properties of the compound. In certain specific aspects, the pharmaceutically acceptable salt is obtained by subjecting the compound of the general formula to a reaction with acids or bases, wherein the acids or bases include, but are not limited to, those found in Stahl and Wermuth, Handbook of Pharmaceutical Salts: Properties, Selection, and Use, 1st Ed., (Wiley, 2002).

It should be understood that references to pharmaceutically acceptable salts include solvent addition forms or crystalline forms, especially solvates or polymorphs. A solvate contains either stoichiometric or non-stoichiometric amount of solvent and is selectively formed during crystallization in a pharmaceutically acceptable solvent such as water and ethanol. Hydrates are formed when the solvent is water, or alcoholates are formed when the solvent is ethanol. The solvates of the compound of general formula (1) are conveniently prepared or formed according to the methods described herein. For example, hydrates of the compound of general formula (1) are conveniently prepared by recrystallization in a mixed solvent of water/organic solvent, wherein the organic solvent used includes, but is not limited to, tetrahydrofuran, acetone, ethanol or methanol. Furthermore, the compounds described herein may be present in either a non-solvated form or a solvated form. In general, the solvated forms are considered equivalent to the non-solvated forms for purposes of the compounds and methods provided herein.

In other specific examples, the compound of general formula (1) is prepared in different forms including, but not limited to, amorphous, pulverized and nanoparticle forms. In addition, the compound of general formula (1) includes crystalline forms, and may also be polymorphs. Polymorphs include different lattice arrangements of the same elements of a compound. Polymorphs generally have different X-ray diffraction spectra, infrared spectra, melting points, density, hardness, crystalline forms, optical and electrical properties, stability and solubility. Different factors such as a recrystallization solvent, crystallization rate, and storage temperature may lead to a single dominant crystalline form.

In another aspect, the compound of general formula (1) may have a chiral center and/or axial chirality, and thus may be present in the form of a racemate, a racemic mixture, a single enantiomer, a diastereomeric compound, a single diastereomer and a cis-trans isomer. Each chiral center or axial chirality will independently produce two optical isomers, and all possible optical isomers, diastereomeric mixtures, and pure or partially pure compounds are included within the scope of the present invention. The present invention is meant to include all such isomeric forms of these compounds.

The compound of the present invention may contain unnatural proportions of atomic isotopes at one or more of the atoms that constitute the compound. For example, the compound may be labeled with radioactive isotopes, such as tritium (³H), iodine-125 (¹²⁵I) and C-14 (¹⁴C). For another example, deuterium can be used to substitute a hydrogen atom to form a deuterated compound. The bond formed by deuterium and carbon is stronger than that formed by ordinary hydrogen and carbon. Compared with an undeuterated medicament, the deuterated medicament generally has the advantages of reduced toxic and side effects, increased pharmaceutical stability, enhanced efficacy, prolonged pharmaceutical *in vivo* half-life and the like. All isotopic variations of the compound of the present invention, whether radioactive or not, are contained within the scope of the present invention.

### Terminology

Unless otherwise stated, the terms used in the present application, including those in the specification and claims, are defined as follows. It must be noted that in the specification and the appended claims, the singular forms "a" and "an" include plural meanings unless clearly indicated otherwise. Unless otherwise stated, conventional methods for mass spectrometry, nuclear magnetic resonance spectroscopy, HPLC, protein chemistry, biochemistry, recombinant DNA techniques and pharmacology are used. As used herein, "or" or "and" refers to "and/or" unless otherwise stated.

Unless otherwise specified, "alkyl" refers to a saturated aliphatic hydrocarbon group, including linear and branched groups containing 1 to 6 carbon atoms. Lower alkyl groups containing 1 to 4 carbon atoms, such as methyl, ethyl, propyl, 2-propyl, n-butyl, isobutyl, or tert-butyl, are preferred. As used herein, "alkyl" includes unsubstituted and substituted alkyl, particularly alkyl substituted with one or more halogens. Preferred alkyl is selected from CH₃, CH₃CH₂, CF₃, CHF₂, CF₃CH₂, CF₃(CH₃)CH, ⁱPr, ⁿPr, ⁱBu, ⁿBu or ^{t}Bu.

Unless otherwise specified, "alkylene" refers to a divalent alkyl as defined above. Examples of alkylene include, but are not limited to, methylene and ethylene.

Unless otherwise specified, "alkenyl" refers to an unsaturated aliphatic hydrocarbon group containing carbon-carbon double bonds, including linear or branched groups containing 1 to 14 carbon atoms. Lower alkenyl groups containing 1 to 4 carbon atoms, such as vinyl, 1-propenyl, 1-butenyl, or 2-methylpropenyl, are preferred.

Unless otherwise specified, "alkenylene" refers to a divalent alkenyl as defined above.

Unless otherwise specified, "alkynyl" refers to an unsaturated aliphatic hydrocarbon group containing carbon-carbon triple bonds, including linear and branched groups containing 1 to 14 carbon atoms. Lower alkynyl groups containing 1 to 4 carbon atoms, such as ethynyl, 1-propynyl, or 1-butynyl, are preferred.

Unless otherwise specified, "alkynylene" refers to a divalent alkynyl as defined above.

Unless otherwise specified, "cycloalkyl" refers to a non-aromatic hydrocarbon ring system (monocyclic, bicyclic or polycyclic), and partially unsaturated cycloalkyl may be referred to as "cycloalkenyl" if the carbocyclic ring contains at least one double bond, or "cycloalkynyl" if the carbocyclic ring contains at least one triple bond. Cycloalkyl may include monocyclic or polycyclic groups (e.g., having 2, 3 or 4 fused rings) and spiro rings. In some embodiments, cycloalkyl is monocyclic. In some embodiments, cycloalkyl is monocyclic or bicyclic. The ring carbon atoms of cycloalkyl may optionally be oxidized to form an oxo or thio group. Cycloalkyl further includes cycloalkylene. In some embodiments, cycloalkyl contains 0, 1 or 2 double bonds. In some embodiments, cycloalkyl contains 1 or 2 double bonds (partially unsaturated cycloalkyl). In some embodiments, cycloalkyl may be fused to aryl, heteroaryl, cycloalkyl and heterocycloalkyl. In some embodiments, cycloalkyl may be fused to aryl, cycloalkyl and heterocycloalkyl. In some embodiments, cycloalkyl may be fused to aryl and heterocycloalkyl. In some embodiments, cycloalkyl may be fused to aryl and cycloalkyl. Examples of cycloalkyl include cyclopropyl, cyclobutyl, cyclopentyl, cyclohexyl, cycloheptyl, cyclopentenyl, cyclohexenyl, cyclohexadienyl, cycloheptatrienyl, norcamphanyl, norpinanyl, norcarnyl, bicyclo[1.1.1]pentyl, bicyclo[2.1.1]hexyl and the like.

Unless otherwise specified, "cycloalkylene" refers to a divalent cycloalkyl as defined above.

Unless otherwise specified, "alkoxy" refers to an alkyl group that bonds to the rest of the molecule through an ether oxygen atom. Representative alkoxy groups are those having 1-6 carbon atoms, such as methoxy, ethoxy, propoxy, isopropoxy, butoxy, isobutoxy, sec-butoxy, and tert-butoxy. As used herein, "alkoxy" includes unsubstituted and substituted alkoxy, particularly alkoxy substituted with one or more halogens. Preferred alkoxy is selected from OCH₃, OCF₃, CHF₂O, CF₃CH₂O, ⁱ⁻PrO, ⁿ⁻PrO, ⁱ⁻BuO, ⁿ⁻BuO or ^{t-}BuO.

Unless otherwise specified, "aryl" refers to an aromatic hydrocarbon group, which is monocyclic or polycyclic; for example, a monocyclic aryl ring is fused to one or more carbocyclic aromatic groups. Examples of aryl include, but are not limited to, phenyl, naphthyl, and phenanthryl. Unless otherwise specified, "aryloxy" refers to an aryl group that bonds to the rest of the molecule through an ether oxygen atom. Examples of aryloxy include, but are not limited to, phenoxy and naphthoxy.

Unless otherwise specified, "arylene" refers to a divalent aryl as defined above. Examples of arylene include, but are not limited to, phenylene, naphthylene, and phenanthrylene.

Unless otherwise specified, "heteroaryl" refers to an aromatic group containing one or more heteroatoms (O, S, or N), and the heteroaryl is monocyclic or polycyclic. For example, a monocyclic heteroaryl ring is fused to one or more carbocyclic aromatic groups or other monocyclic heterocycloalkyl groups. Examples of heteroaryl include, but are not limited to, pyridinyl, pyridazinyl, imidazolyl, pyrimidinyl, pyrazolyl, triazolyl, pyrazinyl, quinolinyl, isoquinolinyl, furanyl, thienyl, isoxazolyl, thiazolyl, oxazolyl, isothiazolyl, pyrrolyl, indolyl, benzimidazolyl, benzofuranyl, benzothiazolyl, benzothienyl, benzoxazolyl, benzopyridinyl, pyrrolopyrimidinyl, 1H-pyrrolo[3,2-b]pyridinyl, 1H-pyrrolo[2,3-c]pyridinyl, 1H-pyrrolo[3,2-c]pyridinyl, 1H-pyrrolo[2,3-b]pyridinyl, 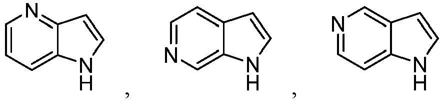 and

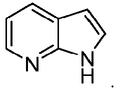

Unless otherwise specified, "heterocycloalkyl" refers to a non-aromatic ring or ring system, which may optionally contain one or more alkenylene as part of the ring structure, having at least one heteroatom ring member independently selected from boron, phosphorus, nitrogen, sulfur, oxygen, and phosphorus. Partially unsaturated heterocycloalkyl may be referred to as "heterocycloalkenyl" if heterocycloalkyl contains at least one double bond, or "heterocycloalkynyl" if the heterocycloalkyl contains at least one triple bond. Heterocycloalkyl may include monocyclic, bicyclic, spiro ring, or polycyclic systems (e.g., having two fused or bridged rings). In some embodiments, heterocycloalkyl is a monocyclic group having 1, 2, or 3 heteroatoms independently selected from nitrogen, sulfur, and oxygen. The ring carbon atoms and heteroatoms of heterocycloalkyl may optionally be oxidized to form oxo or thio groups or other oxidized bonds (e.g., C(O), S(O), C(S) or S(O)2, N-oxides, etc.), or the nitrogen atoms may be quaternized. Heterocycloalkyl may be attached via a ring carbon atom or a ring heteroatom. In some embodiments, heterocycloalkyl contains 0 to 3 double bonds. In some embodiments, heterocycloalkyl contains 0 to 2 double bonds. The definition of heterocycloalkyl further includes moieties (also referred to as partially unsaturated heterocyclic rings) having one or more aromatic rings fused to (i.e., sharing a bond with) the heterocycloalkyl ring, for example, benzo-derivatives of piperidine, morpholine, azepin, thienyl, or the like. Heterocycloalkyl containing a fused aromatic ring may be attached via any ring atom, including ring atoms of the fused aromatic ring. Examples of heterocycloalkyl include, but are not limited to, azetidinyl, azepinyl, dihydrobenzofuranyl, dihydrofuranyl, dihydropyranyl, N-morpholinyl, 3-oxa-9-azaspiro[5.5]undecyl, 1-oxa-8-azaspiro[4.5]decyl, piperidinyl, piperazinyl, oxopiperazinyl, pyranyl, pyrrolidinyl, quininyl, tetrahydrofuranyl, tetrahydropyranyl, 1,2,3,4-tetrahydroquinolinyl, tropanyl, 4,5,6,7-tetrahydrothiazolo[5,4-c]pyridinyl, 4,5,6,7-tetrahydro-1H-imidazo[4,5-c]pyridine, N-methylpiperidinyl, tetrahydroimidazolyl, pyrazolidinyl, butyrolactam, valerolactam, imidazolidinonyl, hydantoinyl, dioxolanyl, phthalimidyl, pyrimidine-2,4(1*H*,3*H*)-dione, 1,4-dioxanyl, morpholinyl, thiomorpholinyl, thiomorpholinyl-S-oxide, thiomorpholinyl-S,S-oxide, piperazinyl, pyranyl, pyridonyl, 3-pyrrolinyl, thiopyranyl, pyronyl, tetrahydrothienyl, 2-azaspiro[3.3]heptanyl, indolinyl,

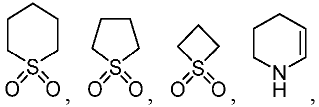

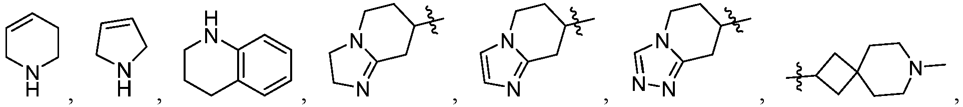

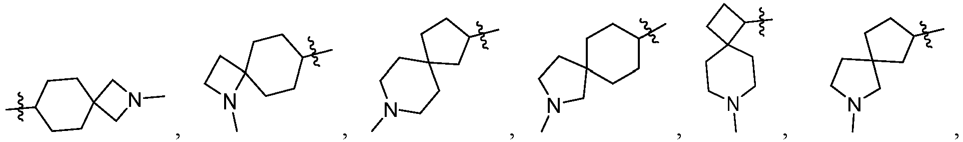

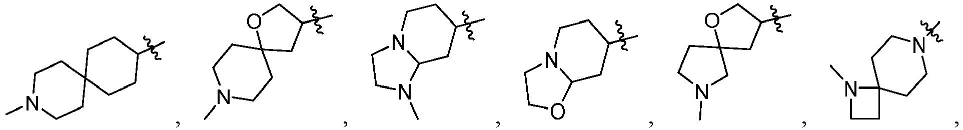

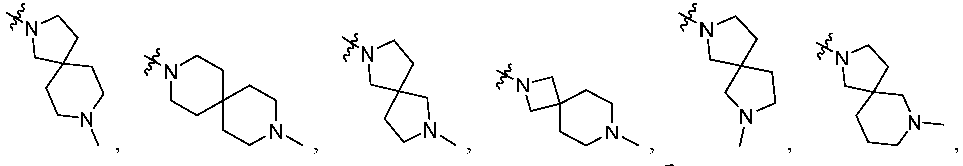

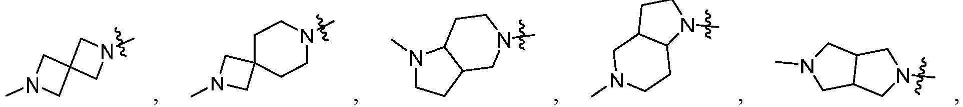

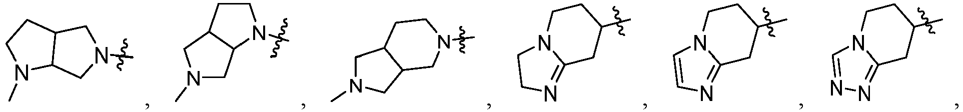

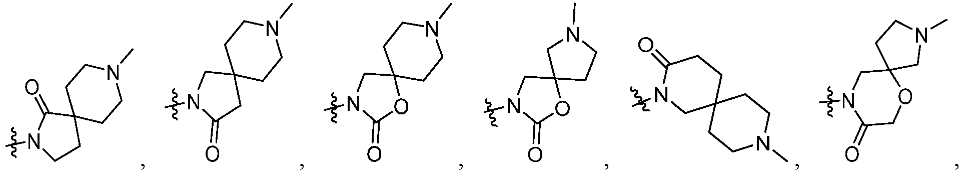

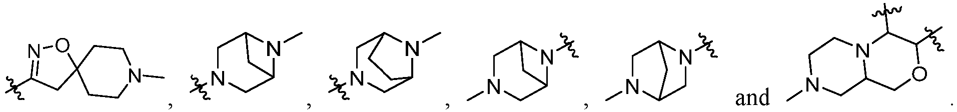

Unless otherwise specified, "heterocycloalkylidene" means a divalent heterocycloalkyl group as defined above.

Unless otherwise specified, "oxo" refers to =O; for example, a group formed by substitution of carbon with one oxo is "carbonyl 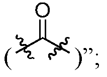 a group formed by substitution of sulfur with one oxo is "sulfinyl 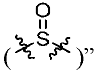 and a group formed by substitution of sulfur with two oxos is "sulfonyl

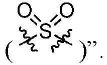

Unless otherwise specified, "halogen" (or halo) refers to fluorine, chlorine, bromine or iodine. The term "halo" (or "halogenated") before a group name indicates that the group is partially or fully halogenated, that is, substituted in any combination with F, Cl, Br or I, preferably with F or Cl.

"Optional" or "optionally" means that the subsequently described event or circumstance may, but does not necessarily, occur, and the description includes instances where the event or circumstance occurs and instances where the event or circumstance does not occur.

Unless otherwise specified, it is to be understood that the word "comprises", or variants thereof such as "includes" or "contains", refers to the inclusion of the elements or integers, or groups of elements or integers, specified, but not to the exclusion of any other elements or integers, or groups of elements or integers, exclude any other element or integer, or group of elements or integers.

The substituent "-O-CH₂-O-" means that two oxygen atoms in the substituent are linked to two adjacent carbon atoms in the heterocycloalkyl, aryl or heteroaryl, for example:

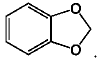

When the number of a linker group is 0, such as -(CH₂)₀-, it means that the linker group is a single bond.

When one of the variables is selected from a chemical bond, it means that the two groups linked by this variable are linked directly. For example, when L in X-L-Y represents a chemical bond, it means that the structure is actually X-Y.

The term "membered ring" includes any cyclic structure. The term "membered" is intended to refer to the number of backbone atoms that form a ring. For example, cyclohexyl, pyridinyl, pyranyl and thiopyranyl are six-membered rings, and cyclopentyl, pyrrolyl, furanyl and thienyl are five-membered rings.

The term "moiety" refers to a specific portion or functional group of a molecule. A chemical moiety is generally considered to be a chemical entity contained in or attached to a molecule. Unless otherwise stated, the absolute configuration of a stereogenic center is represented by a wedged solid bond ( 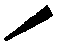 ) and a wedged dashed bond ( 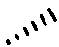 ), and the relative configuration of a stereogenic center is represented by a straight solid bond ( 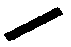 ) and a straight dashed bond ( 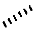 ). A wavy line ( 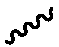 ) represents a wedged solid bond ( 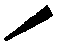 ) or a wedged dashed bond ( 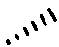 ), or a wavy line ( 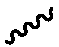 ) represents a straight solid bond ( 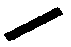 ) or a straight dashed bond ( 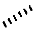 ).

Unless otherwise stated, a single bond or a double bond is represented by -̅ -̅ -̅.

### Specific Pharmaceutical and Medical Terminology

The term "acceptable", as used herein, means that a formula component or an active ingredient does not unduly and adversely affect a general therapeutic target's health.

The terms "treatment", "treatment course", and "therapy", as used herein, include alleviating, inhibiting, or ameliorating a symptom or condition of a disease; inhibiting the development of complications; ameliorating or preventing underlying metabolic syndrome; inhibiting the development of a disease or symptom, e.g., controlling the progression of a disease or condition; alleviating a disease or symptom; leading to disease or symptom regression; and alleviating a complication caused by a disease or symptom, or preventing or treating a sign caused by a disease or symptom. As used herein, a compound or pharmaceutical composition, when administered, can ameliorate a disease, symptom, or condition, which particularly refers to ameliorating the severity, delaying the onset, slowing the progression, or reducing the duration of the disease. Fixed or temporary administration, or continuous or intermittent administration, may be attributed to or associated with the administration.

"Active ingredient" refers to the compound of general formula (1), and pharmaceutically acceptable inorganic or organic salts of the compound of general formula (1). The compound of the present invention may contain one or more asymmetric centers (chiral center or axial chirality) and thus occurs in the forms of a racemate, a racemic mixture, a single enantiomer, a diastereomeric compound and a single diastereomer. Asymmetric centers that may be present depend on the nature of the various substituents on the molecule. Each of such asymmetric centers will independently produce two optical isomers, and all possible optical isomers, diastereomeric mixtures and pure or partially pure compounds are included within the scope of the present invention. The present invention is meant to include all such isomeric forms of these compounds.

The terms such as "compound", "composition", "agent", or "medicine or medicament" are used interchangeably herein and all refer to a compound or composition that, when administered to an individual (human or animal), is capable of inducing a desired pharmacological and/or physiological response by local and/or systemic action.

The term "administered, administering, or administration" refers herein to the direct administration of the compound or composition, or the administration of a prodrug, derivative, analog, or the like of the active compound.

Although the numerical ranges and parameters defining the broad scope of the present invention are approximations, the related numerical values set forth in the specific examples have been presented herein as precisely as possible. Any numerical value, however, inherently contains a standard deviation necessarily resulting from certain methods of testing. Herein, "about" generally means that the actual numerical value is within a particular numerical value or range ± 10%, 5%, 1% or 0.5%. Alternatively, the term "about" indicates that the actual numerical value falls within the acceptable standard error of a mean, as considered by those skilled in the art. All ranges, quantities, numerical values, and percentages used herein (e.g., to describe an amount of a material, a length of time, a temperature, an operating condition, a quantitative ratio, and the like) are to be understood as being modified by the word "about", except in the experimental examples or where otherwise explicitly indicated. Accordingly, unless otherwise contrarily stated, the numerical parameters set forth in the specification and the appended claims are all approximations that may vary as desired. At least, these numerical parameters should be understood as the significant digits indicated or the numerical values obtained using conventional rounding rules.

Unless otherwise defined in the specification, the scientific and technical terms used herein have the same meaning as commonly understood by those skilled in the art. Furthermore, nouns in their singular forms used in the specification encompass their plural forms, unless contradicted by context; nouns in their plural forms used also encompass their singular forms.

### Therapeutic Use

The present invention provides a method for treating a disease, including but not limited to a related condition involving FAK protein kinase (e.g., cancer), with the compound of general formula (1) or the pharmaceutical composition of the present invention.

In some embodiments, a method for treating cancer is provided, the method comprising administering to an individual in need thereof an effective amount of any of the aforementioned pharmaceutical compositions comprising the compound of structural general formula (1). In some embodiments, the cancer is related to FAK kinase. In some embodiments, the compound of general formula (1) may be used in combination with other anti-cancer drugs; in some embodiments, the compound of formula (1) may be used in combination with a PD-1 inhibitor. In other embodiments, the cancer is a hematologic cancer and a solid tumor, including but not limited to leukemia, breast cancer, lung cancer, pancreatic cancer, colon cancer, bladder cancer, brain cancer, urothelial cancer, prostate cancer, liver cancer, ovarian cancer, head and neck cancer, gastric cancer, mesothelioma, or all cancer metastases; in other embodiments, the cancer is ovarian cancer, melanoma, breast cancer, head and neck cancer, pancreatic cancer, non-small cell lung cancer, colon cancer, prostate cancer, oral cancer, esophageal cancer, mesothelioma, myeloma, leukemia, and cancer metastases of these cancers.

### Route of administration

The compound and the pharmaceutically acceptable salt thereof of the present invention can be made into various formulations comprising a safe and effective amount of the compound or the pharmaceutically acceptable salt thereof of the present invention, and a pharmaceutically acceptable excipient or carrier, wherein the "safe and effective amount" means that the amount of the compound is sufficient to significantly improve the condition without causing serious side effects. The safe and effective amount of the compound is determined according to the age, condition, course of treatment, and other specific conditions of a treated subject.

The "pharmaceutically acceptable excipient or carrier" refers to one or more compatible solid or liquid fillers or gel substances that are suitable for human use and must be of sufficient purity and sufficiently low toxicity. "Compatible" herein means that the components of the composition are capable of intermixing with the compound of the present invention and with each other, without significantly diminishing the pharmaceutical efficacy of the compound. Examples of pharmaceutically acceptable excipients or carriers include cellulose and derivatives thereof (e.g., sodium carboxymethylcellulose, sodium ethylcellulose, or cellulose acetate), gelatin, talc, solid lubricants (e.g., stearic acid or magnesium stearate), calcium sulfate, vegetable oil (e.g., soybean oil, sesame oil, peanut oil, or olive oil), polyols (e.g., propylene glycol, glycerol, mannitol, or sorbitol), emulsifiers (e.g., Tween^{®}), wetting agents (e.g., sodium lauryl sulfate), colorants, flavoring agents, stabilizers, antioxidants, preservatives, pyrogen-free water, etc.

When the compound of the present invention is administered, it may be administered orally, rectally, parenterally (intravenously, intramuscularly, or subcutaneously), or topically.

Solid dosage forms for oral administration include capsules, tablets, pills, pulvises, and granules. In these solid dosage forms, the active compound is mixed with at least one conventional inert excipient (or carrier), such as sodium citrate or dicalcium phosphate, or with the following ingredients: (a) fillers or extenders, such as starch, lactose, sucrose, glucose, mannitol, and silicic acid; (b) binders, such as hydroxymethyl cellulose, alginate, gelatin, polyvinylpyrrolidone, sucrose, and acacia; (c) humectants, such as glycerol; (d) disintegrants, such as agar, calcium carbonate, potato or tapioca starch, alginic acid, certain complex silicates, and sodium carbonate; (e) solution retarders, such as paraffin; (f) absorption accelerators, such as quaternary ammonium compounds; (g) wetting agents, such as cetyl alcohol and glycerol monostearate; (h) adsorbents, such as kaolin; and (i) lubricants, such as talc, calcium stearate, magnesium stearate, solid polyethylene glycol and sodium lauryl sulfate, or mixtures thereof. In the case of capsules, tablets, and pills, the dosage forms may further include buffers.

Solid dosage forms such as tablets, dragees, capsules, pills and granules can be prepared using coatings and shells such as enteric coatings and other materials well known in the art. They may include opacifying agents, and the active compound or compound in such a composition may be released in a certain part of the digestive tract in a delayed manner. Examples of embedding components that can be used are polymeric substances and wax-based substances. If necessary, the active compound can also be in microcapsule form with one or more of the excipients described above.

Liquid dosage forms for oral administration include pharmaceutically acceptable emulsions, solutions, suspensions, syrups, and elixirs. In addition to the active compound, the liquid dosage form may include inert diluents commonly used in the art, such as water or other solvents, solubilizers and emulsifiers, for example, ethanol, isopropanol, ethyl carbonate, ethyl acetate, propylene glycol, 1,3-butanediol, dimethylformamide, and oils, especially cottonseed oil, peanut oil, corn germ oil, olive oil, castor oil and sesame oil, or mixtures of these substances.

Besides such inert diluents, the composition may further include adjuvants, such as wetting agents, emulsifiers, suspending agents, sweeteners, flavoring agents, and perfuming agents.

In addition to the active compound, suspensions may include suspending agents, such as ethoxylated isostearyl alcohols, polyoxyethylene sorbitol and sorbitan esters, microcrystalline cellulose, aluminum methylate and agar, or mixtures of these substances.

Compositions for parenteral injection may include physiologically acceptable sterile aqueous or anhydrous solutions, dispersions, suspensions or emulsions, and sterile powders for redissolving into sterile injectable solutions or dispersions. Suitable aqueous and non-aqueous carriers, diluents, solvents, or excipients include water, ethanol, polyols, and suitable mixtures thereof. Dosage forms for topical administration of the compound of the present invention include ointments, pulvises, patches, sprays, and inhalants. The active ingredient is mixed under sterile conditions with a physiologically acceptable carrier and any preservatives, buffers or propellants that may be required if necessary.

The compound of the present invention may be administered alone or in combination with other pharmaceutically acceptable compounds. When the pharmaceutical composition is used, a safe and effective amount of the compound of the present invention is administered to a mammal (such as a human) to be treated, wherein the dose of administration is a pharmaceutically effective dose. For a human of 60 kg, the daily dose of administration is usually 1-2000 mg, preferably 50-1000 mg. In determining a specific dose, such factors as the route of administration, the health condition of the patient and the like will also be considered, which are well-known to skilled physicians.

The above features mentioned in the present invention or those mentioned in the examples may be combined arbitrarily. All the features disclosed in this specification may be used with any composition form and the various features disclosed in this specification may be replaced with any alternative features that provide the same, equivalent, or similar purpose. Thus, unless otherwise specified, the features disclosed herein are merely general examples of equivalent or similar features.

### DETAILED DESCRIPTION

Various specific aspects, features, and advantages of the compounds, methods, and pharmaceutical compositions described above will be set forth in detail in the following description, which will make the content of the present invention very clear. It should be understood that the detailed description and examples below describe specific examples for reference only. After reading the description of the present invention, those skilled in the art can make various changes or modifications to the present invention, and such equivalents also fall within the scope of the present application defined herein.

In all the examples, ¹H-NMR spectra were recorded with a Varian Mercury 400 nuclear magnetic resonance spectrometer, and chemical shifts are represented by δ (ppm); silica gel for separation was 200-300 mesh silica gel if not specified, and the ratio of the eluents was a volume ratio.

The following abbreviations are used in the present invention: ACN for acetonitrile; AcOH for anhydrous acetic acid; Acetone for acetone; (Boc)₂O for di-*tert*-butyl dicarbonate; BPO for benzoyl peroxide; CDCl₃ for deuterated chloroform; CD₃I for deuterated iodomethane; DCM for dichloromethane; DIPEA for diisopropylethylamine; Diox for 1,4-dioxane; DMF for *N,N-*dimethylformamide; DMAP for 4-(dimethylamino)pyridine; DMSO for dimethyl sulfoxide; DPPAfor diphenylphosphoryl azide; DCE for 1,2-dichloroethane; EA for ethyl acetate; Et₂O for diethyl ether; EtMgBr for ethylmagnesium bromide; Flash for flash preparative medium-pressure liquid chromatography; Hexane for n-hexane; HOBt for 1-hydroxybenzotriazole; EDCI for 1-(3-dimethylaminopropyl)-3-ethylcarbodiimide hydrochloride; HCl for hydrochloric acid; HPLC for high-performance liquid chromatography; H₂ for hydrogen; h for hour; HOBt for 1-hydroxybenzotriazole; HCHO for formaldehyde; IPA for isopropanol; K₂CO₃ for potassium carbonate; KOAc for potassium acetate; K₃PO₄ for potassium phosphate; LiOH for lithium hydroxide; LC-MS for high-performance liquid chromatography-mass spectrometry; min for minute; MeOH for methanol; mL for milliliter; MS for mass spectrometry; MsOH for methanesulfonic acid; m-CPCA for m-chloroperoxybenzoic acid; n-BuLi for n-butyllithium; NaBH4 for sodium borohydride; n-BuOH for n-butanol; NH3/THF for a solution of ammonia in tetrahydrofuran; NMP for N-methylpyrrolidone; NMR for nuclear magnetic resonance; NBS for N-bromosuccinimide; NaBH(OAc)₃ for sodium triacetoxyborohydride; Oxone for potassium peroxymonosulfonate; Pd/C for palladium on carbon; Pd(PPh₃)₄ for tetrakis(triphenylphosphine)palladium; Pd₂(dba)₃ for tris(dibenzylideneacetone)dipalladium(0); PE for petroleum ether; Py for pyridine; pre-TLC for preparative silica gel thin-layer chromatography; Pd(dppf)₂Cl₂ for [1,1'-bis(diphenylphosphino)ferrocene]palladium dichloride dichloromethane complex; pre-HPLC for preparative high-performance liquid chromatography; PPTS for pyridinium p-toluenesulfonate; TFA for trifluoroacetic acid; T₃P for 1-propanephosphonic anhydride; TLC for thin-layer chromatography; Tf₂O for trifluoromethanesulfonic anhydride; Ti(i-PrO)₄ for tetraisopropyl titanate; TEA for triethylamine; *t*-BuOH for *tert*-butanol; Toluene for methylbenzene; XantPhos for 4,5-bis(diphenylphosphino)-9,9-dimethylxanthene; XPhos for 2-dicyclohexylphosphonium-2',4',6'-triisopropylbiphenyl.

### Synthesis of intermediate IntA:

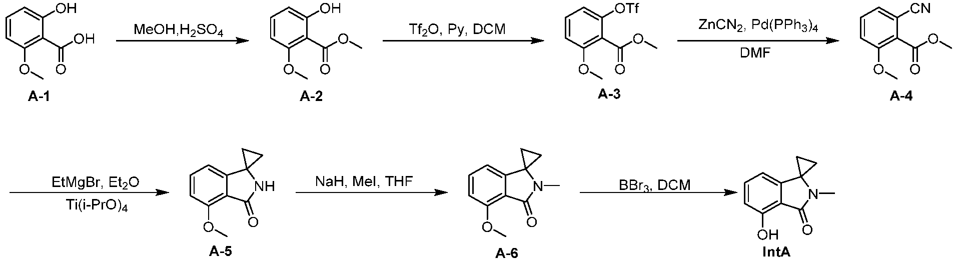

### Synthesis of A-2:

**A-1** (25.0 g, 149 mmol) and MeOH (300 mL) were added to a 1 L single-neck flask and conc H₂SO₄ (9.20 g, 93.8 mmol) was added dropwise at room temperature. The mixture was heated to 80 °C and stirred for 16 h under argon atmosphere. After the completion of the reaction as detected by LC-MS, the mixed solution was concentrated. H₂O (300 mL) was added to the residue. The resulting mixture was extracted with EA (600 mL), and liquid separation was performed. The organic phase was washed with a saturated sodium chloride solution (400 mL), dried over anhydrous sodium sulfate, filtered, and concentrated to give a white product (27.0 g, yield: 99.7%).

### Synthesis of A-3:

**A-2** (27.0 g, 148 mmol), pyridine (35.2 g, 445 mmol), and DCM (400 mL) were added to a 1 L three-necked flask.

The mixture was cooled to 0-5 °C in an ice bath, and Tf₂O (54.4 g, 193 mmol) was then added dropwise. After the dropwise addition was completed, the mixed solution was stirred at room temperature for 2 h. After the completion of the reaction as detected by LC-MS, H₂O (300 mL) was added to the mixed solution to quench the reaction, and liquid separation was performed. The aqueous phase was then extracted with DCM (400 mL). The organic phases were combined, washed with a saturated sodium chloride solution (400 mL), dried over anhydrous sodium sulfate, filtered, and concentrated. The residue was purified by column chromatography to give a brown oily product (25.0 g, yield: 53.7%).

ESI-MS m/z: 315.2 [M+H]⁺.

¹H NMR: (400MHz, CDCl₃) δ = 7.38 (t, *J*=8.4 Hz, 1H), 6.89 (dd, *J*=8.4, 10.9 Hz, 2H), 3.87 (s, 3H), 3.82 (s, 3H).

### Synthesis of A-4:

**A-3** (25.0 g, 79.6 mmol), Zn(CN)₂ (6.54 g, 55.7 mmol), and DMF (250 mL) were added to a 500 mL single-neck flask. After the mixture was purged with argon, Pd(PPh₃)₄ (9.19 g, 7.96 mmol) was added. The mixed solution was heated to 80 °C and stirred for 3 h under argon atmosphere. After the completion of the reaction as detected by LC-MS, H₂O (200 mL) was added to the mixed solution to quench the reaction. The resulting mixture was extracted with EA (400 mL × 2). The organic phase were combined, washed with a satured sodium chloride solution (400 mL × 2), and concentrated. The residue was purified by column chromatography to give a white solid product (9.00 g, yield: 59.2%).

ESI-MS m/z: 192.1 [M+H]⁺.

### Synthesis of A-5:

**A-4** (9.00 g, 47.1 mmol), Et₂O (120 mL), and Ti(i-PrO)₄ (14.7 g, 51.8 mmol) were added to a 500 mL three-necked flask. The mixture was cooled to 0-5 °C in an ice bath, and EtMgBr (3 M, 31.4 mL, 94.2 mmol) was then added dropwise. After the dropwise addition was completed, the mixed solution was stirred at room temperature for 1 h. After the completion of the reaction as detected by LC-MS, a saturated aqueous ammonium chloride solution (400 mL) was added to the mixed solution dropwise at 0-5 °C to quench the reaction. The resulting mixture was extracted with EA (200 mL × 2). The organic phases were combined, washed with a saturated sodium chloride solution (200 mL × 2), and concentrated. The residue was purified by column chromatography to give a brown solid product (2.50 g, yield: 28.1%).

ESI-MS m/z: 190.0 [M+H]⁺.

¹H NMR: (400MHz, DMSO-d₆) δ = 8.37 (s, 1H), 7.48 (t, *J*=7.9 Hz, 1H), 6.94 (d, *J*=8.1 Hz, 1H), 6.76 (d, *J*=7.5 Hz, 1H), 3.89 - 3.79 (m, 3H), 1.44 - 1.27 (m, 4H).

### Synthesis of A-6:

**A-5** (2.50 g, 13.2 mmol) and THF (60 mL) were added to a 250 mL three-necked flask. After the mixture was purged with argon, NaH (793 mg, 19.8 mmol, 60% purity) was added in batches at room temperature. After the addition, the mixed solution was stirred at room temperature for 0.5 h, and MeI (3.75 g, 26.4 mmol) was then added. The mixed solution was stirred at room temperature for another 1.5 h. After the completion of the reaction as detected by LC-MS, a saturated aqueous ammonium chloride solution (100 mL) was added to the mixed solution dropwise at 0-10 °C to quench the reaction. The resulting mixture was extracted with EA (100 mL × 2). The organic phases were combined, washed with a saturated sodium chloride solution (100 mL × 2), and concentrated. The residue was purified by column chromatography to give a brown solid product (2.00 g, yield: 74.5%).

ESI-MS m/z: 204.1 [M+H]⁺.

### Synthesis of IntA:

**A-6** (1.80 g, 8.86 mmol) and DCM (30 mL) were added to a 250 mL three-necked flask. The mixture was cooled to 0-5 °C in an ice bath under argon atmosphere, and BBr₃ (13.3 mL, 1 Min DCM, 13.3 mmol) was then added dropwise. After the dropwise addition was completed, the mixed solution was stirred at room temperature for 16 h. After the completion of the reaction as detected by LC-MS, the mixed solution was concentrated. DCM (100 mL) was added to the residue, followed by quenching with water (50 mL) at 0-10 °C. Sodium bicarbonate was then carefully added to adjust the pH to neutrality. Liquid separation was performed. The aqueous phase was then extracted with DCM (100 mL). The organic phases were combined, washed with a saturated sodium chloride solution (100 mL × 2), and concentrated. The residue was purified by column chromatography to give a brown solid product (1.40 g, yield: 83.5%).

ESI-MS m/z: 190.0 [M+H]⁺.

¹H NMR: (400MHz, DMSO-d₆) δ = 9.56 (s, 1H), 7.34 (t, J=7.8 Hz, 1H), 6.76 (d, J=8.0 Hz, 1H), 6.68 (d, J=7.5 Hz, 1H), 2.75 - 2.68 (m, 3H), 1.70 - 1.57 (m, 2H), 1.34 - 1.18 (m, 2H).

### Synthesis of intermediate IntB:

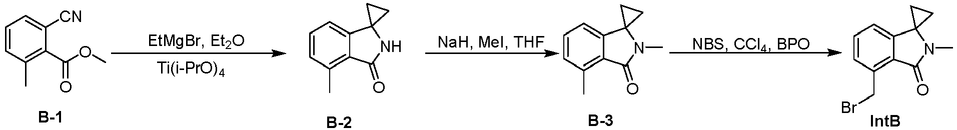

### Synthesis of B-2:

**B-1** (8 g, 45.7 mmol), Et₂O (120 mL), and Ti(i-PrO)₄ (14.3 g, 50.3 mmol) were added to a 250 mL three-necked flask. The mixture was cooled to 0-5 °C in an ice bath, and EtMgBr (3 M, 30.5 mL, 91.5 mmol) was then added dropwise. After the dropwise addition was completed, the mixed solution was stirred at room temperature for 1 h. After the completion of the reaction as detected by LC-MS, a saturated aqueous ammonium chloride solution (200 mL) was added to the mixed solution dropwise at 0-5 °C to quench the reaction. The resulting mixture was extracted with EA (200 mL × 2). The organic phases were combined, washed with a saturated sodium chloride solution (200 mL × 2), and concentrated. The residue was purified by column chromatography to give a brown solid product (1.6 g, yield: 20.1%).

ESI-MS m/z: 174.0 [M+H]⁺.

### Synthesis of B-3:

**B-2** (1.6 g, 9.24 mmol) and THF (48 mL) were added to a 250 mL three-necked flask. After the mixture was purged with argon, NaH (554 mg, 13.86 mmol, 60% purity) was added in batches at room temperature. After the addition, the mixed solution was stirred at room temperature for 0.5 h, and MeI (2.63 g, 18.5 mmol) was then added. The mixed solution was stirred at room temperature for another 1.5 h. After the completion of the reaction as detected by LC-MS, a saturated aqueous ammonium chloride solution (50 mL) was added to the mixed solution dropwise at 0-10 °C to quench the reaction. The resulting mixture was extracted with EA (50 mL × 2). The organic phases were combined, washed with a saturated sodium chloride solution (50 mL × 2), and concentrated. The residue was purified by column chromatography to give a brown solid product (1.42 g, yield: 82.1%).

ESI-MS m/z: 188.1 [M+H]⁺.

### Synthesis of IntB:

**B-3** (1.4 g, 7.48 mmol), benzoyl peroxide (194 mg, 0.8 mmol), and carbon tetrachloride (42 mL) were added to a 250 mL single-neck flask. After the mixture was purged with argon, NBS (1.46 g, 8.2 mmol) was added at room temperature. After the addition, the mixed solution was heated to 80 °C and stirred for 16 h. After the basic completion of the reaction as detected by LC-MS, the mixed solution was concentrated, and the residue was purified by Flash to give a light brown solid product (1.02 g, yield: 51.3%).

ESI-MS m/z: 266.1/268.1[M+H]⁺.

### Synthesis of intermediate IntC:

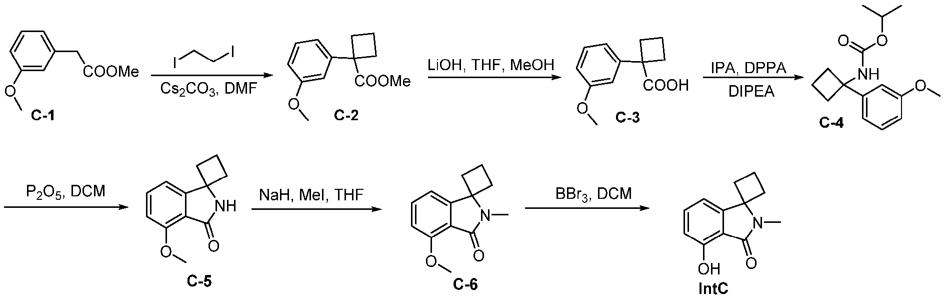

### Synthesis of C-2:

**C-1** (18 g, 0.10 mol), DMF (400 mL), cesium carbonate (81.5 g, 0.25 mol), and 1,2-diiodoethane (33.8 g, 0.12 mol) were added to a 1 L single-neck flask. The mixed solution was stirred at room temperature for 20 h under argon atmosphere. After the completion of the reaction as detected by LC-MS, EA (200 mL)/PE (400 mL) and water (400 mL) were added to the mixed solution. The mixture was stirred, and liquid separation was performed. The organic phase was washed twice with a saturated sodium chloride solution (400 mL × 2), dried over anhydrous sodium sulfate, filtered, and concentrated to dryness to give a pale brown oily product (17.5 g, yield: 79.5%).

ESI-MS m/z: 221.2 [M+H]⁺.

### Synthesis of C-3:

**C-2** (17.5 g, 79.5 mmol), THF (200 mL), MeOH (100 mL), water (100 mL), and lithium hydroxide monohydrate (16.8 g, 0.4 mol) were added to a 1 L single-neck flask. The mixed solution was stirred at room temperature for 20 h under argon atmosphere. After the completion of the reaction as detected by LC-MS, the mixed solution was concentrated until there was about half of the volume left, and the pH was then adjusted to 3-4 with 2 N HCl. The resulting mixture was extracted twice with EA (200 mL × 2). The organic phases were combined, washed with a saturated sodium chloride solution (200 mL × 2), dried over anhydrous sodium sulfate, filtered, and concentrated to dryness to give a pale brown oily product (14.3 g, yield: 88.1%).

ESI-MS m/z: 207.2 [M+H]⁺.

### Synthesis of C-4:

**C-3** (14.1 g, 69 mmol), isopropanol (200 mL), and DIPEA (22.3 g, 172.5 mmol) were added to a 1 L three-necked flask. The mixed solution was purged with argon, and diphenylphosphoryl azide (22.8 g, 82.8 mmol) was then added dropwise in an ice bath. After the dropwise addition was completed, the mixed solution was stirred at room temperature for 20 min. The mixed solution was then heated to 50 °C and stirred for another 20 h. After the completion of the reaction as detected by LC-MS, the mixture was concentrated. EA (200 mL) and water (200 mL) were added to the residue. The resulting mixture was stirred, and liquid separation was performed. The aqueous phase was then extracted with EA (50 mL). The organic phases were combined, washed with a saturated sodium chloride solution(100 mL × 2), and concentrated. The residue was purified by column chromatography to give a colorless oily product (7.6 g, yield: 41.8%)

ESI-MS m/z: 264.3 [M+H]⁺.

### Synthesis of C-5:

**C-4** (7.6 g, 28.86 mmol), DCM (200 mL), and P₂O₅ (16.3 g, 115 mmol) were added to a 500 mL single-neck flask. The mixed solution was heated to 50 °C and stirred for 20 h under argon atmosphere. After the completion of the reaction as detected by LC-MS, the mixed solution was cooled and then poured slowly into ice water (200 mL). After the mixed solution was stirred for 30 min, liquid separation was performed. The organic phase was then washed twice with a saturated sodium chloride solution (100 mL × 2) and concentrated. The residue was purified by column chromatography to give a pale brown oily product (3.2 g, yield: 54.6%)

ESI-MS m/z: 204.2 [M+H]⁺.

### Synthesis of C-6:

**C-5** (3.2 g,15.7 mmol) and THF (65 mL) were added to a 250 mL three-necked flask. After the mixture was purged with argon, NaH (793 mg, 19.8 mmol, 60%) was added in batches at room temperature. After the addition, the mixed solution was stirred at room temperature for 0.5 h, and MeI (3.75 g, 26.4 mmol) was then added. The mixed solution was stirred at room temperature for another 1.5 h. After the completion of the reaction as detected by LC-MS, a saturated aqueous ammonium chloride solution (100 mL) was added to the mixed solution dropwise at 0-10 °C to quench the reaction. The resulting mixture was extracted with EA (100 mL × 2). The organic phases were combined, washed with a saturated sodium chloride solution (100 mL × 2), and concentrated. The residue was purified by column chromatography to give a brown solid product (2.2 g, yield: 64.6%).

ESI-MS m/z: 218.3 [M+H]⁺.

### Synthesis of IntC:

**C-6** (2.2 g, 10.12 mmol) and DCM (30 mL) were added to a 250 mL three-necked flask. The mixture was cooled to 0-5 °C in an ice bath under argon atmosphere, and BBr₃ (20.2 mL, 1 M in DCM, 20.2 mmol) was then added dropwise. After the dropwise addition was completed, the mixed solution was stirred at room temperature for 16 h. After the completion of the reaction as detected by LC-MS, the mixed solution was concentrated. DCM (100 mL) was added to the residue, followed by quenching with water (50 mL) at 0-10 °C. Sodium bicarbonate was then carefully added to adjust the pH to neutrality. Liquid separation was performed. The aqueous phase was then extracted with DCM (100 mL). The organic phases were combined, washed with a saturated sodium chloride solution (100 mL × 2), and concentrated. The residue was purified by column chromatography to give a brown solid product (1.45 g, yield: 70.5%).

ESI-MS m/z: 204.2 [M+H]⁺.

### Example 1. Synthesis ofN-methyl-4-((4-((2'-methyl-3'-oxospiro[cyclopropane-1,1'-isoindolin]-4'-yl)oxy)-5-(trifluoromethyl)pyrimidin-2-yl)amino)benzamide (Compound 1)

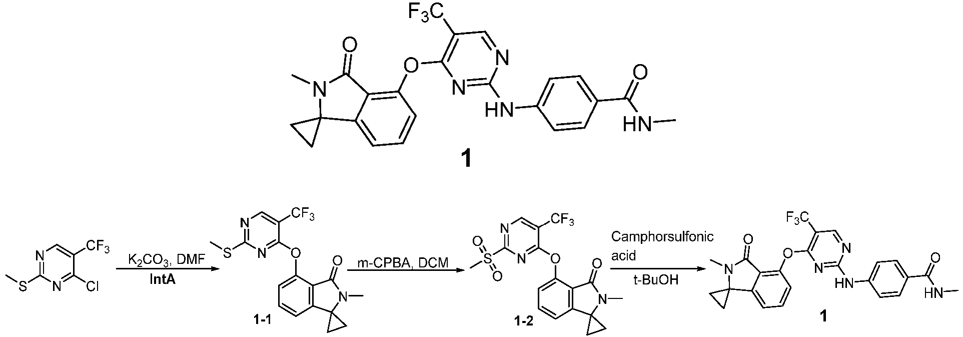

### Synthesis of 1-1:

**IntA** (1.19 g, 6.29 mmol) and 4-chloro-2-(methylthio)-5-(trifluoromethyl)pyrimidine (1.51 g, 6.60 mmol) were dissolved in N,N-dimethylformamide (15 mL), and anhydrous potassium carbonate (2.17 g, 15.72 mmol) was added. The mixed solution was stirred at room temperature overnight. After the completion of the reaction as detected by LC-MS, the reaction solution was poured into water (150 mL), and a large amount of solid precipitated. The resulting mixture was stirred at room temperature for 30 min and then filtered. The filter cake was washed with water and dried under vacuum to give a white solid crude product (2.39 g, yield: 100%).

ESI-MS m/z: 382.1 [M+H]⁺.

### Synthesis of 1-2:

The crude product **1-1** (2.39 g, 6.29 mmol) described above was dissolved in DCM (25 mL), and m-CPBA (2.12 g, 12.3 mmol) was added at room temperature. The mixed solution was stirred at room temperature for 1 h under argon atmosphere, and m-CPBA (2.12 g, 12.3 mmol) was then added. The mixed solution was stirred at room temperature for another 2 h. After the completion of the reaction as detected by LC-MS, the resulting mixture was filtered. DCM (100 mL) and a saturated sodium thiosulfate solution (50 mL) were added to the filtrate, and the mixture was stirred at room temperature for 20 min. Liquid separation was performed. The organic phase was then washed with a saturated sodium thiosulfate solution (50 mL) and a saturated sodium bicarbonate solution (50 mL), dried over anhydrous sodium sulfate, filtered, and concentrated. The residue was purified by column chromatography on 100-200 mesh silica gel (elution gradient PE/EA = 2:1 → DCM/EA = 2:1) to give a white solid product (1.67 g, yield: 64.2%)

ESI-MS m/z: 414.1 [M+H]⁺.

### Synthesis of compound 1:

The compound **1-2** described above (69 mg, 0.167 mmol) and 4-amino-*N*-methylbenzamide (30 mg, 0.2 mmol) were dissolved in super-dry *tert*-butanol (5 mL), and camphorsulfonic acid (46 mg, 0.2 mmol) was then added. The mixed solution was heated at reflux for 24 h under argon atmosphere. After the completion of the reaction as detected by LC-MS, a saturated sodium bicarbonate solution (5 mL) was added to the reaction solution to quench the reaction. The resulting mixture was concentrated under reduced pressure. The residue was purified by Flash and lyophilized to give a white solid product (17 mg, yield: 21.1%)

ESI-MS m/z: 484.1 [M+H]⁺.

### Example 2. Synthesis of 3-methoxy-4-((4-((2'-methyl-3'-oxospiro[cyclopropane-1,1'-isoindolin]-4'-yl)oxy)-5-(trifluoromethyl)pyrimidin-2-yl)amino)-N-(1-methylpiperidin-4-yl)benzamide (Compound 3)

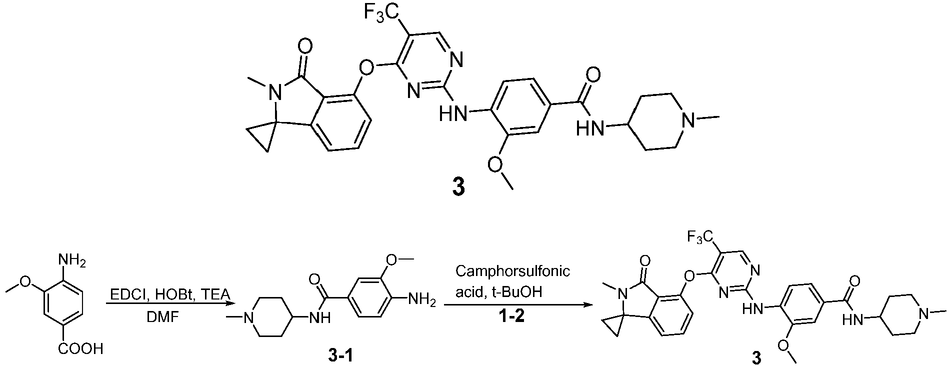

### Synthesis of 3-1:

4-Amino-3-methoxybenzoic acid (500 mg, 3.0 mmol) and 1-methylpiperidin-4-amine (350 mg, 3.0 mmol) were dissolved in DMF (10 mL), and HOBt (607 mg, 4.5 mmol), EDCI (863 mg, 4.5 mmol), and TEA (900 mg, 8.9 mmol) were added. The mixed solution was stirred at room temperature for 16 h. After the completion of the reaction as detected by LC-MS, water (50 mL) was added to the reaction solution, and the resulting mixture was extracted with ethyl acetate (50 mL × 3). The organic phases were combined, washed twice with a saturated sodium chloride solution (50 mL × 2), dried over anhydrous sodium sulfate, filtered, and concentrated under reduced pressure to give a white solid product (708 mg, yield: 90.0%).

ESI-MS m/z: 264.2 [M+H]⁺.

### Synthesis of compound 3:

Compounds **1-2** (138 mg, 334 µmol) and **3-1** (88 mg, 334 µmol) were dissolved in super-dry *tert*-butanol (5 mL), and camphorsulfonic acid (92 mg, 0.4 mmol) was then added. The mixed solution was heated at reflux for 24 h under argon atmosphere. After the completion of the reaction as detected by LC-MS, a saturated sodium bicarbonate solution (5 mL) was added to the reaction solution to quench the reaction. The resulting mixture was concentrated under reduced pressure. The residue was purified by Flash and lyophilized to give a white solid product (33.4 mg, yield: 16.3%).

ESI-MS m/z: 564.3 [M+H]⁺.

¹H-NMR (400 MHz, CDCl₃) δ 8.55 (d, *J =* 8.4 Hz, 1H), 7.94 (s, 1H), 7.64 (t, *J =* 7.8 Hz, 1H), 7.25 (s, 2H), 7.21 (d, *J* = 8.0 Hz, 1H), 7.09 (d, *J* = 7.7 Hz, 1H), 6.85 (s, 1H), 6.30 - 6.21 (m, 1H), 4.07 (m, 1H), 3.88 (s, 3H), 3.17 (d, *J*= 12.0 Hz, 2H), 2.69 (s, 3H), 2.51 (s, 3H), 2.46 (t, *J*= 11.8 Hz, 2H), 2.08 (d, *J =* 12.9 Hz, 2H), 1.88 (q, *J =* 12.0 Hz, 2H), 1.60 (t, *J =* 7.0 Hz, 2H), 1.44 (t, *J* = 4.1 Hz, 2H).

The target **compounds 2, 4, and 5** in Table 1 were obtained with reference to a synthetic method similar to that in Example 2 using different starting materials.

**Table 1**

| **Compound** | **Structural formula** | **MS: [M+H]⁺** | **Compound** | **Structural formula** | **MS: [M+H]⁺** |
|---|---|---|---|---|---|
| **2** | 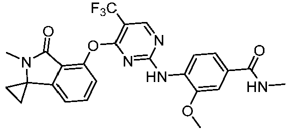 | **514.1** | **4** | 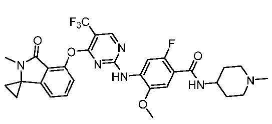 | **615.2** |
| **5** | 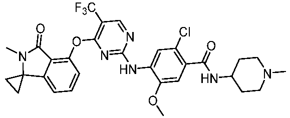 | **631.2** | | | |

### Example 3. Synthesis of 2-fluoro-5-methoxy-4-((4-((2'-methyl-3'-oxospiro[cyclopropane-1,1'-isoindolin]-4'-yl)oxy)-5-(trifluoromethyl)pyrimidin-2-yl)amino)-N-(7-methyl-7-azaspiro[3.5]nonan-2-yl)benzamide (Compound 8)

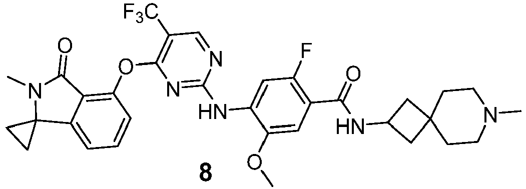

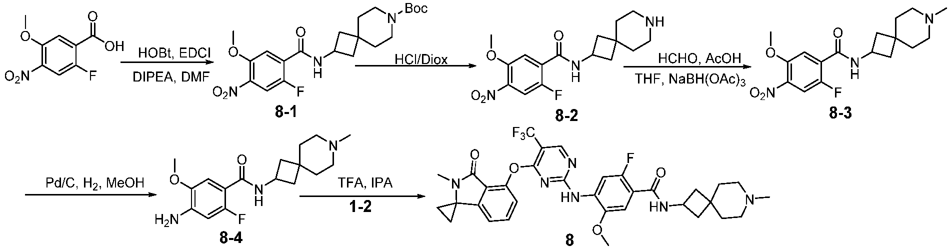

### Synthesis of 8-1:

**2-Fluoro-5-methoxy-4-nitrobenzoic acid** (610 mg, 2.08 mmol), ***tert*-butyl 2-amino-7-azaspiro[3.5]nonane-7-carboxylate** (500 mg, 2.08 mol), HOBt (420 mg, 3.1 mmol), EDCI (598 mg, 3.1 mmol), and DIPEA (1.1 mL) were added to DMF (10 mL). The mixture was purged with argon and then stirred at room temperature for 20 h. After the completion of the reaction as detected by LC-MS, water (20 mL) was added to the mixed solution. The mixture was stirred at room temperature for 30 min and then filtered. The filter cake was washed with water and dried under vacuum to give a product (930 mg, yield: >100%).

ESI-MS m/z: 438.2 [M+H]⁺.

### Synthesis of 8-2:

The **8-1** (930 mg, crude, 2.08 mmol) described above was added to EA (10 mL). After complete dissolution, 4 M HCl/Diox (10 mL, 40 mmol) was added. The mixture was stirred at room temperature for 3 h. After the completion of the reaction as detected by LC-MS, the mixture was concentrated to dryness. EA (20 mL) and a saturated sodium bicarbonate solution (20 mL) were added to the residue. The resulting mixture was stirred, and liquid separation was performed. The aqueous phase was then extracted twice with EA (20 mL × 2). The organic phases were combined, washed with a saturated sodium chloride solution, dried over anhydrous sodium sulfate, and filtered. The filtrate was concentrated to dryness to give a brownish-yellow oily product (508 mg, yield: 72.4%).

ESI-MS m/z: 338.1 [M+H]⁺.

### Synthesis of 8-3:

The compound **8-2** described above (508 mg, 1.51 mmol) was added to THF (10 mL). Glacial acetic acid (136 mg, 2.265 mmol) and an aqueous formaldehyde solution (147 mg, 37%, 1.8 mmol) were added at room temperature. The mixed solution was stirred at room temperature for 30 min. NaBH(OAc)₃ (479 mg, 2.26 mmol) was then added, and the mixed solution was stirred at room temperature for another 20 h. After the completion of the reaction as detected by LC-MS, EA (20 mL) and a saturated sodium bicarbonate solution (20 mL) were added to the mixed solution. The mixed solution was stirred for 20 min, and liquid separation was then performed. The aqueous phase was extracted twice with EA (20 mL × 2). The organic phases were combined, washed with a saturated sodium chloride solution, dried over anhydrous sodium sulfate, and filtered. The filtrate was concentrated to dryness to give a brown oily crude product (630 mg, yield: >100%).

ESI-MS m/z: 352.1 [M+H]⁺.

### Synthesis of 8-4:

The **8-3** (630 mg, crude, 1.51 mmol) described above and 10% wet Pd/C (100 mg, containing 50%-55% water) were added to MeOH (20 mL). The mixed solution was purged with hydrogen three times, and then vigorously stirred at room temperature under normal pressure for 20 h. After the completion of the reaction as detected by LC-MS, the mixed solution was filtered through diatomite. The filter cake was rinsed with MeOH, and the filtrate was concentrated to dryness to give a white solid product (433 mg, yield: 89%).

ESI-MS m/z: 322.1 [M+H]⁺.

### Synthesis of compound 8:

The compound **8-4** described above (50 mg, 0.156 mmol) and 1-2 (59 mg, 0.142 mmol) were added to isopropanol (5 mL). TFA (18 mg, 0.156 mmol) was added at room temperature. The mixed solution was purged with argon, heated to 80 °C, and stirred for 3 h. After the basic completion of the reaction as detected by LC-MS, the mixture was concentrated, and the residue was purified by Flash and lyophilized to give an off-white solid product (9 mg, yield: 9.7%).

ESI-MS m/z: 655.3 [M+H]⁺.

The target **compounds 6-7 and compounds 9-34** in Table 2 were obtained with reference to a synthetic method similar to that in Example 3 using different starting materials.

**Table 2**

| **Compound** | **Structural formula** | **MS: [M+H]⁺** | **Compound** | **Structural formula** | **MS: [M+H]⁺** |
|---|---|---|---|---|---|
| **6** | 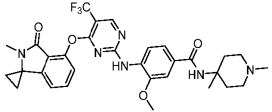 | **611.2** | 7 | 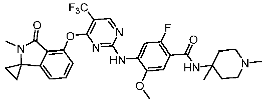 | **629.2** |
| **9** | 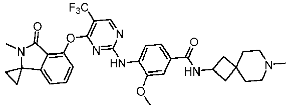 | **637.2** | **10** | 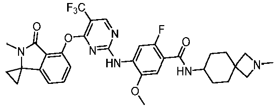 | **655.2** |
| **11** | 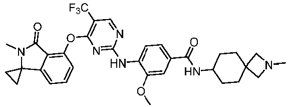 | **637.2** | **12** | 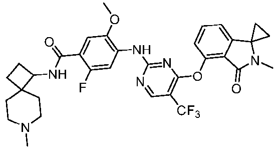 | **655.2** |
| **13** | 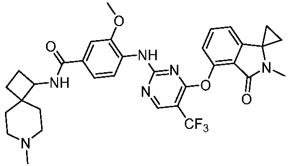 | **637.2** | **14** | 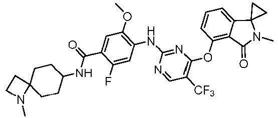 | **655.2** |
| **15** | 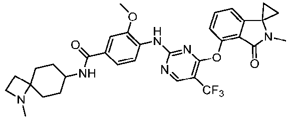 | **637.2** | **16** | 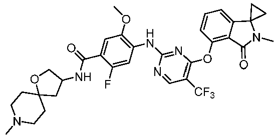 | **671.2** |
| **17** | 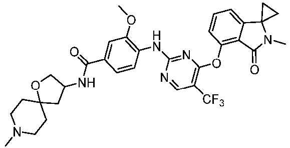 | **653.2** | **18** | 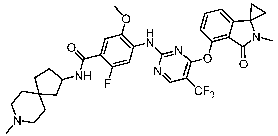 | **669.2** |
| **19** | 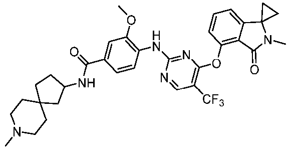 | **651.2** | **20** | 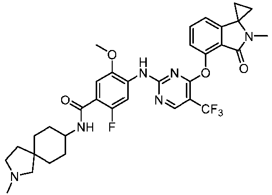 | **669.2** |
| **21** | 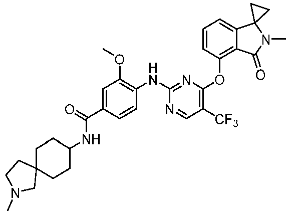 | **651.2** | **22** | 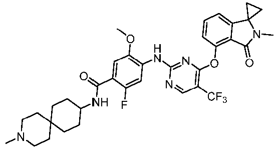 | **683.2** |
| **23** | 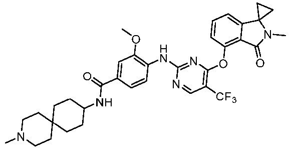 | **665.3** | **24** | 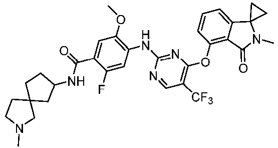 | **655.2** |
| **25** | 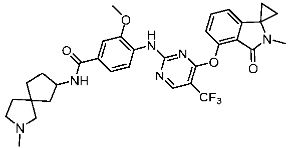 | **637.2** | **26** | 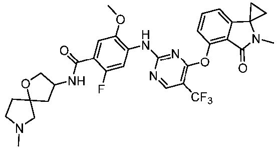 | **657.2** |
| **27** | 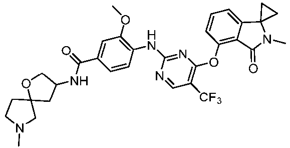 | **639.2** | **28** | 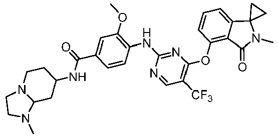 | **638.2** |
| **29** | 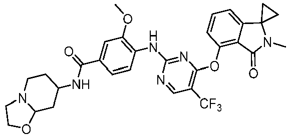 | **625.2** | **30** | 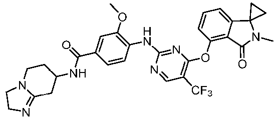 | **622.2** |
| **31** | 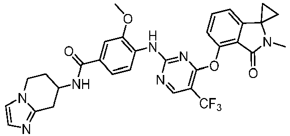 | **620.2** | **32** | 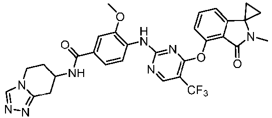 | **621.2** |
| **33** | 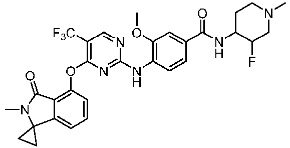 | **615.2** | **34** | 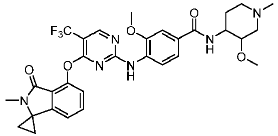 | **627.2** |

### Example 4. 3-Methoxy-4-((4-((2'-methyl-3'-oxospiro[cyclopropane-1,1'-isoindolin]-4'-yl)oxy)-5-(trifluoromethyl)pyrimidin-2-yl)amino)-N-(piperidin-4-yl)benzamide (Synthesis of Compound 35)

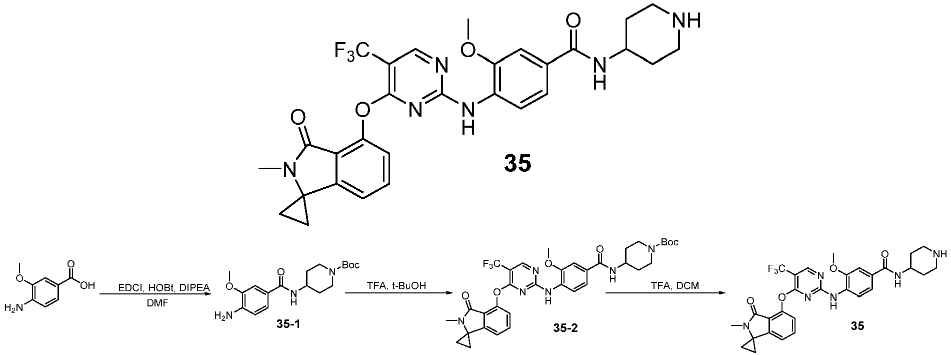

### Synthesis of 35-1:

**3-Methoxy-4-nitrobenzoic acid** (835 mg, 5.0 mmol), ***tert*-butyl 4-aminopiperidine-1-carboxylate** (1.0 g, 5.0 mol), HOBt (1.01 g, 7.5 mmol), EDCI (1.45 g, 7.5 mmol), and DIPEA (2.66 mL) were added to DMF (20 mL). The mixture was purged with argon and then stirred at room temperature for 20 h. After the completion of the reaction as detected by LC-MS, water (40 mL) was added to the mixed solution. The mixture was stirred at room temperature for 30 min and then filtered. The filter cake was washed with water and dried under vacuum to give a product (1.59 g, yield: 91%).

ESI-MS m/z: 350.2 [M+H]⁺.

### Synthesis of 35-2:

The compound **35-1** described above (700 mg, 2.0 mmol) and **1-2** (744 mg, 1.8 mmol) were added to super-dry *tert*-butanol (20 mL). TFA (228 mg, 2.0 mmol) was added at room temperature. The mixed solution was purged with argon, heated to 80 °C, and stirred for 20 h. After the basic completion of the reaction as detected by LC-MS, the mixture was concentrated, and the residue was purified by Flash and lyophilized to give an off-white solid product (319 mg, yield: 26%).

ESI-MS m/z: 683.3 [M+H]⁺.

### Synthesis of compound 35:

The compound **35-2** described above (310 mg, 0.454 mmol) was added to DCM (10 mL). TFA (456 mg, 4.0 mmol) was added at room temperature. The mixed solution was stirred at room temperature for 5 h under argon atmosphere. After the basic completion of the reaction as detected by LC-MS, a saturated sodium bicarbonate solution (10 mL) was added to the mixture to quench the reaction, and liquid separation was performed. The aqueous phase was extracted with DCM (10 mL × 3). The organic phases were combined, washed with a saturated sodium chloride solution (10 mL), dried over anhydrous sodium sulfate, filtered, and concentrated to dryness to give a crude product (280 mg, yield: 107%). About 30 mg of the crude product was taken for pre-TLC (DCM:MeOH = 10:1) purification to give a white solid product (11 mg).

ESI-MS m/z: 582.2 [M+H]⁺.

### Example 5. 3-Methoxy-4-((4-((2'-methyl-3'-oxospiro[cyclopropane-1,1'-isoindolin]-4'-yl)oxy)-5-(trifluoromethyl)pyrimidin-2-yl)amino)-N-(piperidin-4-yl)benzamide (Synthesis of Compound 36)

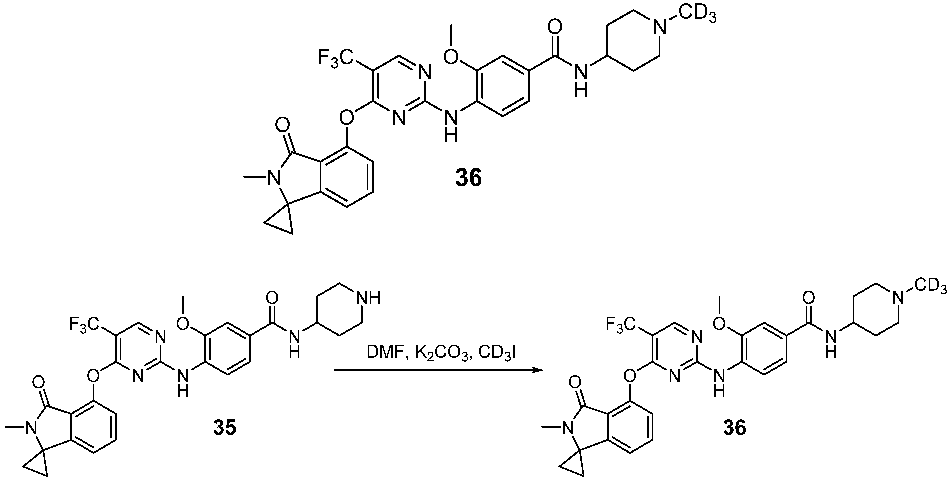

### Synthesis of compound 36:

The compound **35** described above (50 mg, crude, 0.086 mmol) and anhydrous potassium carbonate (36 mg, 0.26 mmol) were added to DMF (2 mL). CD₃I (12 mg, 0.083 mmol) was added at room temperature. The mixed solution was stirred at room temperature for 2 h under argon atmosphere. After the basic completion of the reaction as detected by LC-MS, the mixed solution was purified by Flash and lyophilized to give a white solid product (16 mg, yield: 31%). ESI-MS m/z: 600.2 [M+H]⁺.

¹H-NMR (400 MHz, CDCl₃) δ 8.54 (d, *J =* 8.4 Hz, 1H), 7.94 (s, 1H), 7.61 (t, *J =* 7.8 Hz, 1H), 7.25 (s, 2H), 7.16 (d, *J =* 8.0 Hz, 1H), 7.06 (d, *J* = 7.7 Hz, 1H), 6.85 (s, 1H), 6.31 - 6.24 (m, 1H), 4.07 (m, 1H), 3.86 (s, 3H), 3.19 (d, *J* = 12.0 Hz, 2H), 2.63 (s, 3H), 2.46 (t, *J =* 11.8 Hz, 2H), 2.12 (d, *J* = 12.9 Hz, 2H), 1.81 (q, *J* = 12.0 Hz, 2H), 1.62 (t, *J =* 7.0 Hz, 2H), 1.45 (t, *J =* 4.1 Hz, 2H).

The target **compounds 37-48** in Table 3 were obtained with reference to synthetic methods similar to those in Example 4 and Example 5 using different starting materials.

**Table 3**

| **Compound** | **Structural formula** | **MS: [M+H]⁺** | **Compound** | **Structural formula** | **MS: [M+H]⁺** |
|---|---|---|---|---|---|
| **37** | 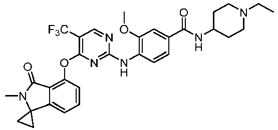 | **611.2** | **38** | 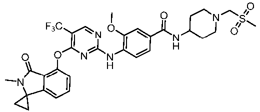 | **675.2** |
| **39** | 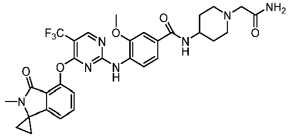 | **640.2** | **40** | 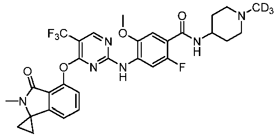 | **618.2** |
| **41** | 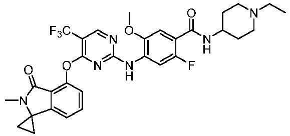 | **629.2** | **42** | 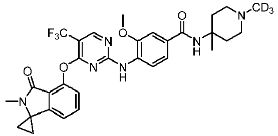 | **614.2** |
| **43** | 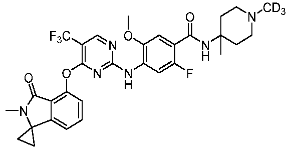 | **632.2** | **44** | 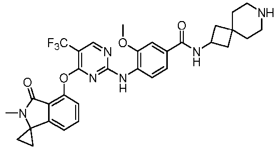 | **623.2** |
| **45** | 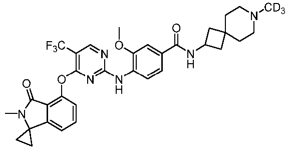 | **640.2** | **46** | 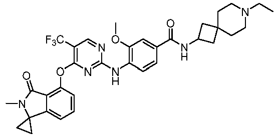 | **651.2** |
| **47** | 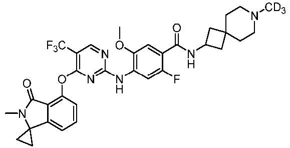 | **658.2** | **48** | 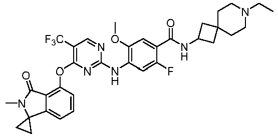 | **669.2** |

The target **compounds 49-73 and compounds 77-91** in Table 4 were obtained with reference to synthetic methods similar to those in Example 2 and Example 3 using different starting materials.

**Table 4**

| **Compound** | **Structural formula** | **MS: [M+H]⁺** | **Compound** | **Structural formula** | **MS: [M+H]⁺** |
|---|---|---|---|---|---|
| **49** | 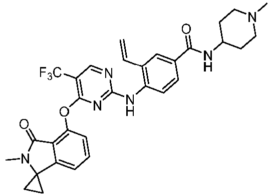 | **593.2** | **50** | 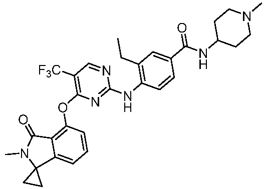 | **595.2** |
| **51** | 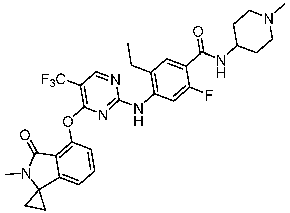 | **613.2** | **52** | 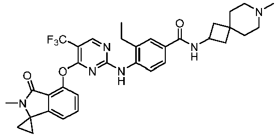 | **635.3** |
| **53** | 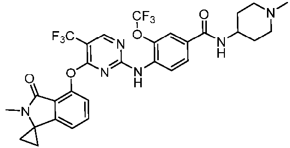 | **651.2** | **54** | 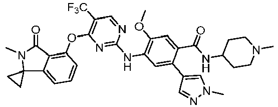 | **677.3** |
| **55** | 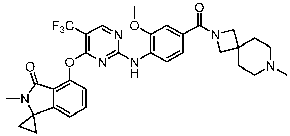 | **623.2** | **56** | 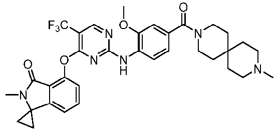 | **651.3** |
| **57** | 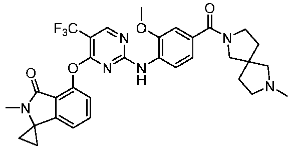 | **623.2** | **58** | 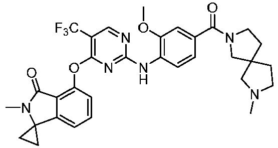 | **623.2** |
| **59** | 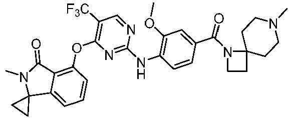 | **623.2** | **60** | 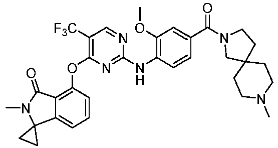 | **637.2** |
| **61** | 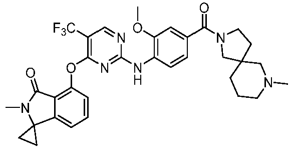 | **637.2** | **62** | 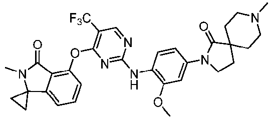 | **623.2** |
| **63** | 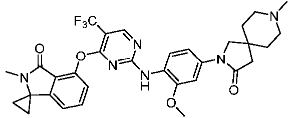 | **623.2** | **64** | 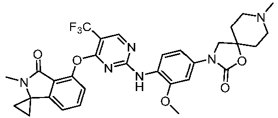 | **625.2** |
| **65** | 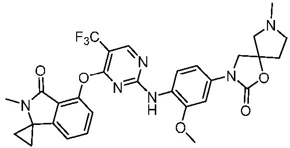 | **611.2** | **66** | 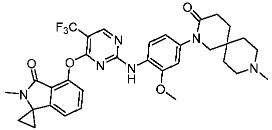 | **637.2** |
| **67** | 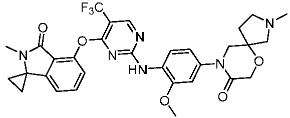 | **625.2** | **68** | 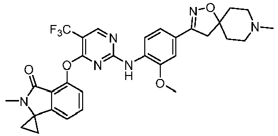 | **609.2** |
| **69** | 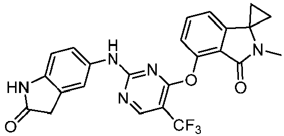 | **482.1** | **70** | 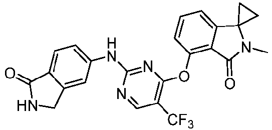 | **482.2** |
| **71** | 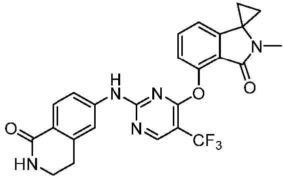 | **496.2** | **72** | 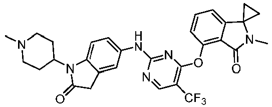 | **579.2** |
| **73** | 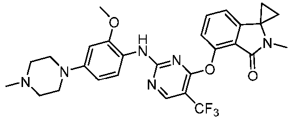 | **555.2** | **77** | 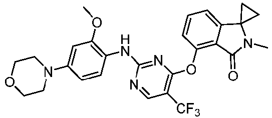 | **542.2** |
| **78** | 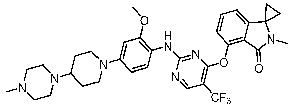 | **638.3** | **79** | 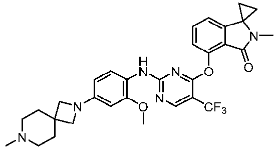 | **595.2** |
| **80** | 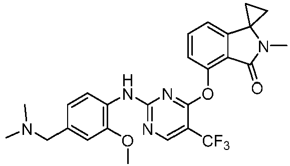 | **514.2** | **81** | 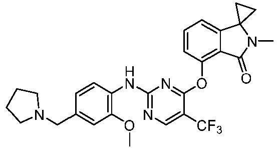 | **540.2** |
| **82** | 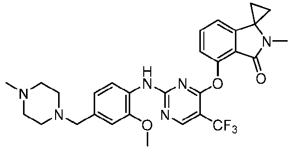 | **569.2** | **83** | 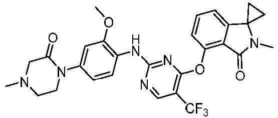 | **569.2** |
| **84** | 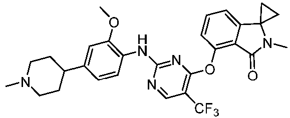 | **554.2** | **85** | 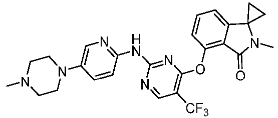 | **526.2** |
| **86** | 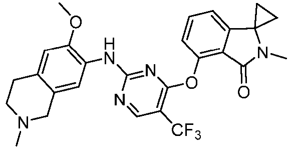 | **526.2** | **87** | 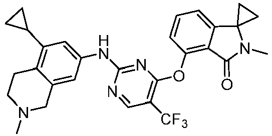 | **546.2** |
| **88** | 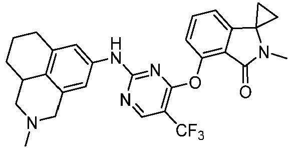 | **536.2** | **89** | 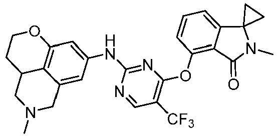 | **538.2** |
| **90** | 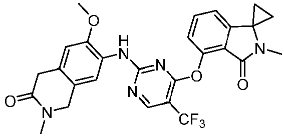 | **540.2** | **91** | 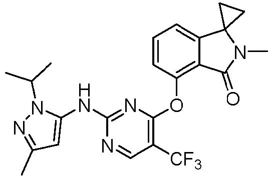 | **473.2** |

### Example 6. Synthesis of 4'-((2-((2-methoxy-4-(piperazin-1-yl)phenyl)amino)-5-(trifluoromethyl)pyrimidin-4-yl)oxy)-2'-methylspiro[cyclopropane-1,1'-isoindolin]-3'-one (Compound 74)

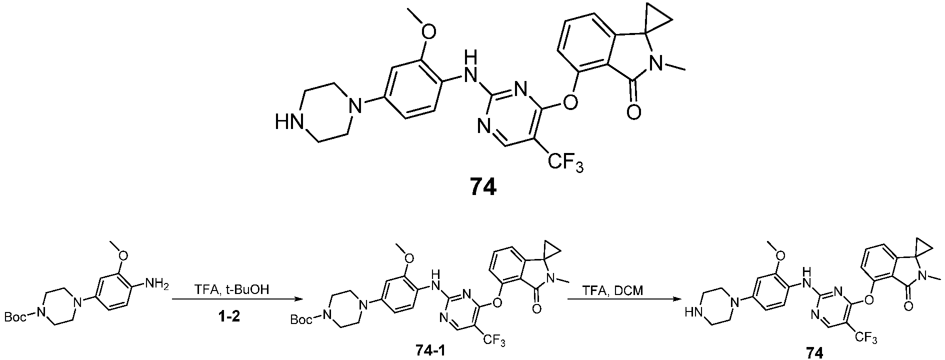

### Synthesis of 74-1:

*tert*-Butyl 4-(4-amino-3-methoxyphenyl)piperazine-1-carboxylate (614 mg, 2.0 mmol) and 1-2 (744 mg, 1.8 mmol) were added to super-dry tert-butanol (20 mL). TFA (228 mg, 2.0 mmol) was added at room temperature. The mixed solution was purged with argon, heated to 80 °C, and stirred for 20 h. After the basic completion of the reaction as detected by LC-MS, the mixture was concentrated, and the residue was purified by Flash and lyophilized to give an off-white solid product (623 mg, yield: 54.0%).

ESI-MS m/z: 641.2 [M+H]⁺.

### Synthesis of compound 74:

The compound **74-1** described above (600 mg, 0.936 mmol) was added to DCM (20 mL). TFA (1.14 g, 10 mmol) was added at room temperature. The mixed solution was stirred at room temperature for 5 h under argon atmosphere. After the basic completion of the reaction as detected by LC-MS, a saturated sodium bicarbonate solution (20 mL) was added to the mixture to quench the reaction, and liquid separation was performed. The aqueous phase was extracted with DCM (20 mL × 3). The organic phases were combined, washed with a saturated sodium chloride solution (10 mL), dried over anhydrous sodium sulfate, filtered, and concentrated to dryness to give a crude product (412 mg, yield: 81.4%). About 30 mg of the crude product was taken for pre-TLC (DCM:MeOH = 10: 1) purification to give a white solid product (9 mg).

ESI-MS m/z: 541.2 [M+H]⁺.

### Example 7. Synthesis of 2-(4-(3-methoxy-4-((4-((2'-methyl-3'-oxospiro[cyclopropane-1,1'-isoindolin]-4'-yl)oxy)-5-(trifluoromethyl)pyrimidin-2-yl)amino)phenyl)piperazin-1-yl)-N,N-dimethylacetamide (Compound 75)

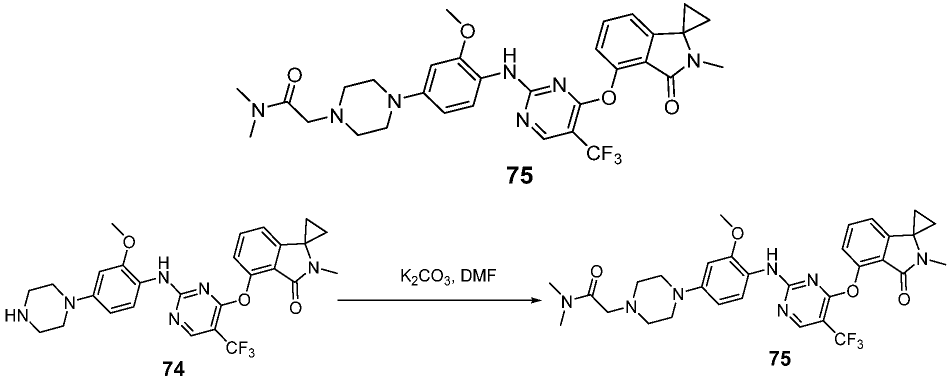

### Synthesis of compound 75:

The compound **74** described above (54 mg, crude, 0.1 mmol) and anhydrous potassium carbonate (36 mg, 0.26 mmol) were added to DMF (2 mL). 2-Chloro-*N,N*-dimethylacetamide (10 mg, 0.082 mmol) was added at room temperature. The mixed solution was stirred at room temperature for 2 h under argon atmosphere. After the basic completion of the reaction as detected by LC-MS, the mixed solution was purified by Flash and lyophilized to give a white solid product (11 mg, yield: 21%).

ESI-MS m/z: 626.2 [M+H]⁺.

### Example 8. Synthesis of 4'-((2-((2-methoxy-4-(4-(2-methoxyethyl)piperazin-1-yl)phenyl)amino)-5-(trifluoromethyl)pyrimidin-4-yl)oxy)-2'-methylspiro[cyclopropane-1,1'-isoindolin]-3'-one (Compound 76)

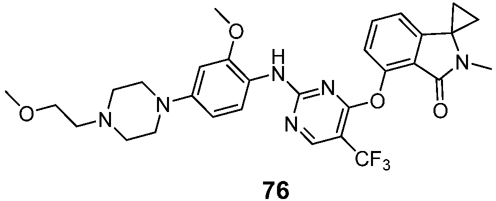

Compound 76 was obtained with reference to the synthetic method of Example 75 using **compound 74** and 1-bromo-2-methoxyethane as starting materials.

### Example 9. Synthesis of 4'-((2-((5,6-dihydro-4H-pyrrolo[1,2-b]pyrazol-2-yl)amino)-5-(trifluoromethyl)pyrimidin-4-yl)oxy)-2'-methylspiro[cyclopropane-1,1'-isoindolin]-3'-one (Compound 92)

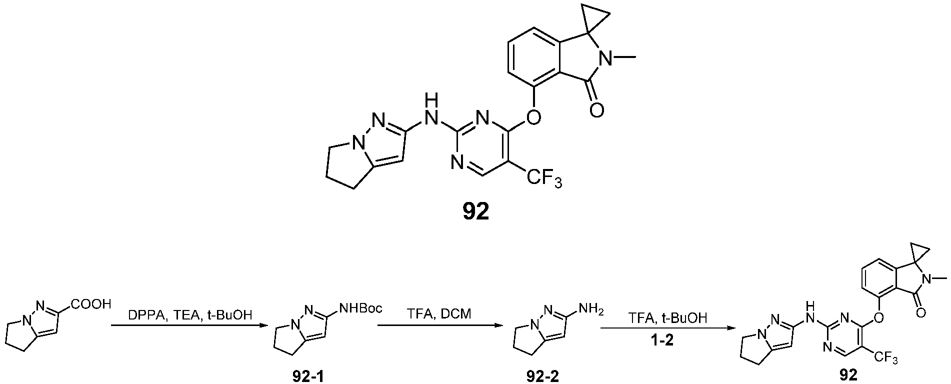

### Synthesis of 92-1:

5,6-Dihydro-4H-pyrrolo[1,2-b]pyrazole-2-carboxylic acid (500 mg, 3.286 mmol), DPPA (1.4 g, 4.929 mmol), and TEA (665 mg, 6.572 mmol) were added to tert-butanol (10 mL). The mixed solution was purged with argon, heated to 80 °C, and stirred for 20 h. After the basic completion of the reaction as detected by LC-MS, the mixture was concentrated. The residue was purified by Flash, extracted, dried, and concentrated to give an off-white solid product (294 mg, yield: 33%).

ESI-MS m/z: 224.0 [M+H]⁺.

### Synthesis of 92-2:

The compound **92-1** described above (294 mg, 1.3 mmol) and TFA (2 mL) were added to DCM (3 mL). The mixed solution was stirred at room temperature for 20 h. After the basic completion of the reaction as detected by LC-MS, the mixture was concentrated, and the residue was purified by Flash and lyophilized to give an off-white solid product (200 mg, yield: >100%).

ESI-MS m/z: 124.0 [M+H]⁺.

### Synthesis of compound 92:

The compound **92-2** described above (34 mg, 0.145 mmol) and **1-2** (50 mg, 0.121 mmol) were added to super-dry *tert*-butanol (2 mL). TFA (14 mg, 0.121 mmol) was added at room temperature. The mixed solution was purged with argon, heated to 80 °C, and stirred for 20 h. After the basic completion of the reaction as detected by LC-MS, the mixture was concentrated, and the residue was purified by HPLC and lyophilized to give an off-white solid product (10 mg, yield: 18.1%).

ESI-MS m/z: 457.1 [M+H]⁺.

The target compounds **93-98** in Table 5 were obtained with reference to a synthetic method similar to that in Example 9 using different starting materials.

**Table 5**

| **Compound** | **Structural formula** | **MS: [M+H]⁺** | **Compound** | **Structural formula** | **MS: [M+H]⁺** |
|---|---|---|---|---|---|
| **93** | 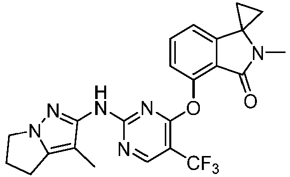 | **471.2** | **94** | 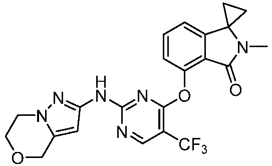 | **473.2** |
| **95** | 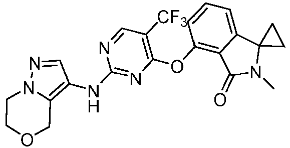 | **473.2** | **96** | 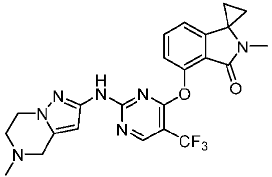 | **486.2** |
| **97** | 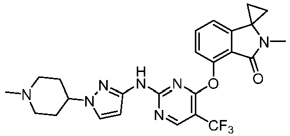 | **514.2** | **98** | 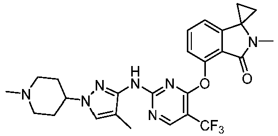 | **528.2** |

### Example 10. Synthesis of 3-methoxy-4-((4-((2'-methyl-3'-oxospiro[cyclopropane-1,1'-isoindolin]-4'-yl)methyl)-5-(trifluoromethyl)pyrimidin-2-yl)amino)-N-(1-methylpiperidin-4-yl)benzamide (Compound 99)

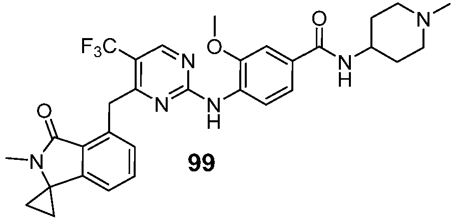

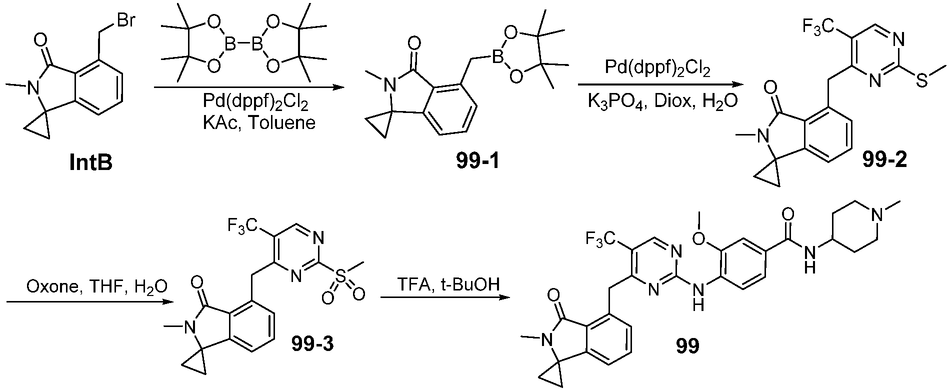

### Synthesis of 99-1:

**IntB** (1 g, 3.76 mmol), bis(pinacolato)diboron (1.91 g, 7.52 mmol), and potassium acetate (1.32 g, 15 mmol) were added to methylbenzene (50 mL). After the mixture was purged with argon, Pd(dppf)₂Cl₂ (292 mg, 0.4 mmol) was added. The mixed solution was heated to 50 °C and stirred for 20 h under argon atmosphere. After the completion of the reaction as detected by LC-MS, the mixed solution was purified by column chromatography to give a product (740 mg, yield: 62.8%).

ESI-MS m/z: 314.1 [M+H]⁺.

### Synthesis of 99-2:

The compound **99-1** described above (740 mg, 2.363 mmol), 4-chloro-2-(methylthio)-5-(trifluoromethyl)pyrimidine (648 mg, 2, 835 mmol), and potassium phosphate (1.5 g, 7.09 mmol) were added to Diox (20 mL)/water (4 mL). After the mixture was purged with argon, Pd(dppf)₂Cl₂ (270 mg, 0.37 mmol) was added. The mixed solution was heated to 100 °C and stirred for 16 h under argon atmosphere. After the completion of the reaction as detected by LC-MS, the mixed solution was concentrated, and the residue was purified by Flash to give a product (435 mg, yield: 48.5%).

ESI-MS m/z: 380.2 [M+H]⁺.

### Synthesis of 99-3:

The compound **99-2** described above (430 mg, 1.133 mmol) was added to a mixed solution of THF (10 mL)/water (2 mL), and Oxone (2.8 g, 4.533 mmol) was added. The mixture was stirred at room temperature for 6 h. After the completion of the reaction as detected by LC-MS, water (20 mL) was added to the system, followed by extraction with EA (2O mL). The organic phase was washed twice with a saturated sodium chloride solution (20 mL), dried over anhydrous sodium sulfate, filtered, and concentrated to dryness to give a light brown solid crude product (490 mg, yield: >100%).

ESI-MS m/z: 412.2 [M+H]⁺.

### Synthesis of compound 99:

The compound **99-3** described above (53 mg, 0.13 mmol) and **3-1** (41 mg, 0.156 mmol) were added to super-dry *tert*-butanol (5 mL), and TFA (23 mg, 0.2 mmol) was added at room temperature. The mixed solution was purged with argon, heated to 80 °C, and stirred for 5 h. After the completion of the reaction as detected by LC-MS, the mixture was concentrated, and the residue was purified by Flash and lyophilized to give an off-white solid product (11 mg, yield: 14.2%).

ESI-MS m/z: 595.2 [M+H]⁺.

### Example 11. Synthesis of 2-fluoro-5-methoxy-4-((4-((2'-methyl-3'-oxospiro[cyclopropane-1,1'-isoindolin]-4'-yl)methyl)-5-(trifluoromethyl)pyrimidin-2-yl)amino)-N-(1-methylpiperidin-4-yl)benzamide (Compound 100)

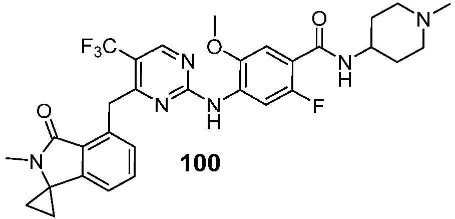

**Compound 100** was obtained with reference to a synthetic method similar to that in **Example 99** using 4-amino-2-fluoro-5-methoxybenzoic acid and compound **99-3** as starting materials.

### Example 12. Synthesis of 3-methoxy-4-((4-((2'-methyl-3'-oxospiro[cyclopropane-1,1'-isoindolin]-4'-yl)methyl)-5-(trifluoromethyl)pyrimidin-2-yl)amino)-N-(7-methyl-7-azaspiro[3.5]nonan-2-yl)benzamide (Compound 101)

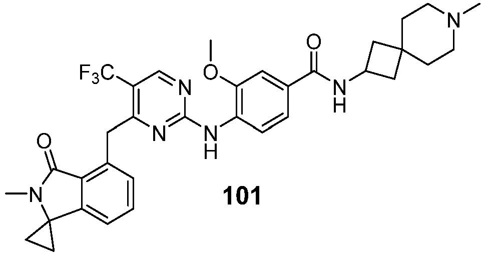

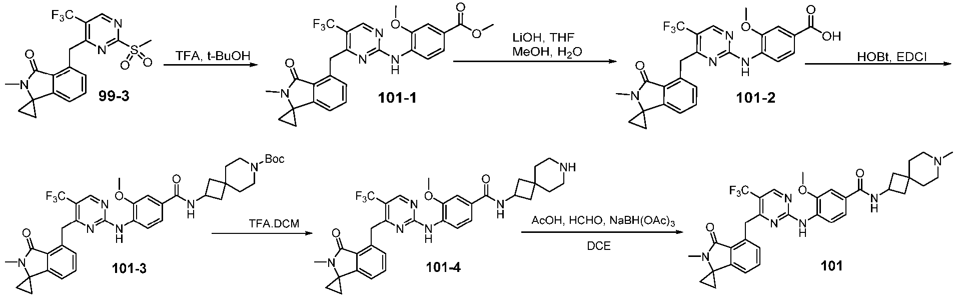

### Synthesis of 101-1:

The compound **99-3** described above (318 mg, 0.65 mmol) and **methyl 4-amino-3-methoxybenzoate** (141 mg, 0.78 mmol) were added to super-dry *tert*-butanol (10 mL), TFA(114 mg, 1.0 mmol) was added at room temperature. The mixed solution was purged with argon, heated to 80 °C, and stirred for 5 h. After the completion of the reaction as detected by LC-MS, the mixture was concentrated, and the residue was purified by Flash and lyophilized to give an off-white solid product (192 mg, yield: 57.6%).

ESI-MS m/z: 513.2 [M+H]⁺.

### Synthesis of 101-2:

The compound **101-1** described above (180 mg, 0.351 mmol) was added to THF (5 mL)/MeOH (2 mL)/water (2 mL), and lithium hydroxide monohydrate (147 mg, 3.51 mmol) was added at room temperature. The mixed solution was stirred at room temperature for 6 h. After the completion of the reaction as detected by LC-MS, the mixed solution was concentrated until there was a small amount left, and water (10 mL) was then added. The pH was adjusted to 2-3 with 2 N HCl aq, and the resulting mixture was extracted three times with EA (20 mL × 3). The organic phases were combined, washed with a saturated sodium chloride solution (10 mL × 2), dried over anhydrous sodium sulfate, filtered, and concentrated to dryness to give a light brown solid product (142 mg, yield: 81.2%).

ESI-MS m/z: 499.2 [M+H]⁺.

### Synthesis of 101-3:

The compound **101-2** described above (50 mg, 0.10 mmol), ***tert*-butyl 2-amino-7-azaspiro[3.5]nonane-7-carboxylate** (24 mg, 0.10 mol), HOBt (21 mg, 0.155 mmol), EDCI (30 mg, 0.155 mg), and DIPEA (39 mg, 0.302 mmol) were added to DMF (5 mL). The mixture was purged with argon and then stirred at room temperature for 20 h. After the completion of the reaction as detected by LC-MS, water (10 mL) was added to the mixed solution, and the resulting mixture was extracted three times with EA (10 mL × 3). The organic phases were combined, washed with a saturated sodium chloride solution (10 mL × 2), dried over anhydrous sodium sulfate, filtered, and concentrated to dryness to give a light brown solid product (80 mg, yield: >100%, crude).

ESI-MS m/z: 721.3 [M+H]⁺.

### Synthesis of 101-4:

The compound **101-3** described above (80 mg, crude, 0.10 mmol) was added to DCM (5 mL), and TFA (114 mg, 1 mmol) was added at room temperature. The mixed solution was stirred at room temperature for 5 h under argon atmosphere. After the basic completion of the reaction as detected by LC-MS, a saturated sodium bicarbonate solution (5 mL) was added to the mixture to quench the reaction, and liquid separation was performed. The aqueous phase was extracted with DCM (10 mL × 3). The organic phases were combined, washed with a saturated sodium chloride solution (10 mL), dried over anhydrous sodium sulfate, filtered, and concentrated to dryness to give a crude product (63 mg, yield: 100%).

ESI-MS m/z: 621.2 [M+H]⁺.

### Synthesis of compound 101:

The compound **101-3** described above (63 mg, 0.1 mmol) was added to DCE (5 mL), and glacial acetic acid (12 mg, 0.2 mmol) and an aqueous formaldehyde solution (41 mg, 37%, 0.5 mmol) were added at room temperature. The mixed solution was stirred at room temperature for 30 min. NaBH(OAc)₃ (106 mg, 0.5 mmol) was then added, and the mixed solution was stirred at room temperature for another 2 h. After the completion of the reaction as detected by LC-MS, the mixed solution was concentrated, and the residue was purified by HPLC and lyophilized to give an off-white solid product (9 mg, yield: 14.2%).

ESI-MS m/z: 635.3 [M+H]⁺.

The target compounds **102-113** in Table 6 were obtained with reference to a synthetic method similar to that in Example 12 using different starting materials.

**Table 6**

| **Compound** | **Structural formula** | **MS: [M+H]⁺** | **Compound** | **Structural formula** | **MS: [M+H]⁺** |
|---|---|---|---|---|---|
| **102** | 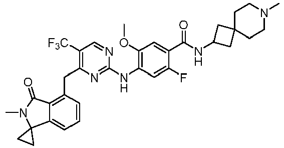 | **653.3** | **103** | 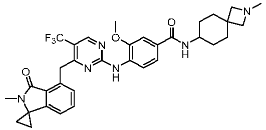 | **635.3** |
| **104** | 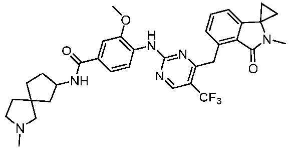 | **635.3** | **105** | 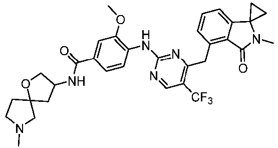 | **637.2** |
| **106** | 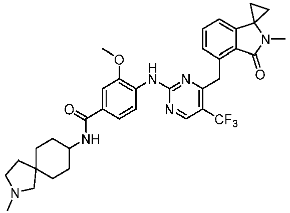 | **649.3** | **107** | 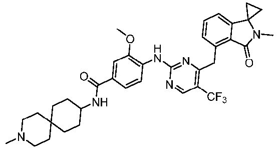 | **663.3** |
| **108** | 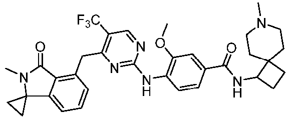 | **635.3** | **109** | 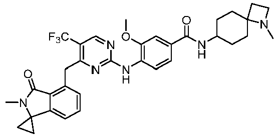 | **635.3** |
| **110** | 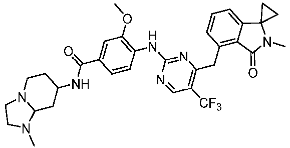 | **636.3** | **111** | 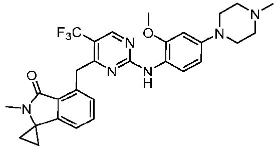 | **553.2** |
| **112** | 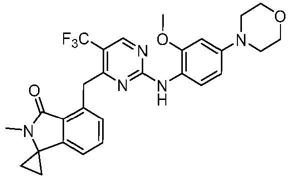 | **540.2** | **113** | 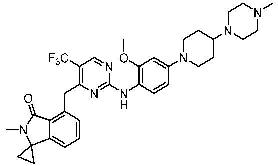 | **636.3** |

### Example 13. Synthesis of 4-((5-chloro-4-((2'-methyl-3'-oxospiro[cyclopropane-1,1'-isoindolin]-4'-yl)oxy)pyrimidin-2-yl)amino)-3-methoxy-N-(1-methylpiperidin-4-yl)benzamide (Compound 114)

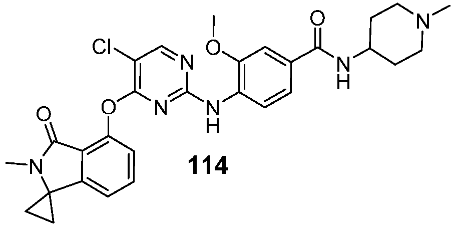

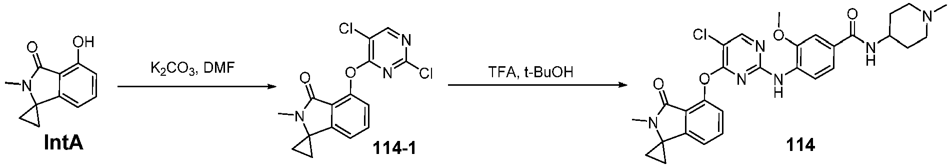

### Synthesis of 114-1:

IntA (378 mg, 2.0 mmol) and 2,4,5-trichloropyrimidine (403 mg, 2.2 mmol) were dissolved in *N*,*N*-dimethylformamide (10 mL), and anhydrous potassium carbonate (828 mg, 6.0 mmol) was added. The mixed solution was stirred at room temperature overnight. After the completion of the reaction as detected by LC-MS, the reaction solution was poured into water (20 mL), and a large amount of solid precipitated. After stirring at room temperature for 30 min, the mixture was filtered. The filter cake was washed with water and then dried under vacuum to give a white solid crude product (544 mg, yield: 80.9%).

ESI-MS m/z: 336.0 [M+H]⁺.

### Synthesis of compound 114:

The crude product **114-1** described above (50 mg, 0.149 mmol) was dissolved in DCM (25 mL), and m-CPBA (2.12 g, 12.3 mmol) was added at room temperature. The mixed solution was stirred at room temperature for 1 h under argon atmosphere, and m-CPBA (2.12 g, 12.3 mmol) was then added. The mixed solution was stirred at room temperature for another 2 h. After the completion of the reaction as detected by LC-MS, the resulting mixture was filtered. DCM (100 mL) and a saturated sodium thiosulfate solution (50 mL) were added to the filtrate, and the mixture was stirred at room temperature for 20 min. Liquid separation was performed. The organic phase was then washed with a saturated sodium thiosulfate solution (50 mL) and a saturated sodium bicarbonate solution (50 mL), dried over anhydrous sodium sulfate, filtered, and concentrated. The residue was purified by column chromatography on 100-200 mesh silica gel (elution gradient PE/EA = 2:1 → DCM/EA = 2:1) to give a white solid product (1.67 g, yield: 64.2%)

ESI-MS m/z: 414.1 [M+H]⁺.

### Synthesis of compound 114:

The crude product **114-1** described above (50 mg, 0.149 mmol) and **3-1** (47 mg, 0.18 mmol) were dissolved in super-dry *tert*-butanol (5 mL), and TFA (23 mg, 0.2 mmol) was then added. The mixed solution was heated at reflux for 24 h under argon atmosphere. After the completion of the reaction as detected by LC-MS, a saturated sodium bicarbonate solution (5 mL) was added to the reaction solution to quench the reaction. The resulting mixture was concentrated under reduced pressure. The residue was purified by Flash and lyophilized to give a white solid product (12 mg, yield: 14.3%)

ESI-MS m/z: 563.0 [M+H]⁺.

The target compounds **115-118** in Table 7 were obtained with reference to a synthetic method similar to that in Example 13 using different starting materials.

**Table 7**

| **Compound** | **Structural formula** | **MS: [M+H]⁺** | **Compound** | **Structural formula** | **MS: [M+H]⁺** |
|---|---|---|---|---|---|
| **115** | 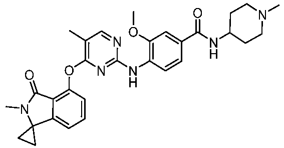 | **543.2** | **116** | 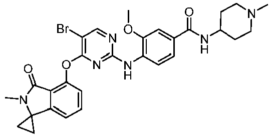 | **607.1** |
| **117** | 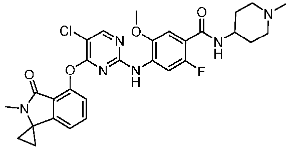 | **581.0** | **118** | 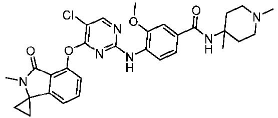 | **578.0** |

### Example 14. Synthesis of 4'-((2-((5-fluoro-2-methoxy-4-(1-methylpiperidin-4-yl)phenyl)amino)-5-(trifluoromethyl)pyrimidin-4-yl)oxy)-2'-methylspiro [cyclopropane-1,1'-isoindolin]-3'-one (Compound 119)

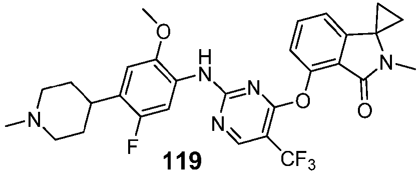

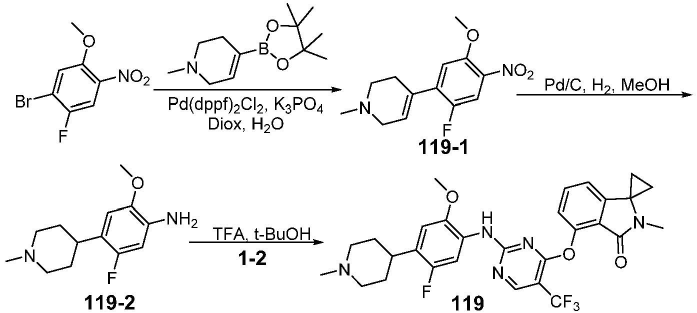

### Synthesis of 119-1:

The compound **1-bromo-2-fluoro-5-methoxy-4-nitrobenzene** described above (375 mg, 1.5 mmol), 1-methyl-1,2,3,6-tetrahydropyridine-4-boronic acid pinacol ester (446 mg, 2.0 mmol), and K₃PO₄ (636 mg, 3.0 mmol) were added to Diox (10 mL) and water (4 mL). After the mixture was purged with argon, Pd(dppf)₂Cl₂ (140 mg, 0.15 mmol) was added. The mixed solution was heated to 80 °C and stirred for 20 h under argon atmosphere. After the completion of the reaction as detected by LC-MS, the mixed solution was filtered. The filter cake was washed with EA (40 mL), and water (20 mL) was added to the filtrate. The resulting mixture was stirred, and liquid separation was performed. The aqueous phase was extracted with EA (20 mL). The organic phases were combined, washed with a saturated sodium chloride solution, and concentrated. The residue was purified by column chromatography to give a product (256 mg, yield: 64%).

ESI-MS m/z: 267.1 [M+H]⁺.

### Synthesis of 119-2:

The compound **119-2** described above (256 mg, 0.962 mmol) and 10% Pd/C (54 mg) were added to MeOH (10 mL). The mixture was purged with H₂ 3 times and then stirred at room temperature under normal pressure for 20 h. After the completion of the reaction as detected by LC-MS, the mixed solution was filtered, and the filtrate was concentrated to dryness to give a purple oily product (253 mg, yield: 100%).

ESI-MS m/z: 239.1 [M+H]⁺.

### Synthesis of compound 119:

The compound **119-2** described above (76 mg, 0.20 mmol) and **1-2** (82 mg, 0.20 mmol) were added to Diox (5 mL), and TFA (25 mg, 0.22 mmol) was added at room temperature. The mixed solution was purged with argon, heated to 90 °C, and stirred for 3 h. A product was detected by LC-MS. The mixture was concentrated, and the residue was purified by Flash and lyophilized to give an off-white solid product (13 mg, yield: 11.4%).

ESI-MS m/z: 572.2 [M+H]⁺.

### Example 15. Synthesis of 4'-((2-((5-fluoro-2-methoxy-4-(7-methyl-2,7-diazaspiro[3.5] nonan-2-yl)phenyl)amino)-5-(trifluoromethyl)pyrimidin-4-yl)oxy)-2'-methylspiro [cyclopropane-1,1'-isoindolin]-3'-one (Compound 120)

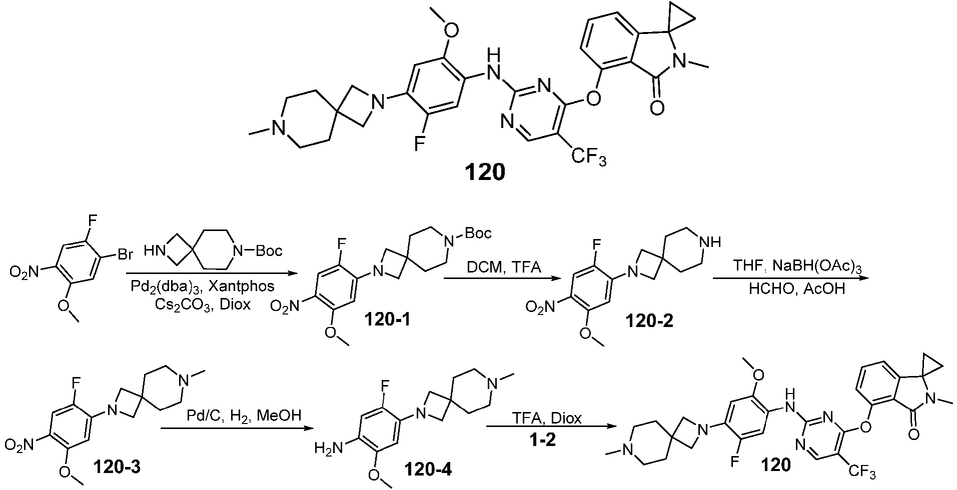

### Synthesis of 120-1:

The compound **1-bromo-2-fluoro-5-methoxy-4-nitrobenzene** described above (770 mg, 3.079 mmol), tert-butyl 2,7-diazaspiro[3.5]nonane-7-carboxylate (697 mg, 3.079 mmol), Xantphos (178 mg, 0.308 mmol), and cesium carbonate (2.0 g, 6.158 mmol) were added to Diox (10 mL). After the mixture was purged with argon, Pd₂(dba)₃ (141 mg, 0.154 mmol) was added. The mixed solution was heated to 100 °C and stirred for 20 h under argon atmosphere. After the completion of the reaction as detected by LC-MS, the mixed solution was filtered. The filter cake was washed with EA (40 mL), and water (20 mL) was added to the filtrate. The resulting mixture was stirred, and liquid separation was performed. The aqueous phase was extracted with EA (20 mL). The organic phases were combined, washed with a saturated sodium chloride solution, and concentrated. The residue was purified by column chromatography to give a product (890 mg, yield: 73.1%).

ESI-MS m/z: 396.2 [M+H]⁺.

### Synthesis of 120-2:

The compound **120-1** described above (400 mg, 1.012 mmol) and TFA (1 mL) were added to DCM (2 mL). The mixture was purged with argon and stirred at room temperature for 2 h. After the completion of the reaction as detected by LC-MS, the mixed solution was concentrated to give a crude product (701 mg, yield: >100%).

ESI-MS m/z: 296.1 [M+H]⁺.

### Synthesis of 120-3:

The compound **120-2** described above (701 mg, crude, 1.012 mmol) and DIPEA (404 mg, 3.135 mmol) were added to THF (10 mL)/MeOH (2 mL). The mixture was stirred at room temperature for 10 min. Glacial acetic acid (137 mg, 2.276 mmol) and an aqueous formaldehyde solution (100 mg, 37%, 1.23 mmol) were then added, and the mixed solution was stirred at room temperature for 20 min. NaBH(OAc)₃ (434 mg, 2.049 mmol) was then added, and the mixed solution was stirred at room temperature for another 1 h. After the completion of the reaction as detected by LC-MS, the mixed solution was concentrated, and the residue was purified by Flash to give a yellow solid product (305 mg, yield: 97.5%).

ESI-MS m/z: 310.1 [M+H]⁺.

### Synthesis of 120-4:

The compound **120-3** described above (305 mg, 0.987 mmol) and 10% Pd/C (34 mg) were added to MeOH (10 mL). The mixture was purged with H₂ 3 times and then stirred at room temperature under normal pressure for 2 h. After the completion of the reaction as detected by LC-MS, the mixed solution was filtered, and the filtrate was concentrated to dryness to give a purple-ish black oily product (303 mg, yield: 100%).

ESI-MS m/z: 280.1 [M+H]⁺.

### Synthesis of compound 120:

The compound **120-4** described above (135 mg, 0.483 mmol) and **1-2** (200 mg, 0.483 mmol) were added to Diox (10 mL), and TFA (66 mg, 0.58 mmol) was added at room temperature. The mixed solution was purged with argon, heated to 90 °C, and stirred for 3 h. A reaction product was detected by LC-MS. The mixture was concentrated, and the residue was purified by Flash and lyophilized to give an off-white solid product (35 mg, yield: 11.8%).

ESI-MS m/z: 613.2 [M+H]⁺.

### Example 16. Synthesis of 4'-((2-((5-fluoro-2-methoxy-4-(4-(4-methylpiperazin-1-yl)piperidin-1-yl)phenyl)amino)-5-(trifluoromethyl)pyrimidin-4-yl)oxy)-2'-methylspiro[cyclopropane-1,1'-isoindolin]-3'-one (Compound 121)

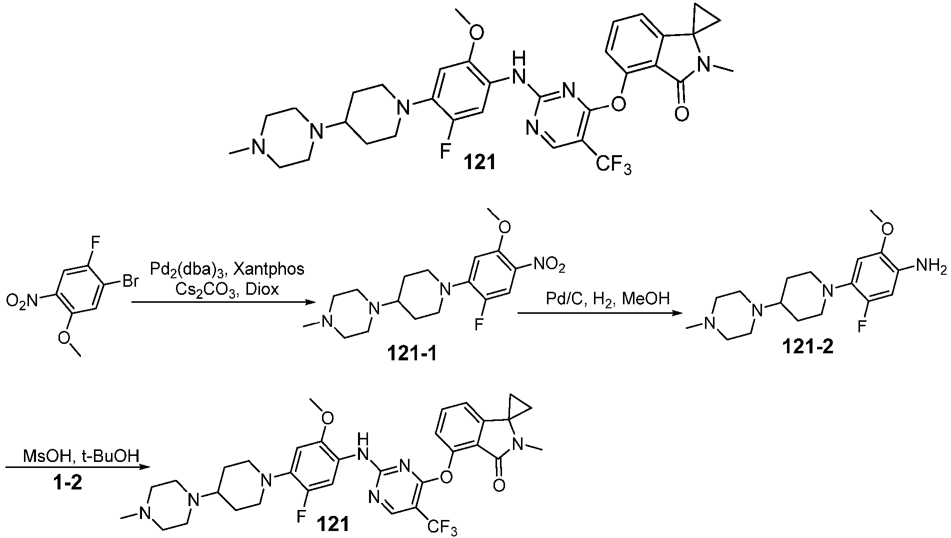

### Synthesis of 121-1:

The compound **1-bromo-2-fluoro-5-methoxy-4-nitrobenzene** described above (1.0 g, 4.0 mmol), 1-methyl-4-(piperidin-4-yl)piperazine (733 mg, 4.0 mmol), Xantphos (231 mg, 0.4 mmol), and cesium carbonate (2.6 g, 8.0 mmol) were added to Diox (20 mL). After the mixture was purged with argon, Pd₂(dba)₃ (183 mg, 0.20 mmol) was added. The mixed solution was heated to 100 °C and stirred for 20 h under argon atmosphere. After the completion of the reaction as detected by LC-MS, the mixed solution was filtered. The filter cake was washed with DCM (50 mL), and water (50 mL) was added to the filtrate. The resulting mixture was stirred, and liquid separation was performed. The aqueous phase was extracted with DCM (50 mL). The organic phases were combined, washed with a saturated sodium chloride solution, and concentrated. The residue was purified by Flash to give a yellow solid product (715 mg, yield: 50.7%).

ESI-MS m/z: 353.2 [M+H]⁺.

### Synthesis of 121-2:

The compound **121-1** described above (715 mg, 2.03 mmol) and 10% Pd/C (70 mg) were added to MeOH (10 mL). The mixture was purged with H₂ 3 times and then stirred at room temperature under normal pressure for 4 h. After the completion of the reaction as detected by LC-MS, the mixed solution was filtered, and the filtrate was concentrated to dryness to give a purple-ish black solid product (641 mg, yield: 98%).

ESI-MS m/z: 323.2 [M+H]⁺.

### Synthesis of compound 121:

The compound **121-2** described above (510 mg, 1.582 mmol) and **1-2** (654 mg, 1.582 mmol) were added to *t*-BuOH (10 mL), and MsOH (273 mg, 2.847 mmol) was added at room temperature. The mixed solution was purged with argon, heated to 90 °C, and stirred for 6 h. A reaction product was detected by LC-MS. The mixture was concentrated. The residue was purified by Flash, and the impure product was further purified by preparative HPLC and lyophilized to give an off-white solid product (405 mg, yield: 39%).

ESI-MS m/z: 656.3 [M+H]⁺.

The target compounds **122-129** in Table 8 were obtained with reference to synthetic methods similar to those in Examples 7 and 8 and Examples 15 and 16 using different starting materials.

**Table 8**

| **Compound** | **Structural formula** | **MS: [M+H]⁺** | **Compound** | **Structural formula** | **MS: [M+H]⁺** |
|---|---|---|---|---|---|
| **122** | 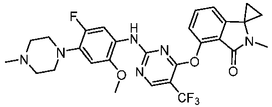 | **573.2** | **123** | 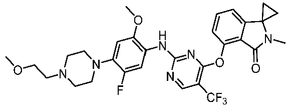 | **617.2** |
| **124** | 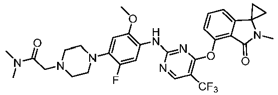 | **644.2** | **125** | 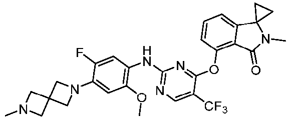 | **585.2** |
| **126** | 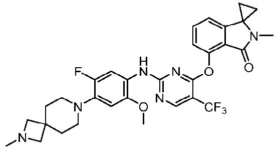 | **613.2** | **127** | 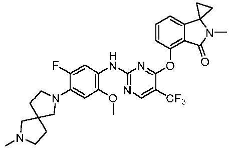 | **613.2** |
| **128** | 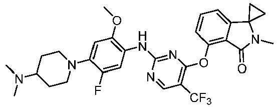 | **601.2** | **129** | 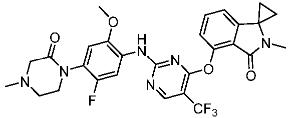 | **587.2** |

### Example 17. Synthesis of 4'-((5-cyclopropyl-2-((2-methoxy-4-(4-methylpiperazin-1-yl)phenyl)amino)pyrimidin-4-yl)oxy)-2'-methylspiro [cyclopropane-1,1'-isoindolin]-3'-one (Compound 130)

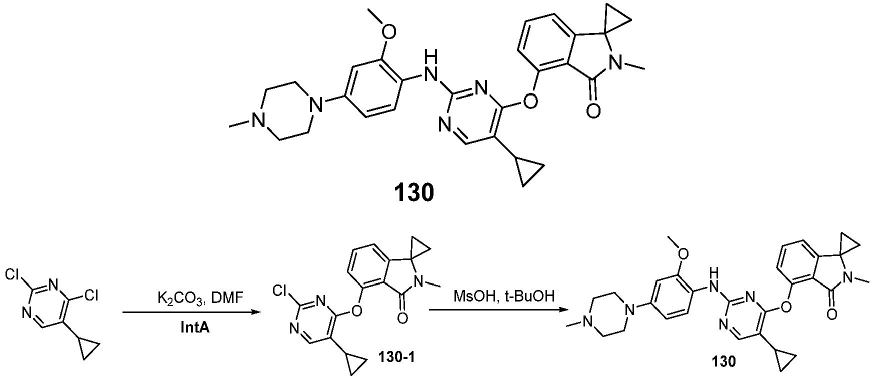

### Synthesis of 130-1:

The compounds **2,4-dichloro-5-cyclopropylpyrimidine** (500 mg, 2.646 mmol) and **IntA** (500 mg, 2.646 mmol) were added to DMF (5 mL), and the mixture was cooled to 0-5 °C in an ice bath under argon atmosphere. Potassium carbonate (750 mg, 5.435 mmol) was then added. The mixed solution was stirred at 0-5 °C for 30 min, then heated to room temperature, and stirred for another 5 h. After the completion of the reaction as detected by LC-MS, water (10 mL) was added to the mixed solution. The resulting mixture was stirred for 15 min and then filtered. The filter cake was washed with water and then dried under vacuum to give a product (622 mg, yield: 68.8%).

ESI-MS m/z: 342.1 [M+H]⁺.

### Synthesis of compound 130:

The compound **130-1** described above (100 mg, 0.293 mmol) and **2-methoxy-4-(4-methylpiperazin-1-yl)aniline** (78 mg, 0.352 mmol) were added to *t*-BuOH (5 mL), and MsOH (51 mg, 0.531 mmol) was added at room temperature. The mixed solution was purged with argon, heated to 90 °C, and stirred for 6 h. A reaction product was detected by LC-MS. The mixture was concentrated. The residue was purified by Flash, and the impure product was further purified by pre-TLC to give an off-white solid product (15 mg, yield: 9.72%).

ESI-MS m/z: 527.2 [M+H]⁺.

### Example 18. Synthesis of 4'-((5-cyclopropyl-2-((2-methoxy-4-(7-methyl-2,7-diazaspiro [3.5]nonan-2-yl)phenyl)amino)pyrimidin-4-yl)oxy)-2'-methylspiro[cyclopropane-1,1'-isoindolin]-3'-one (Compound 131)

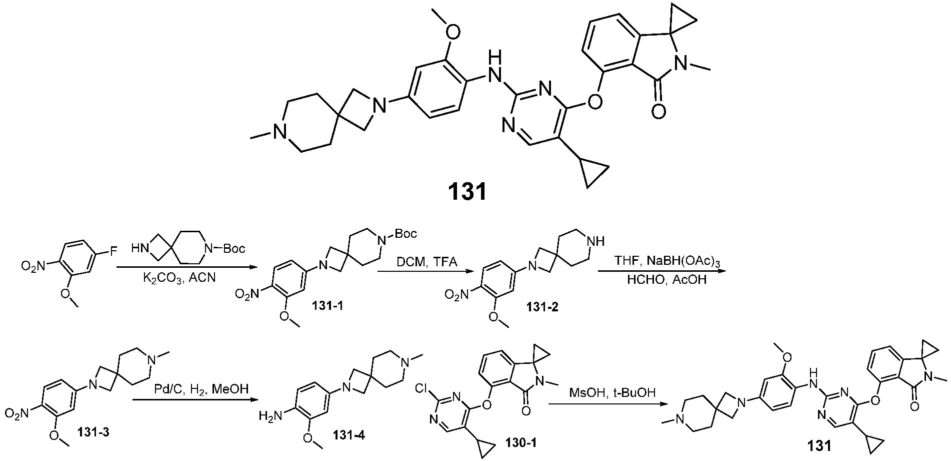

### Synthesis of 131-1:

The compound **4-fluoro-2-methoxy-1-nitrobenzene** described above (794 mg, 4.64 mmol), *tert*-butyl 2,7-diazaspiro[3.5]nonane-7-carboxylate (1 g, 4.42 mmol), and anhydrous potassium carbonate (1.3 g, 9.28 mmol) were added to acetonitrile (10 mL). The mixture was purged with argon, then heated to 85 °C, and stirred for 40 h. After the basic completion of the reaction as detected by LC-MS, the mixed solution was concentrated. EA (20 mL) and water (20 mL) were added to the residue. The resulting mixture was stirred, and liquid separation was performed. The aqueous phase was extracted with EA (20 mL). The organic phases were combined, washed with a saturated sodium chloride solution, and concentrated. The residue was purified by column chromatography to give a product (1.5 g, yield: 90%).

ESI-MS m/z: 378.2 [M+H]⁺.

### Synthesis of 131-2:

The compound **131-1** described above (680 mg, 1.8 mmol) and TFA (2 mL) were added to DCM (4 mL). The mixture was purged with argon and stirred at room temperature for 2 h. After the completion of the reaction as detected by LC-MS, the mixed solution was concentrated. EA (5 mL) and PE (5 mL) were added to the residue. The resulting mixture was stirred at room temperature for 30 min and then filtered. The filter cake was dried under vacuum to give a yellow solid product (730 mg, yield: >100%).

ESI-MS m/z: 278.1 [M+H]⁺.

### Synthesis of 131-3:

The compound **131-2** described above (730 mg, 1.8 mmol) and DIPEA(233 mg, 1.8 mmol) were added to THF (20 mL)/MeOH (4 mL). The mixture was stirred at room temperature for 10 min. Glacial acetic acid (216 mg, 3.6 mmol) and an aqueous formaldehyde solution (175 mg, 37%, 2.16 mmol) were then added, and the mixed solution was stirred at room temperature for 20 min. NaBH(OAc)₃ (763 mg, 3.6 mmol) was then added, and the mixed solution was stirred at room temperature for another 1 h. After the completion of the reaction as detected by LC-MS, the mixed solution was concentrated, and the residue was purified by Flash to give a yellow foamy solid product (516 mg, yield: 98%).

ESI-MS m/z: 292.1 [M+H]⁺.

### Synthesis of 131-4:

The compound **131-3** described above (512 mg, 1.757 mmol) and 10% Pd/C (50 mg) were added to MeOH (10 mL). The mixture was purged with H₂ 3 times and then stirred at room temperature under normal pressure for 20 h. After the completion of the reaction as detected by LC-MS, the mixed solution was filtered, and the filtrate was concentrated to dryness to give a purple-ish black oily product (460 mg, yield: 100%).

ESI-MS m/z: 262.1 [M+H]⁺.

### Synthesis of compound 131:

The compound **130-1** described above (100 mg, 0.293 mmol) and **131-4** (92 mg, 0.352 mmol) were added to *t*-BuOH (5 mL), and MsOH (51 mg, 0.531 mmol) was added at room temperature. The mixed solution was purged with argon, heated to 90 °C, and stirred for 6 h. A reaction product was detected by LC-MS. The mixture was concentrated. The residue was purified by Flash, and the impure product was further purified by pre-HPLC and lyophilized to give an off-white solid product (13 mg, yield: 7.83%).

ESI-MS m/z: 567.3 [M+H]⁺.

### Example 19. Synthesis of 4-((5-chloro-4-((2'-methyl-3'-oxospiro[cyclopropane-1,1'-isoindolin]-4'-yl)oxy)pyrimidin-2-yl)amino)-2-fluoro-5-methoxy-N-(7-methyl-7-azaspiro[3.5]nonan-2-yl)benzamide (Compound 132)

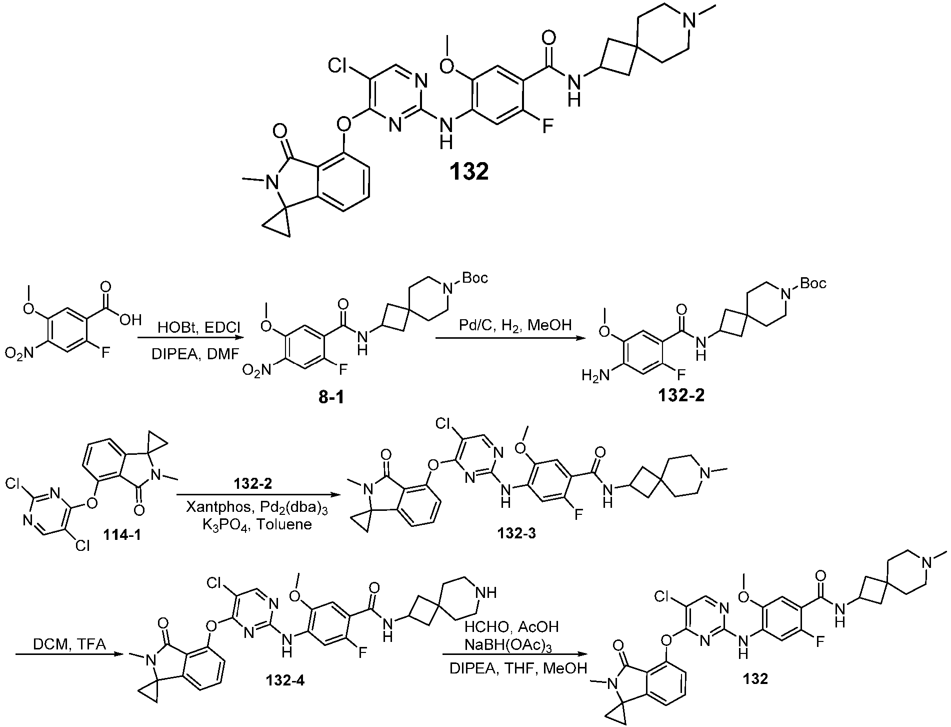

### Synthesis of 8-1:

**2-Fluoro-5-methoxy-4-nitrobenzoic acid** (1.22 g, 4.16 mmol), ***tert*-butyl 2-amino-7-azaspiro[3.5]nonane-7-carboxylate** (1.0 g, 4.16 mol), HOBt (840 mg, 6.2 mmol), EDCI (1.2 g, 6.2 mmol), and DIPEA (2.2 mL) were added to DMF (20 mL). The mixture was purged with argon and then stirred at room temperature for 20 h. After the completion of the reaction as detected by LC-MS, water (40 mL) was added to the mixed solution. The resulting mixture was stirred at room temperature for 30 min and then filtered. The filter cake was washed with water and dried under vacuum to give a product (1.9 g, yield: >100%).

ESI-MS m/z: 438.2 [M+H]⁺.

### Synthesis of 132-2:

The compound **8-1** described above (900 mg, 2.06 mmol) and 10% Pd/C (90 mg) were added to MeOH/THF (10 mL/10 mL). The mixture was purged with Hz 3 times and then stirred at room temperature under normal pressure for 20 h. After the completion of the reaction as detected by LC-MS, the mixed solution was filtered, and the filtrate was concentrated to dryness to give a pale gray foamy solid product (880 mg, yield: >100%).

ESI-MS m/z: 408.2 [M+H]⁺.

### Synthesis of 132-3:

The compound **132-2** described above (315 mg, 0.773 mmol) and compound **114-1** (200 mg, 0.595 mmol), Xantphos (69 mg, 0.119 mmol), and anhydrous potassium phosphate (379 mg, 1.785 mmol) were added to methylbenzene (10 mL). After the mixture was purged with argon, Pd₂(dba)₃ (54 mg, 0.06 mmol) was added. The mixed solution was heated to 100 °C and stirred for 8 h under argon atmosphere. After the completion of the reaction as detected by LC-MS, the mixed solution was directly loaded onto a column and purified by silica gel column chromatography to give a yellowish brown solid product (95 mg, yield: 22.6%).

ESI-MS m/z: 707.3 [M+H]⁺.

### Synthesis of 132-4:

The compound **132-3** described above (95 mg, 0.134 mmol) and TFA (0.3 mL) were added to DCM (5 mL). The mixture was purged with argon and stirred at room temperature for 2 h. After the completion of the reaction as detected by LC-MS, the mixed solution was concentrated to give a crude product (172 mg, yield: >100%).

ESI-MS m/z: 607.2 [M+H]⁺.

### Synthesis of compound 132:

The compound **132-4** described above (172 mg, crude, 0.134 mmol) and DIPEA (168 mg, 1.3 mmol) were added to THF (5 mL)/MeOH (1 mL). The mixed solution was stirred at room temperature for 10 min. Glacial acetic acid (16 mg, 0.27 mmol) and an aqueous formaldehyde solution (17 mg, 37%, 0.204 mmol) were then added, and the mixed solution was stirred at room temperature for 20 min. NaBH(OAc)₃ (57 mg, 0.27 mmol) was then added, and the mixed solution was stirred at room temperature for another 1 h. After the completion of the reaction as detected by LC-MS, the mixed solution was concentrated, and the residue was purified by Flash to give an off-white solid product (32 mg, yield: 38.4%).

ESI-MS m/z: 621.2 [M+H]⁺.

### Example 20. Synthesis of 4-((5-chloro-4-((2'-methyl-3'-oxospiro[cyclopropane-1,1'-isoindolin]-4'-yl)oxy)pyrimidin-2-yl)amino)-3-methoxy-N-(7-methyl-7-azaspiro[3.5]nonan-2-yl)benzamide (Compound 133)

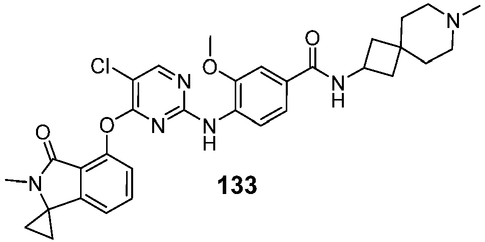

The target compound 133 was obtained with reference to a synthetic method similar to that in Example 132 using 3-methoxy-4-nitrobenzoic acid as a starting material.

### Example 21. Synthesis of 4'-((5-chloro-2-((5-fluoro-2-methoxy-4-(1-methylpiperidin-4-yl)phenyl)amino)pyrimidin-4-yl)oxy)-2'-methylspiro [cyclopropane-1,1'-isoindolin]-3'-one (Compound 134)

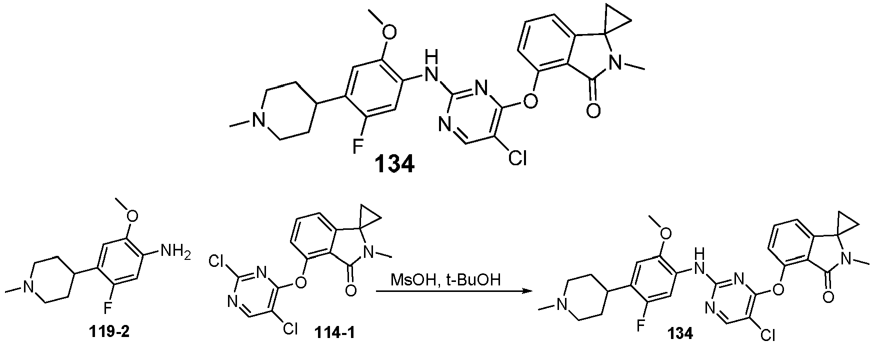

### Synthesis of compound 134:

The compound **114-1** described above (80 mg, 0.238 mmol) and **119-2** (68 mg, 0.286 mmol) were added to *t*-BuOH (5 mL), and MsOH (34 mg, 0.354 mmol) was added at room temperature. The mixed solution was purged with argon, heated to 90 °C, and stirred for 6 h. A reaction product was detected by LC-MS. The mixture was concentrated. The residue was purified by Flash, and the impure product was further purified by pre-HPLC and lyophilized to give an off-white solid product (16 mg, yield: 12.5%).

ESI-MS m/z: 538.2 [M+H]⁺.

### Example 22. Synthesis of 4'-((5-chloro-2-((2-methoxy-4-(1-methylpiperidin-4-yl)phenyl)amino)pyrimidin-4-yl)oxy)-2'-methylspiro [cyclopropane-1,1'-isoindolin]-3'-one (Compound 135)

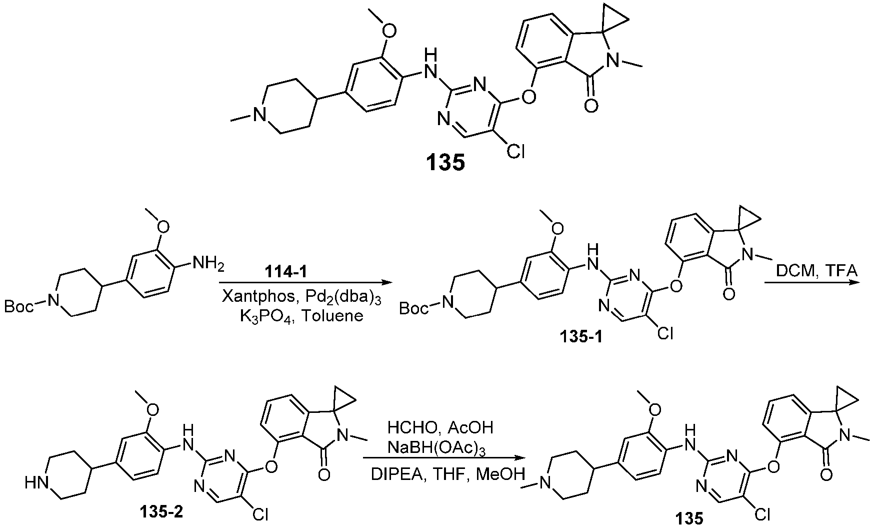

### Synthesis of 135-1:

***tert*-Butyl 4-(4-amino-3-methoxyphenyl)piperidine-1-carboxylate** (237 mg, 0.773 mmol) and compound **114-1** (200 mg, 0.595 mmol), Xantphos (69 mg, 0.119 mmol), and anhydrous potassium phosphate (379 mg, 1.785 mmol) were added to methylbenzene (10 mL). After the mixture was purged with argon, Pd₂(dba)₃ (54 mg, 0.06 mmol) was added. The mixed solution was heated to 100 °C and stirred for 8 h under argon atmosphere. After the completion of the reaction as detected by LC-MS, the mixed solution was directly loaded onto a column and purified by silica gel column chromatography to give a yellowish brown solid product (198 mg, yield: 54.9%).

ESI-MS m/z: 606.2 [M+H]⁺.

### Synthesis of 135-2:

The compound **135-1** described above (198 mg, 0.327 mmol) and TFA (0.5 mL) were added to DCM (10 mL). The mixture was purged with argon and stirred at room temperature for 2 h. After the completion of the reaction as detected by LC-MS, the mixed solution was concentrated to give a crude product (271 mg, yield: >100%).

ESI-MS m/z: 506.2 [M+H]⁺.

### Synthesis of compound 135:

The compound **135-2** described above (141 mg, crude, 0.17 mmol) and DIPEA (168 mg, 1.3 mmol) were added to THF (5 mL)/MeOH (1 mL). The mixed solution was stirred at room temperature for 10 min. Glacial acetic acid (20 mg, 0.33 mmol) and an aqueous formaldehyde solution (17 mg, 37%, 0.204 mmol) were then added, and the mixed solution was stirred at room temperature for 20 min. NaBH(OAc)₃ (72 mg, 0.34 mmol) was then added, and the mixed solution was stirred at room temperature for another 1 h. After the completion of the reaction as detected by LC-MS, the mixed solution was concentrated, and the residue was purified by Flash to give a yellow solid product (47 mg, yield: 53.2%).

ESI-MS m/z: 520.2 [M+H]⁺.

### Example 23. Synthesis of 4'-((5-chloro-2-((2-methoxy-4-(7-methyl-2,7-diazaspiro[3.5] nonan-2-yl)phenyl)amino)pyrimidin-4-yl)oxy)-2'-methylspiro[cyclopropane-1,1'-isoindolin]-3'-one (Compound 136)

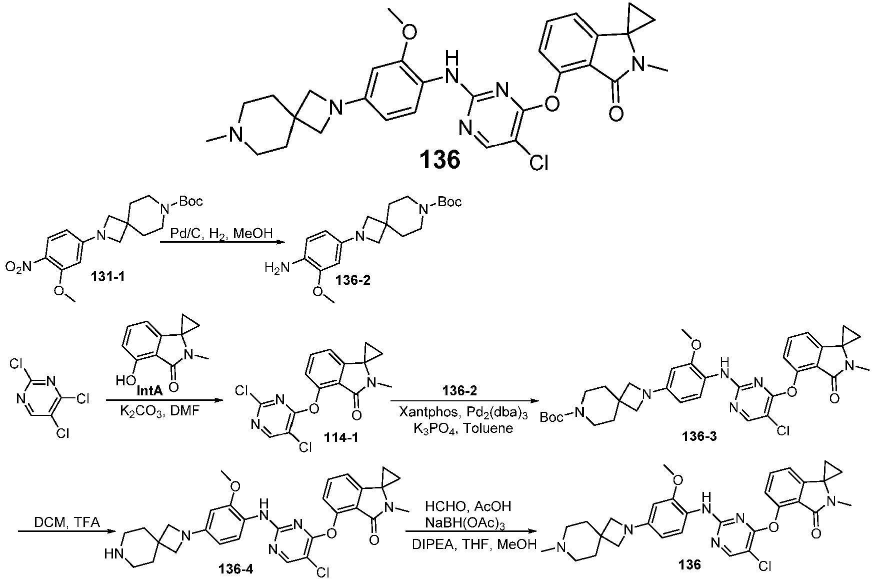

### Synthesis of 136-2:

The compound **131-1** described above (500 mg, 1.325 mmol) and 10% Pd/C (50 mg) were added to MeOH/THF (10 mL/4 mL). The mixture was purged with H₂ 3 times and then stirred at room temperature under normal pressure for 2 h. After the completion of the reaction as detected by LC-MS, the mixed solution was filtered, and the filtrate was concentrated to dryness to give a purple-ish black foamy solid product (450 mg, yield: 96%).

ESI-MS m/z: 348.1 [M+H]⁺.

### Synthesis of 114-1:

The compounds **2,4,5-trichloropyrimidine** (500 mg, 2.73 mmol) and **IntA** (516 mg, 2.73 mmol) were added to DMF (5 mL), and the mixture was cooled to 0-5 °C in an ice bath under argon atmosphere. Potassium carbonate (755 mg, 5.46 mmol) was then added. The mixed solution was stirred at 0-5 °C for 1 h. After the completion of the reaction as detected by LC-MS, water (10 mL) was added to the mixed solution. The resulting mixture was stirred for 15 min and then filtered. The filter cake was washed with water and then dried under vacuum to give a product (836 mg, yield: 91%).

ESI-MS m/z: 336.0 [M+H]⁺.

### Synthesis of 136-3:

The compound **136-2** described above (269 mg, 0.773 mmol) and compound **114-1** (200 mg, 0.595 mmol), Xantphos (69 mg, 0.119 mmol), and anhydrous potassium phosphate (379 mg, 1.785 mmol) were added to methylbenzene (10 mL). After the mixture was purged with argon, Pd₂(dba)₃ (54 mg, 0.06 mmol) was added. The mixed solution was heated to 100 °C and stirred for 8 h under argon atmosphere. After the completion of the reaction as detected by LC-MS, the mixed solution was directly loaded onto a column and purified by silica gel column chromatography to give a yellowish brown solid product (110 mg, yield: 28%).

ESI-MS m/z: 647.3 [M+H]⁺.

### Synthesis of 136-4:

The compound **136-3** described above (110 mg, 0.17 mmol) and TFA (0.3 mL) were added to DCM (5 mL). The mixture was purged with argon and stirred at room temperature for 2 h. After the completion of the reaction as detected by LC-MS, the mixed solution was concentrated to give a crude product (241 mg, yield: >100%).

ESI-MS m/z: 547.2 [M+H]⁺.

### Synthesis of compound 136:

The compound **136-4** described above (241 mg, crude, 0.17 mmol) and DIPEA (168 mg, 1.3 mmol) were added to THF (5 mL)/MeOH (1 mL). The mixed solution was stirred at room temperature for 10 min. Glacial acetic acid (20 mg, 0.33 mmol) and an aqueous formaldehyde solution (17 mg, 37%, 0.204 mmol) were then added, and the mixed solution was stirred at room temperature for 20 min. NaBH(OAc)₃ (72 mg, 0.34 mmol) was then added, and the mixed solution was stirred at room temperature for another 1 h. After the completion of the reaction as detected by LC-MS, the mixed solution was concentrated, and the residue was purified by Flash to give a yellow solid product (79 mg, yield: 82.8%).

ESI-MS m/z: 561.3 [M+H]⁺.

### Example 24. Synthesis of 4'-((5-chloro-2-((5-fluoro-2-methoxy-4-(7-methyl-2,7-diazaspiro [3.5]nonan-2-yl)phenyl)amino)pyrimidin-4-yl)oxy)-2'-methylspiro[cyclopropane-1,1'-isoindolin]-3'-one (Compound 137)

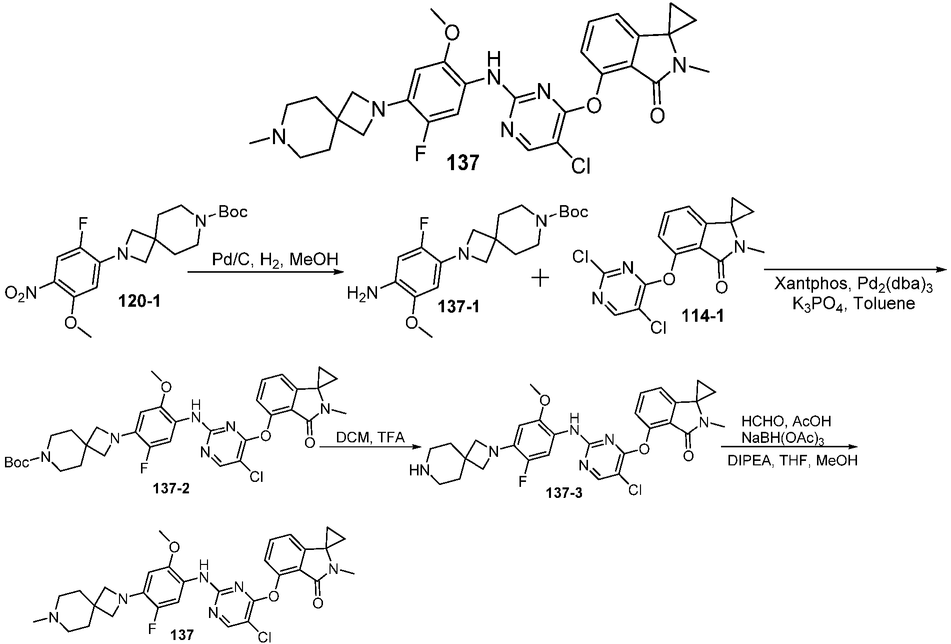

### Synthesis of 137-1:

The compound **120-1** described above (1.2 g, 3.0 mmol) and 10% Pd/C (120 mg) were added to MeOH/THF (10 mL/10 mL). The mixture was purged with H₂ 3 times and then stirred at room temperature under normal pressure for 20 h. After the completion of the reaction as detected by LC-MS, the mixed solution was filtered, and the filtrate was concentrated to dryness to give a purple-ish black foamy solid product (1.1 g, yield: 98%).

ESI-MS m/z: 366.2 [M+H]⁺.

### Synthesis of 137-2:

The compound **137-1** described above (283 mg, 0.774 mmol) and compound **114-1** (200 mg, 0.595 mmol), Xantphos (69 mg, 0.119 mmol), and anhydrous potassium phosphate (379 mg, 1.785 mmol) were added to methylbenzene (10 mL). After the mixture was purged with argon, Pd₂(dba)₃ (54 mg, 0.06 mmol) was added. The mixed solution was heated to 100 °C and stirred for 8 h under argon atmosphere. After the completion of the reaction as detected by LC-MS, the mixed solution was directly loaded onto a column and purified by silica gel column chromatography to give a yellowish brown gummy product (213 mg, yield: 46%).

ESI-MS m/z: 665.4 [M+H]⁺.

### Synthesis of 137-3:

The compound **137-2** described above (213 mg, 0.32 mmol) and TFA (0.5 mL) were added to DCM (5 mL). The mixture was purged with argon and stirred at room temperature for 2 h. After the completion of the reaction as detected by LC-MS, the mixed solution was concentrated to give a crude product (361 mg, yield: >100%).

ESI-MS m/z: 565.3 [M+H]⁺.

### Synthesis of compound 137:

The compound **137-3** described above (361 mg, crude, 0.32 mmol) and DIPEA (223 mg, 1.73 mmol) were added to THF (5 mL)/MeOH (1 mL). The mixed solution was stirred at room temperature for 10 min. Glacial acetic acid (40 mg, 0.67 mmol) and an aqueous formaldehyde solution (32 mg, 37%, 0.384 mmol) were then added, and the mixed solution was stirred at room temperature for 20 min. NaBH(OAc)₃ (136 mg, 0.64 mmol) was then added, and the mixed solution was stirred at room temperature for another 1 h. After the completion of the reaction as detected by LC-MS, the mixed solution was concentrated, and the residue was purified by Flash to give a yellow solid product (143 mg, yield: 77.1%).

ESI-MS m/z: 579.3 [M+H]⁺.

The target **compounds 138-152** in Table 9 were obtained with reference to synthetic methods similar to those in Example 23 and Example 24 using different starting materials.

**Table 9**

| **Compound** | **Structural formula** | **MS: [M+H]⁺** | **Compound** | **Structural formula** | **MS: [M+H]⁺** |
|---|---|---|---|---|---|
| **138** | 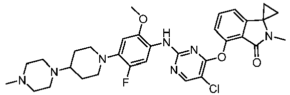 | **622.3** | **139** | 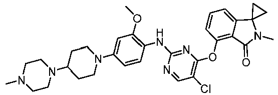 | **604.3** |
| **140** | 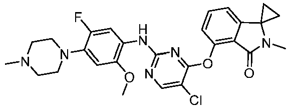 | **539.2** | **141** | 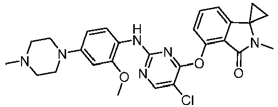 | **521.2** |
| **142** | 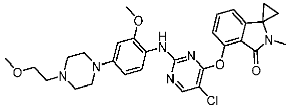 | **565.2** | **143** | 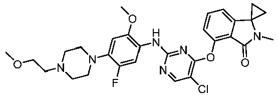 | **583.2** |
| **144** | 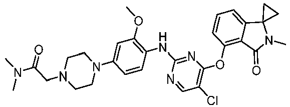 | **592.2** | **145** | 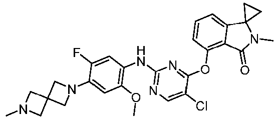 | **551.2** |
| **146** | 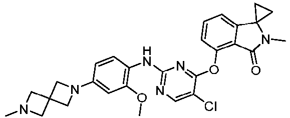 | **533.2** | **147** | 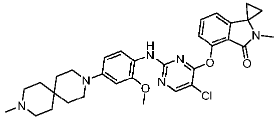 | **589.3** |
| **148** | 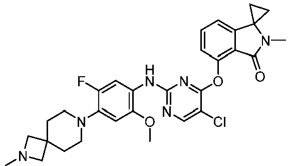 | **579.2** | **149** | 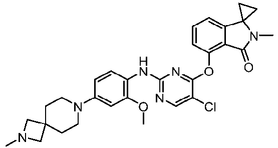 | **561.2** |
| **150** | 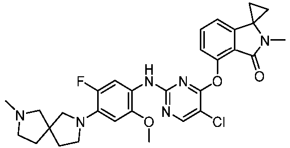 | **579.2** | **151** | 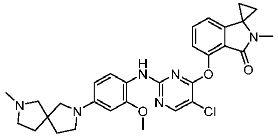 | **561.2** |
| **152** | 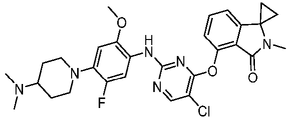 | **567.2** | | | |

### Example 25. Synthesis of 4'-((5-chloro-2-((2-methoxy-4-((4-methylpiperazin-1-yl)methyl)phenyl)amino)pyrimidin-4-yl)oxy)-2'-methylspiro[cyclopropane-1,1'-isoindolin]-3'-one (Compound 153)

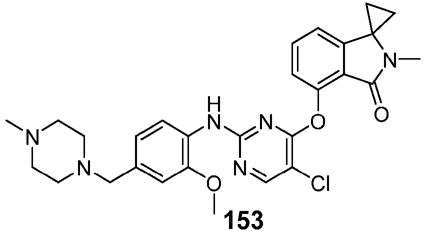

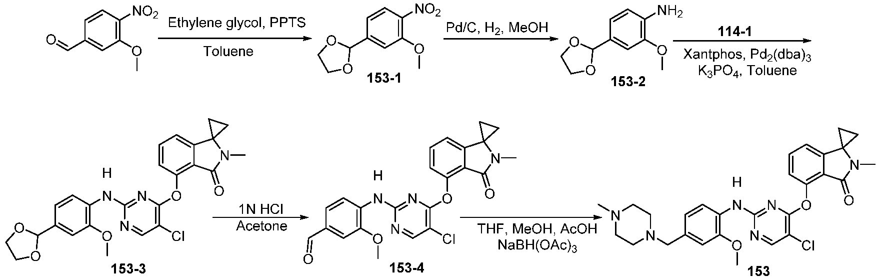

### Synthesis of 153-1:

**3-Methoxy-4-nitrobenzaldehyde** (1.81 g, 10.0 mmol), ethylene glycol (5 mL), PPTS (1.26 g, 5.0 mmol), and methylbenzene (50 mL) were added to a 100 mL single-neck flask. The mixed solution was purged with argon, heated to 130 °C, refluxed to remove water, and stirred for 20 h. After the completion of the reaction as detected by LC-MS, the mixed solution was cooled and washed with water (50 mL), and the aqueous phase was extracted with methylbenzene (50 mL). The organic phases were combined, washed twice with a saturated sodium chloride solution (50 mL), dried over anhydrous sodium sulfate, filtered, and concentrated to dryness to give a brown oily product (1.78 g, yield: 79%).

ESI-MS m/z: 226.1 [M+H]⁺.

### Synthesis of 153-2:

The compound **152-1** described above (1.78 g, 7.90 mmol) and 10% Pd/C (200 mg) were added to MeOH (100 mL). The mixture was purged with H₂ 3 times and then stirred at room temperature under normal pressure for 20 h. After the completion of the reaction as detected by LC-MS, the mixed solution was filtered. The filtrate was concentrated to dryness, and the residue was purified by column chromatography to give a pale brown oily product (1.33 g, yield: 86.2%). ESI-MS m/z: 196.1 [M+H]⁺.

### Synthesis of 153-3:

The compound **153-2** described above (174 mg, 0.893 mmol) and compound **114-1** (200 mg, 0.595 mmol), Xantphos (69 mg, 0.119 mmol), and anhydrous potassium phosphate (379 mg, 1.785 mmol) were added to methylbenzene (10 mL). After the mixture was purged with argon, Pd₂(dba)₃ (54 mg, 0.06 mmol) was added. The mixed solution was heated to 100 °C and stirred for 8 h under argon atmosphere. After the completion of the reaction as detected by LC-MS, the mixed solution was directly loaded onto a column and purified by silica gel column chromatography to give a yellowish brown gummy product (232 mg, yield: 78.7%).

ESI-MS m/z: 495.1 [M+H]⁺.

### Synthesis of 153-4:

The compound **153-3** described above (232 mg, 0.469 mmol) and 1 N HCl (1.0 mL) were added to acetone/water (10 mL/3 mL). The mixture was purged with argon, heated to 60 °C, and stirred for 5 h. After the completion of the reaction as detected by LC-MS, EA (10 mL) and water (20 mL) were added to the mixed solution. The resulting mixture was stirred, and liquid separation was performed. The aqueous phase was extracted with EA (10 mL). The organic phases were combined, washed with a saturated sodium bicarbonate solution (10 mL) and a saturated sodium chloride solution (10 mL), dried over anhydrous sodium sulfate, filtered, and concentrated to give a crude product (198 mg, yield: 93.6%).

ESI-MS m/z: 451.1 [M+H]⁺.

### Synthesis of compound 153:

The compound **153-4** described above (45 mg, 0.10 mmol), N-methylpiperazine (20 mg, 0.2 mmol), and glacial acetic acid (24 mg, 0.4 mmol) were added to THF (5 mL), and the mixture was stirred at room temperature for 15 min. NaBH(OAc)₃ (85 mg, 0.4 mmol) was then added, and the mixed solution was stirred at room temperature for another 1 h. After the completion of the reaction as detected by LC-MS, the mixed solution was concentrated, and the residue was purified by Flash to give an off-white solid product (11 mg, yield: 20.6%).

ESI-MS m/z: 535.2 [M+H]⁺.

The target compounds **154-156** in Table 10 were obtained with reference to a synthetic method similar to that in Example 25 using different starting materials.

**Table 10**

| **Compound** | **Structural formula** | **MS: [M+H]⁺** | **Compound** | **Structural formula** | **MS: [M+H]⁺** |
|---|---|---|---|---|---|
| **154** | 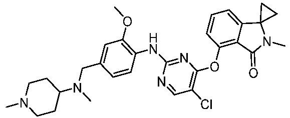 | **563.2** | **155** | 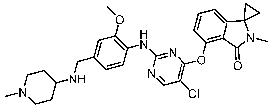 | **549.2** |
| **156** | 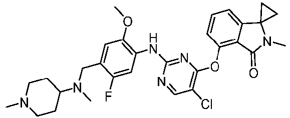 | **581.2** | | | |

### Example 26. Synthesis of 4'-((5-chloro-2-((2-methoxy-4-(3-(4-methylpiperazin-1-yl)azetidin-1-yl)phenyl)amino)pyrimidin-4-yl)oxy)-2'-methylspiro[cyclopropane-1,1'-isoindolin]-3'-one (Compound 157)

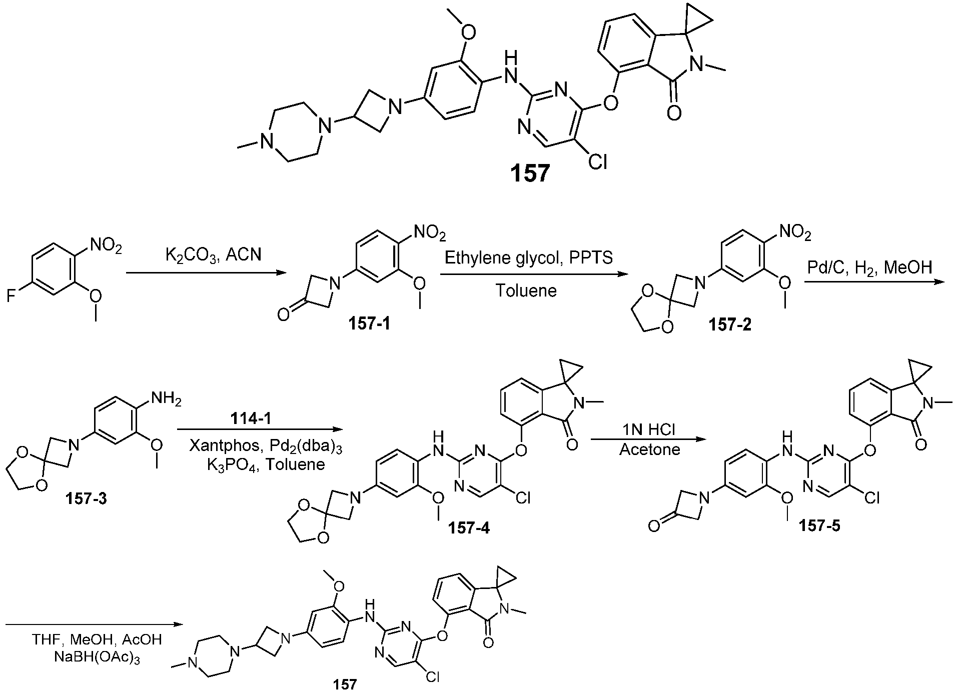

### Synthesis of 157-1:

The compound **4-fluoro-2-methoxy-1-nitrobenzene** described above (855 mg, 5.0 mmol), azetidin-3-ol (730 mg, 10.0 mmol), and anhydrous potassium carbonate (2.07 g, 15 mmol) were added to acetonitrile (50 mL). The mixture was purged with argon, heated to 85 °C, and stirred for 20 h. After the basic completion of the reaction as detected by LC-MS, the mixed solution was concentrated. EA (20 mL) and water (20 mL) were added to the residue. The resulting mixture was stirred, and liquid separation was performed. The aqueous phase was extracted with EA (20 mL). The organic phases were combined, washed with a saturated sodium chloride solution, and concentrated. The residue was purified by column chromatography to give a product (793 mg, yield: 71.4%).

ESI-MS m/z: 223.0 [M+H]⁺.

### Synthesis of 157-2:

The compound **157-1** described above (793 mg, 3.569 mmol), ethylene glycol (2 mL), PPTS (448 mg, 1.784 mmol), and methylbenzene (30 mL) were added to a 100 mL single-neck flask. The mixed solution was purged with argon, heated to 130 °C, refluxed to remove water, and stirred for 20 h. After the completion of the reaction as detected by LC-MS, the mixed solution was cooled and washed with water (50 mL), and the aqueous phase was extracted with methylbenzene (50 mL). The organic phases were combined, washed twice with a saturated sodium chloride solution (50 mL), dried over anhydrous sodium sulfate, filtered, and concentrated to dryness to give a brown solid product (685 mg, yield: 72.1%).

ESI-MS m/z: 267.1 [M+H]⁺.

### Synthesis of 157-3:

The compound **157-2** described above (685 mg, 2.573 mmol) and 10% Pd/C (100 mg) were added to MeOH (30 mL). The mixture was purged with Hz 3 times and then stirred at room temperature under normal pressure for 20 h. After the completion of the reaction as detected by LC-MS, the mixed solution was filtered, and the filtrate was concentrated to dryness to give a purple-ish black solid product (715 mg, yield: >100%).

ESI-MS m/z: 237.1 [M+H]⁺.

### Synthesis of 157-4:

The compound **157-3** described above (715 mg, 2.573 mmol) and compound **114-1** (672 mg, 2.0 mmol), Xantphos (116 mg, 0.2 mmol), and anhydrous potassium phosphate (1.272 g, 6.0 mmol) were added to methylbenzene (50 mL). After the mixture was purged with argon, Pd₂(dba)₃ (93 mg, 0.1 mmol) was added. The mixed solution was heated to 100 °C and stirred for 8 h under argon atmosphere. After the completion of the reaction as detected by LC-MS, the mixed solution was directly loaded onto a column and purified by silica gel column chromatography to give a yellowish brown gummy product (546 mg, yield: 50.9%).

ESI-MS m/z: 536.2 [M+H]⁺.

### Synthesis of 157-5:

The compound **157-4** described above (546 mg, 1.019 mmol) and 1 N HCl (2.0 mL) were added to acetone/water (30 mL/10 mL). The mixture was purged with argon, heated to 60 °C, and stirred for 5 h. After the completion of the reaction as detected by LC-MS, the mixed solution was concentrated until there was about half of the volume left, and EA (30 mL) and water (20 mL) were added to the residue. The resulting mixture was stirred, and liquid separation was performed. The aqueous phase was extracted with EA (30 mL). The organic phases were combined, washed with a saturated sodium bicarbonate solution (30 mL) and a saturated sodium chloride solution (30 mL), dried over anhydrous sodium sulfate, filtered, and concentrated to give a crude product (387 mg, yield: 77.2%).

ESI-MS m/z: 492.1 [M+H]⁺.

### Synthesis of compound 157:

The compound **157-5** described above (49 mg, 0.10 mmol), N-methylpiperazine (20 mg, 0.2 mmol), and glacial acetic acid (24 mg, 0.4 mmol) were added to THF (5 mL), and the mixture was stirred at room temperature for 15 min. NaBH(OAc)₃ (85 mg, 0.4 mmol) was then added, and the mixed solution was stirred at room temperature for another 1 h. After the completion of the reaction as detected by LC-MS, the mixed solution was concentrated, and the residue was purified by Flash to give an off-white solid product (16 mg, yield: 27.8%).

ESI-MS m/z: 576.2 [M+H]⁺.

The target compounds **158-173** in Table 11 were obtained with reference to synthetic methods similar to those in Examples 23 and 26 using different starting materials.

**Table 11**

| **Compound** | **Structural formula** | **MS: [M+H]⁺** | **Compound** | **Structural formula** | **MS: [M+H]⁺** |
|---|---|---|---|---|---|
| **158** | 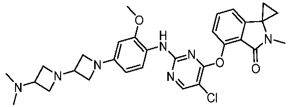 | **576.2** | **159** | 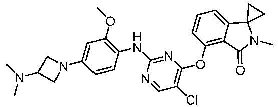 | **521.2** |
| **160** | 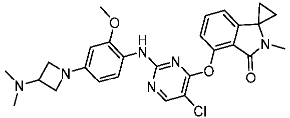 | **590.3** | **161** | 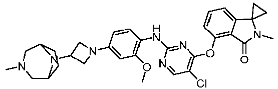 | **602.3** |
| **162** | 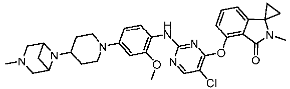 | **614.3** | **163** | 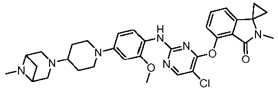 | **616.3** |
| **164** | 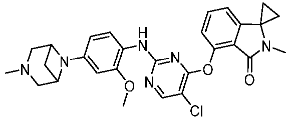 | **533.2** | **165** | 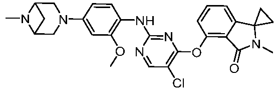 | **533.2** |
| **166** | 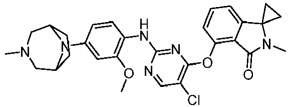 | **547.2** | **167** | 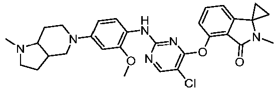 | **561.2** |
| **168** | 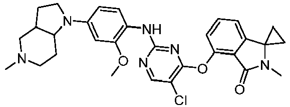 | **561.2** | **169** | 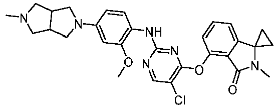 | **547.2** |
| **170** | 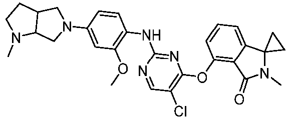 | **547.2** | **171** | 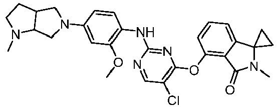 | **547.2** |
| **172** | 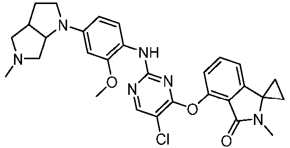 | **547.2** | **173** | 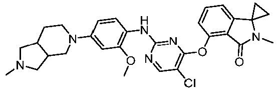 | **561.2** |

### Example 27. Synthesis of 4-((5-bromo-4-((2'-methyl-3'-oxospiro[cyclopropane-1,1'-isoindolin]-4'-yl)oxy)pyrimidin-2-yl)amino)-2-fluoro-5-methoxy-N-(1-methylpiperidin-4-yl)benzamide (Compound 174)

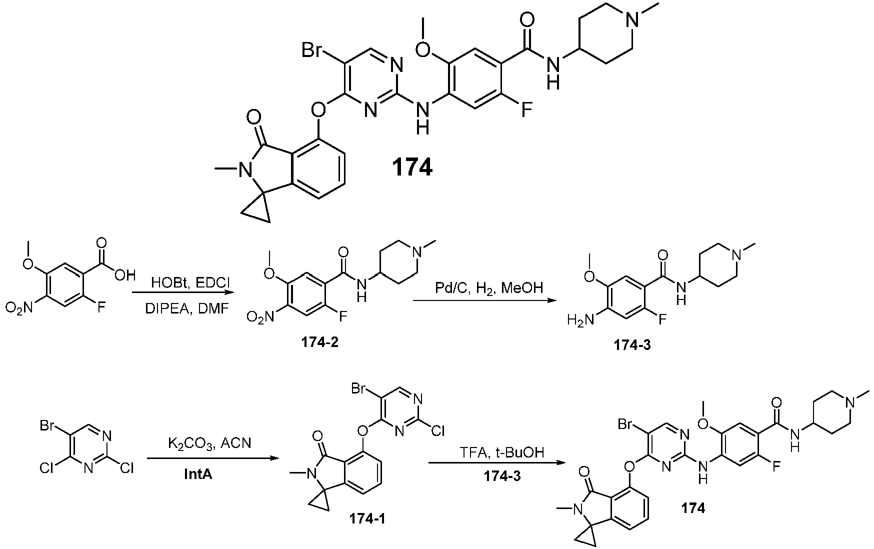

### Synthesis of 174-2:

**2-Fluoro-5-methoxy-4-nitrobenzoic acid** (610 mg, 2.08 mmol), **1-methylpiperidin-4-amine** (260 mg, 2.29 mol), HOBt (420 mg, 3.1 mmol), EDCI (598 mg, 3.1 mmol), and DIPEA (1.1 mL) were added to DMF (10 mL). The mixture was purged with argon and then stirred at room temperature for 20 h. After the completion of the reaction as detected by LC-MS, water (20 mL) and EA (30 mL) were added to the mixed solution. The resulting mixture was stirred, and liquid separation was performed. The aqueous phase was extracted with EA (30 mL). The organic phases were combined, washed twice with a saturated sodium chloride solution (30 mL), dried over anhydrous sodium sulfate, filtered, and concentrated to dryness to give a brown oily product (530 mg, yield: 81.9%).

ESI-MS m/z: 312.1 [M+H]⁺.

### Synthesis of 174-3:

The **174-2** described above (530 mg, crude, 1.703 mmol) and 10% Pd/C (50 mg, containing 50%-55% water) were added to MeOH (20 mL). The mixed solution was purged with hydrogen three times and then vigorously stirred at room temperature under normal pressure for 20 h. After the completion of the reaction as detected by LC-MS, the mixed solution was filtered through diatomite. The filter cake was rinsed with MeOH, and the filtrate was concentrated to dryness to give a pale gray solid product (483 mg, yield: 100%).

ESI-MS m/z: 282.1 [M+H]⁺.

### Synthesis of 174-1:

The compounds **2,4-dichloro-5-bromopyrimidine** (455 mg, 2.0 mmol) and **IntA** (378 mg, 2.0 mmol) were added to DMF (5 mL), and the mixture was cooled to 0-5 °C in an ice bath under argon atmosphere. Potassium carbonate (553 mg, 4.0 mmol) was then added. The mixed solution was stirred at 0-5 °C for 1 h. After the completion of the reaction as detected by LC-MS, water (10 mL) was added to the mixed solution. The resulting mixture was stirred for 15 min and then filtered. The filter cake was washed with water and then dried under vacuum to give a product (667 mg, yield: 87.6%).

ESI-MS m/z: 380.0 [M+H]⁺.

### Synthesis of compound 174:

The compound **174-1** described above (50 mg, 0.131 mmol) and **174-3** (42 mg, 0.148 mmol) were added to *tert*-butanol (3 mL), and TFA (19 mg, 0.169 mmol) was added at room temperature. The mixed solution was purged with argon, heated to 95 °C, and stirred for 8 h. A reaction product was detected by LC-MS. The mixture was concentrated, and the residue was purified by Flash followed by preparative HPLC and lyophilized to give an off-white solid product (5 mg, yield: 6.1%).

ESI-MS m/z: 625.1 [M+H]⁺.

The target compounds **175-176** in Table 12 were obtained with reference to a synthetic method similar to that in Example 27 using different starting materials.

**Table 12**

| **Compound** | **Structural formula** | **MS: [M+H]⁺** | **Compound** | **Structural formula** | **MS: [M+H]⁺** |
|---|---|---|---|---|---|
| **175** | 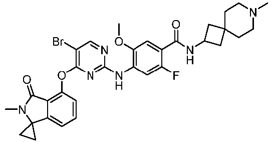 | **665.2** | **176** | 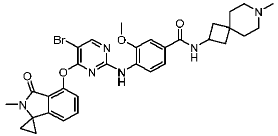 | **647.2** |

### Example 28. Synthesis of 4'-((5-bromo-2-((2-methoxy-4-(7-methyl-2,7-diazaspiro[3.5] nonan-2-yl)phenyl)amino)pyrimidin-4-yl)oxy)-2'-methylspiro[cyclopropane-1,1'-isoindolin]-3'-one (Compound 177)

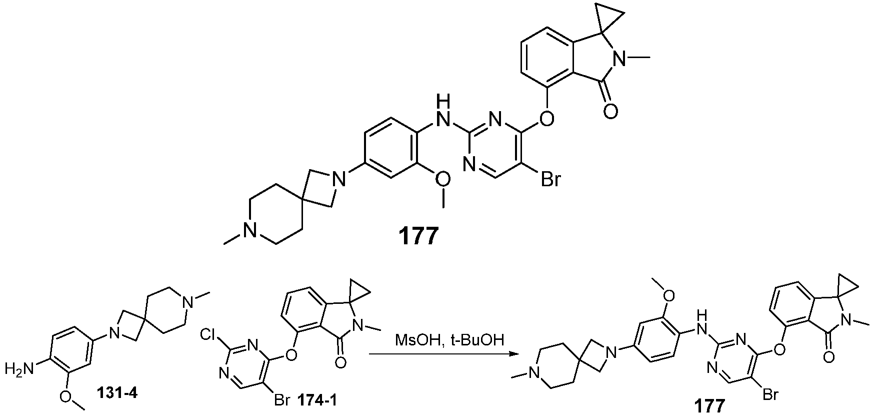

### Synthesis of compound 177:

The compound **174-1** described above (50 mg, 0.131 mmol) and **131-4** (41 mg, 0.157 mmol) were added to *t*-BuOH (5 mL), and MsOH (25 mg, 0.260 mmol) was added at room temperature. The mixed solution was purged with argon, heated to 90 °C, and stirred for 6 h. A reaction product was detected by LC-MS. The mixture was concentrated. The residue was purified by Flash, and the impure product was further purified by pre-HPLC and lyophilized to give an off-white solid product (9 mg, yield: 11.3%).

ESI-MS m/z: 605.2 [M+H]⁺.

The target compounds **178-191** in Table 13 were obtained with reference to a synthetic method similar to that in Example 28 using different starting materials.

**Table 13**

| **Compound** | **Structural formula** | **MS: [M+H]⁺** | **Compound** | **Structural formula** | **MS: [M+H]⁺** |
|---|---|---|---|---|---|
| **178** | 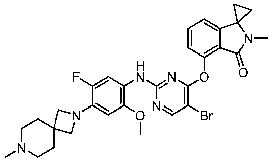 | **623.2** | **179** | 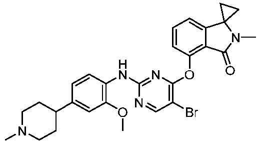 | **564.1** |
| **180** | 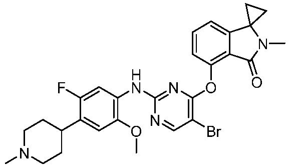 | **582.1** | **181** | 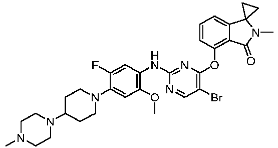 | **666.2** |
| **182** | 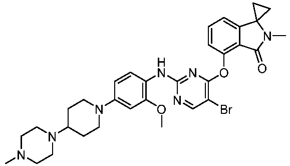 | **648.2** | **183** | 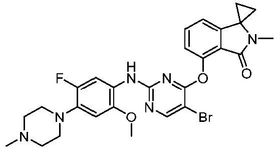 | **583.1** |
| **184** | 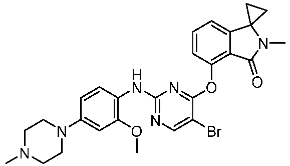 | **565.1** | **185** | 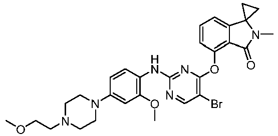 | **609.2** |
| **186** | 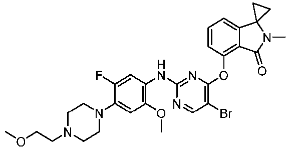 | **627.2** | **187** | 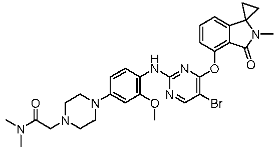 | **636.2** |
| **188** | 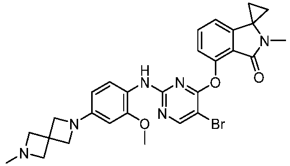 | **577.1** | **189** | 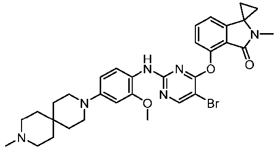 | **633.2** |
| **190** | 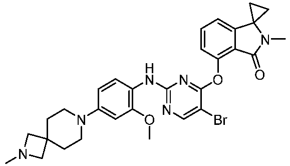 | **605.2** | **191** | 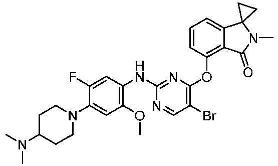 | **611.2** |

### Example 29. Synthesis of 4-((5-cyano-4-((2'-methyl-3'-oxospiro[cyclopropane-1,1'-isoindolin]-4'-yl)oxy)pyrimidin-2-yl)amino)-3-methoxy-N-(1-methylpiperidin-4-yl)benzamide (Compound 192)

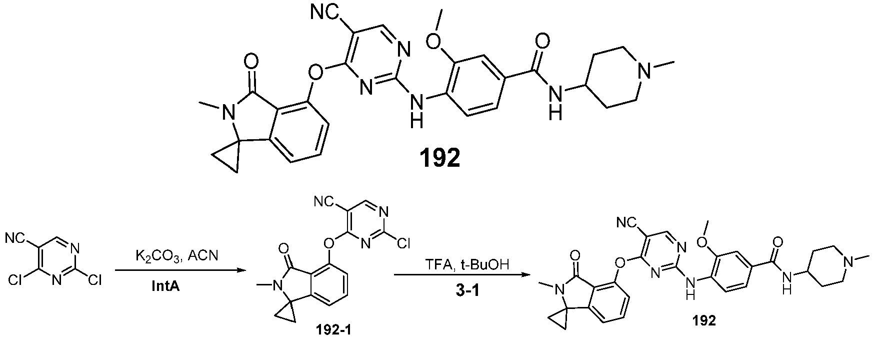

### Synthesis of 192-1:

The compounds **2,4-dichloro-5-cyanopyrimidine** (696 mg, 4.0 mmol) and **IntA** (756 mg, 4.0 mmol) were added to DMF (10 mL), and the mixture was cooled to 0-5 °C in an ice bath under argon atmosphere. Potassium carbonate (1.11 g, 8.0 mmol) was then added. The mixed solution was stirred at 0-5 °C for 1 h. After the completion of the reaction as detected by LC-MS, water (20 mL) was added to the mixed solution. The resulting mixture was stirred for 15 min and then filtered. The filter cake was washed with water and then purified by column chromatography to give an off-white solid product (747 mg, yield: 57.2%).

ESI-MS m/z: 327.0 [M+H]⁺.

### Synthesis of compound 192:

The compound **192-1** described above (50 mg, 0.153 mmol) and **3-1** (48 mg, 0.184 mmol) were added to *tert*-butanol (3 mL), and TFA (35 mg, 0.306 mmol) was added at room temperature. The mixed solution was purged with argon, heated to 95 °C, and stirred for 8 h. A reaction product was detected by LC-MS. The mixture was concentrated, and the residue was purified by preparative HPLC and lyophilized to give an off-white solid product (16 mg, yield: 18.9%).

ESI-MS m/z: 554.3 [M+H]⁺.

The target compounds **193-195** in Table 14 were obtained with reference to a synthetic method similar to that in Example 29 using different starting materials.

**Table 14**

| **Compound** | **Structural formula** | **MS: [M+H]⁺** | **Compound** | **Structural formula** | **MS: [M+H]⁺** |
|---|---|---|---|---|---|
| **193** | 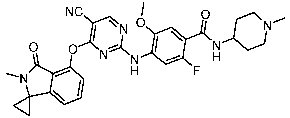 | **572.2** | **194** | 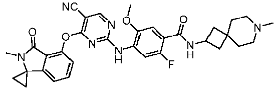 | **612.3** |
| **195** | 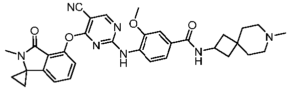 | **594.3** | | | |

### Example 30. Synthesis of 2-((2-methoxy-4-(7-methyl-2,7-diazaspiro[3.5]nonan-2-yl)phenyl)amino)-4-((2'-methyl-3'-oxospiro [cyclopropane-1,1'-isoindolin]-4'-yl)oxy)pyrimidine-5-carbonitrile (Compound 196)

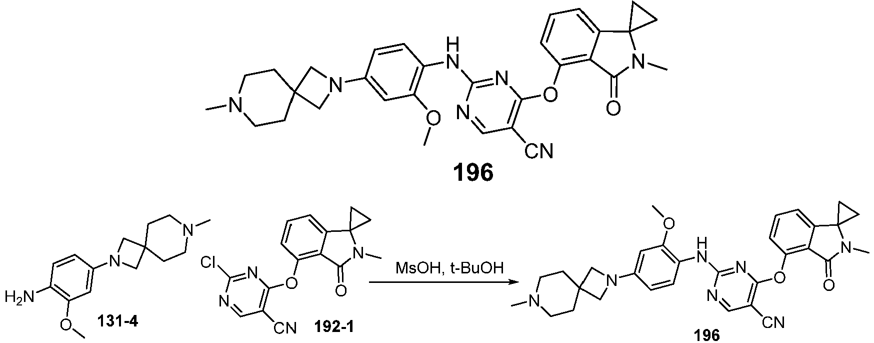

### Synthesis of compound 196:

The compound **192-1** described above (50 mg, 0.153 mmol) and **131-4** (41 mg, 0.157 mmol) were added to *t*-BuOH (5 mL), and MsOH (25 mg, 0.260 mmol) was added at room temperature. The mixed solution was purged with argon, heated to 90 °C, and stirred for 6 h. A reaction product was detected by LC-MS. The mixture was concentrated. The residue was purified by pre-HPLC and lyophilized to give an off-white solid product (14 mg, yield: 16.6%).

ESI-MS m/z: 552.3 [M+H]⁺.

The target compounds **197-202** in Table 15 were obtained with reference to a synthetic method similar to that in Example 30 using different starting materials.

**Table 15**

| **Compound** | **Structural formula** | **MS: [M+H]⁺** | **Compound** | **Structural formula** | **MS: [M+H]⁺** |
|---|---|---|---|---|---|
| **197** | 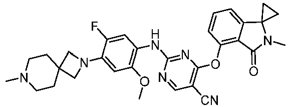 | **570.3** | **198** | 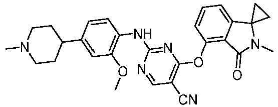 | **511.2** |
| **199** | 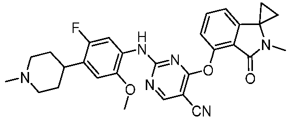 | **529.2** | **200** | 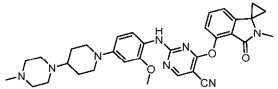 | **595.3** |
| **201** | 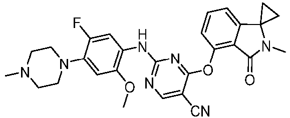 | **530.2** | **202** | 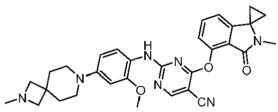 | **552.2** |

### Example 31. Synthesis of 4'-((5-chloro-2-((2-methoxy-4-(7-methyl-2,7-diazaspiro[3.5] nonan-2-yl)phenyl)amino)pyrimidin-4-yl)oxy)-2'-methylspiro[cyclobutane-1,1'-isoindolin]-3'-one (Compound 203)

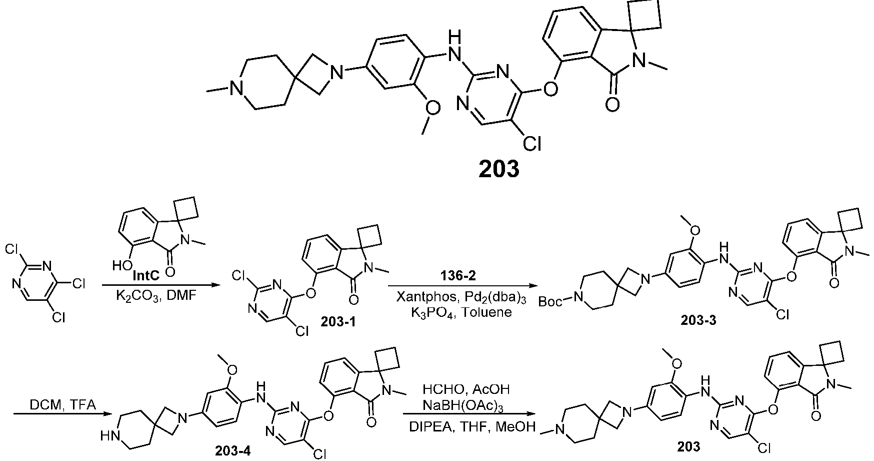

### Synthesis of 203-1:

The compounds **2,4,5-trichloropyrimidine** (500 mg, 2.73 mmol) and **IntC** (555 mg, 2.73 mmol) were added to DMF (5 mL), and the mixture was cooled to 0-5 °C in an ice bath under argon atmosphere. Potassium carbonate (755 mg, 5.46 mmol) was then added. The mixed solution was stirred at 0-5 °C for 1 h. After the completion of the reaction as detected by LC-MS, water (10 mL) was added to the mixed solution. The resulting mixture was stirred for 15 min and then filtered. The filter cake was washed with water and then dried under vacuum to give a product (767 mg, yield: 80.4%).

ESI-MS m/z: 350.0 [M+H]⁺.

### Synthesis of 203-3:

The compound **136-2** described above (269 mg, 0.773 mmol) and compound **203-1** (208 mg, 0.595 mmol), Xantphos (69 mg, 0.119 mmol), and anhydrous potassium phosphate (379 mg, 1.785 mmol) were added to methylbenzene (10 mL). After the mixture was purged with argon, Pd₂(dba)₃ (54 mg, 0.06 mmol) was added. The mixed solution was heated to 100 °C and stirred for 8 h under argon atmosphere. After the completion of the reaction as detected by LC-MS, the mixed solution was directly loaded onto a column and purified by silica gel column chromatography to give a yellowish brown solid product (136 mg, yield: 34.6%).

ESI-MS m/z: 661.3 [M+H]⁺.

### Synthesis of 203-4:

The compound **203-3** described above (130 mg, 0.197 mmol) and TFA (0.3 mL) were added to DCM (5 mL). The mixture was purged with argon and stirred at room temperature for 2 h. After the completion of the reaction as detected by LC-MS, the mixed solution was concentrated to give a crude product (211 mg, yield: >100%).

ESI-MS m/z: 560.2 [M+H]⁺.

### Synthesis of compound 203:

The compound **203-4** described above (50 mg, crude, 0.047 mmol) and DIPEA (46 mg, 0.36 mmol) were added to THF (5 mL)/MeOH (1 mL). The mixed solution was stirred at room temperature for 10 min. Glacial acetic acid (6 mg, 0.10 mmol) and an aqueous formaldehyde solution (8 mg, 37%, 0.10 mmol) were then added, and the mixed solution was stirred at room temperature for 20 min. NaBH(OAc)₃ (36 mg, 0.17 mmol) was then added, and the mixed solution was stirred at room temperature for another 1 h. After the completion of the reaction as detected by LC-MS, the mixed solution was concentrated, and the residue was purified by Flash to give a yellow solid product (7 mg, yield: 25.9%).

ESI-MS m/z: 575.2 [M+H]⁺.

The target compounds **204-218** in Table 16 were obtained with reference to a synthetic method similar to that in Example 31 using different starting materials.

**Table 16**

| **Compound** | **Structural formula** | **MS: [M+H]⁺** | **Compound** | **Structural formula** | **MS: [M+H]⁺** |
|---|---|---|---|---|---|
| **204** | 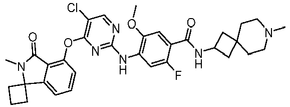 | **635.3** | **205** | 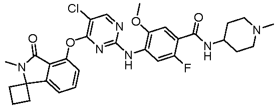 | **595.2** |
| **206** | 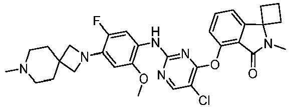 | **593.2** | **207** | 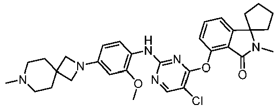 | **589.2** |
| **208** | 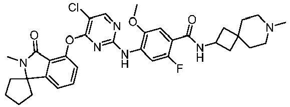 | **649.3** | **209** | 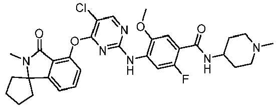 | **608.2** |
| **210** | 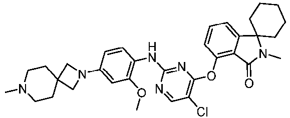 | **603.3** | **211** | 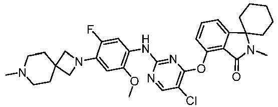 | **621.3** |
| **212** | 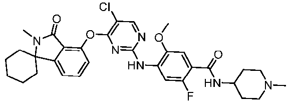 | **623.3** | **213** | 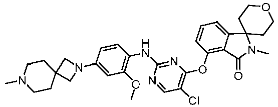 | **605.3** |
| **214** | 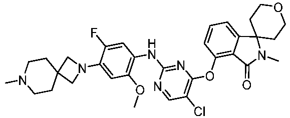 | **623.3** | **215** | 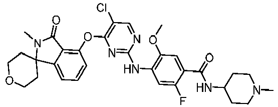 | **625.2** |
| **216** | 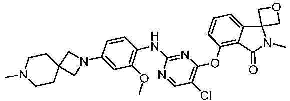 | **577.2** | **217** | 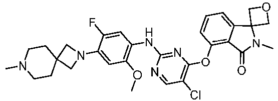 | **595.2** |
| **218** | 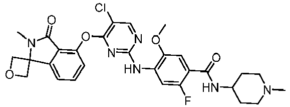 | **597.2** | | | |

**The nuclear magnetic resonance data of some of the compounds of this patent are shown in Table 17 below.**

**Table 17**

| **Compound No.** | **NMR data** |
|---|---|
| **3** | ¹H NMR (400 MHz, CDCl₃) δ 8.55 (d, *J* = 8.4 Hz, 1H), 7.94 (s, 1H), 7.64 (t, *J* = 7.8 Hz, 1H), 7.25 (s, 2H), 7.21 (d, *J* = 8.0 Hz, 1H), 7.09 (d, *J* = 7.7 Hz, 1H), 6.85 (s, 1H), 6.30 - 6.21 (m, 1H), 4.07 (s, 1H), 3.88 (s, 3H), 3.17 (d, *J* = 12.0 Hz, 2H), 2.69 (s, 3H), 2.51 (s, 3H), 2.46 (t, *J* = 11.8 Hz, 2H), 2.08 (d, *J* = 12.9 Hz, 2H), 1.88 (q, *J* = 12.0 Hz, 2H), 1.60 (t, *J* = 7.0 Hz, 2H), 1.44 (t, *J* = 4.1 Hz, 2H). |
| **4** | ¹H NMR (400 MHz, CDCl₃) δ 8.60 (s, 1H), 8.03 (s, 1H), 7.66 (t, J = 7.9 Hz, 1H), 7.43 (d, J = 7.0 Hz, 1H), 7.22 (d, J = 7.9 Hz, 1H), 7.12 (d, J = 7.7 Hz, 1H), 6.52 (s, 1H), 4.07 (s, 1H), 3.88 (s, 3H), 3.02 (m, 2H), 2.71 (s, 3H), 2.59 - 2.29 (m, 5H), 2.09 (d, J = 13.0 Hz, 2H), 1.81 (s, 2H), 1.61 - 1.54 (m, 2H), 1.42 (d, J = 6.4 Hz, 2H). |
| **27** | ¹H NMR (400 MHz, CDCl₃) δ 8.56 (s, 1H), 7.96 - 7.85 (m, 1H), 7.63 (t, *J =* 7.7 Hz, 1H), 7.24 (s, 1H), 7.21 (d, *J* = 7.9 Hz, 1H), 7.06 (dd, *J* = 7.7, 3.5 Hz, 1H), 6.83 (s, 1H), 6.23 (d, *J* = 7.2 Hz, 1H), 4.66 (s, 1H), 4.04 (ddd, *J* = 24.3, 9.7, 5.7 Hz, 1H), 3.87 (s, 3H), 3.75 (ddd, *J* = 16.0, 9.6, 3.3 Hz, 1H), 2.80 (tt, J = 11.7, 8.1 Hz, 2H), 2.69 (s, 3H), 2.60 - 2.52 (m, 1H), 2.51 - 2.39 (m, 2H), 2.38 (d, *J* = 3.0 Hz, 3H), 2.11 (ddd, J = 23.4, 12.0, 6.1 Hz, 2H), 2.01-1.86 (m, 2H), 1.60 (q, *J* = 5.8 Hz, 2H), 1.41 (hept, *J* = 2.0 Hz, 2H). |
| **71** | ¹H NMR (400 MHz, DMSO-d₆) δ 9.92 (s, 2H), 8.62 (s, 1H), 7.64 (d, J = 8.0 Hz, 1H), 7.25 (t, J= 12.8 Hz, 2H), 6.88 (d, *J* = 43.5 Hz, 2H), 6.43 (s, 1H), 2.65 (s, 3H), 2.31 (s, 2H), 1.72 (s, 2H), 1.46 (s, 2H), 1.22 (s, 2H). |
| **73** | ¹H NMR (400 MHz, CDCl₃) δ 8.49 (s, 1H), 7.59 (s, 2H), 7.35 - 6.89 (m, 3H), 6.42 (s, 1H), 5.93 (s, 1H), 3.78 (s, 3H), 3.08 (s, 4H), 2.71 (s, 3H), 2.57 (s, 4H), 2.35 (s, 3H), 1.74 - 1.30 (m, 4H). |
| **77** | ¹H NMR (400 MHz, CDCl₃) δ 8.50 (s, 1H), 7.60 (t, J = 7.8 Hz, 1H), 7.26 (s, 2H), 7.20 (d, J = 8.0 Hz, 1H), 7.04 (d, J = 7.7 Hz, 1H), 6.41 (s, 1H), 5.94 (s, 1H), 3.85 (t, J = 4.9 Hz, 4H), 3.80 (s, 3H), 3.04 (d, J = 5.7 Hz, 4H), 2.73 (s, 3H), 1.64 - 1.56 (m, 2H), 1.40 (t, J = 4.0 Hz, 2H). |
| **78** | ¹H NMR (400 MHz, CDCl₃) δ 8.49 (s, 1H), 7.60 (t, J = 7.8 Hz, 1H), 7.27 - 7.25 (m, 2H), 7.19 (d, J = 7.9 Hz, 1H), 7.04 (d, J = 7.6 Hz, 1H), 6.42 (s, 1H), 5.94 (s, 1H), 3.79 (s, 3H), 3.55 (d, J = 11.8 Hz, 2H), 2.72 (s, 3H), 2.70 - 2.41 (m, 9H), 2.32 (s, 3H), 1.93 (d, J = 12.3 Hz, 2H), 1.75 - 1.63 (m, 4H), 1.63 - 1.56 (m, 2H), 1.42 - 1.36 (m, 2H). |
| **79** | ¹H NMR (400 MHz, CDCl₃) δ 8.47 (s, 1H), 7.58 (t, *J =* 7.8 Hz, 1H), 7.26 (s, 2H), 7.18 (d, *J* = 7.9 Hz, 1H), 7.01 (d, *J* = 7.7 Hz, 1H), 5.91 (d, *J* = 2.4 Hz, 1H), 5.47 (s, 1H), 3.77 (s, 3H), 3.52 (s, 4H), 2.73 (s, 3H), 2.51 - 2.30 (m, 4H), 2.29 (s, 3H), 1.85 (t, *J* = 5.3 Hz, 4H), 1.61 - 1.58 (m, 2H), 1.42 - 1.36 (m, 2H). |
| **87** | ¹H NMR (400 MHz, DMSO-d₆) δ 9.96 (s, 1H), 8.64 (s, 1H), 7.69 (t, J = 8.0 Hz, 1H), 7.33 (d, J = 6.7 Hz, 1H), 7.28 (d, J = 8.0 Hz, 1H), 6.69 (d, J = 32.9 Hz, 2H), 2.70 (s, 3H), 2.63 (m, 3H), 2.50 (d, J = 10.6 Hz, 3H), 2.29 (s, 3H), 1.72 (q, J = 5.5 Hz, 3H), 1.41 (q, J = 5.6 Hz, 2H), 0.79 (d, J = 8.2 Hz, 2H), 0.35 (s, 2H). |
| **89** | ¹H NMR (400 MHz, DMSO-d₆) δ 10.01 (s, 1H), 8.65 (s, 1H), 7.68 (t, J = 7.8 Hz, 1H), 7.33 (d, J = 7.6 Hz, 1H), 7.26 (d, J = 8.0 Hz, 1H), 6.41 (d, J = 41.4 Hz, 2H), 4.26 (d, J = 10.7 Hz, 1H), 4.02 (t, J = 11.6 Hz, 1H), 3.15 (d, J = 5.2 Hz, 1H), 2.86 (t, J = 13.1 Hz, 2H), 2.64 (s, 3H), 2.29 (s, 3H), 1.85 - 1.65 (m, 4H), 1.52 - 1.26 (m, 4H). |
| **114** | ¹H NMR (400 MHz, CDCl₃) δ 8.33 (s, 1H), 7.71 (s, 1H), 7.68 - 7.59 (m, 2H), 7.25 - 7.20 (m, 2H), 7.06 (d, J = 7.7 Hz, 1H), 6.83 (d, J = 8.6 Hz, 1H), 5.87 (d, J = 8.0 Hz, 1H), 3.97 (s, 1H), 3.87 (s, 3H), 2.88 (d, J = 11.4 Hz, 2H), 2.70 (s, 3H), 2.34 (s, 3H), 2.20 (t, J = 11.6 Hz, 2H), 2.05 (d, J = 12.5 Hz, 2H), 1.87 (s, 2H), 1.66 - 1.59 (m, 2H), 1.45 - 1.38 (m, 2H). |
| **117** | ¹H NMR (400 MHz, CDCl₃) δ 8.35 (s, 1H), 7.81 (s, 1H), 7.64 (t, *J* = 7.8 Hz, 1H), 7.41 (d, *J* = 7.3 Hz, 1H), 7.35 (s, 1H), 7.22 (d, *J =* 8.0 Hz, 1H), 7.06 (d, *J =* 7.7 Hz, 1H), 6.50 (dd, *J* = 15.2, 7.7 Hz, 1H), 3.98 (s, 1H), 3.85 (s, 3H), 2.80 (d, *J* = 11.4 Hz, 2H), 2.71 (s, 3H), 2.31 (s, 3H), 2.18 (t, *J* = 11.4 Hz, 2H), 2.03 (d, *J* = 12.2 Hz, 2H), 1.97 (s, 2H), 1.61-1.54 (m, 2H), 1.42 - 1.35 (m, 2H). |
| **120** | ¹H NMR (400 MHz, CDCl₃) δ 8.48 (s, 1H), 7.65 (s, 1H), 7.58 (t, *J* =7.9 Hz, 1H), 7.26 - 7.22 (m, 1H), 7.17 (d, *J =* 8.0 Hz, 1H), 7.03 (d, *J* = 7.6 Hz, 1H), 5.90 (d, *J =* 8.1 Hz, 1H), 3.76 (s, 3H), 3.60 (s, 4H), 2.73 (s, 3H), 2.37 (m, 4H), 2.28 (s, 3H), 1.85 (t, *J* = 5.0 Hz, 4H), 1.60 - 1.54 (m, 2H), 1.43 - 1.38 (m, 2H). |
| **136** | ¹H NMR (400 MHz, CDCl₃) δ 8.23 (s, 1H), 7.58 (t, *J* = 7.8 Hz, 1H), 7.31-7.25 (m, 2H), 7.20 (d, *J* = 8.0 Hz, 1H), 7.01 (d, *J* = 7.7 Hz, 1H), 5.91 (d, *J* = 2.3 Hz, 1H), 5.58 (s, 1H), 3.76 (s, 3H), 3.51 (s, 4H), 2.73 (s, 3H), 2.39 (m, 4H), 2.29 (s, 3H), 1.84 (m, 4H), 1.62 - 1.57 (m, 2H), 1.43 - 1.36 (m, 2H). |
| **137** | ¹H NMR (400 MHz, CDCl₃) δ 8.25 (d, *J* = 0.9 Hz, 1H), 7.59 (t, *J* = 7.8 Hz, 1H), 7.38 (s, 1H), 7.20 (d, *J* = 7.9 Hz, 1H), 7.11 (d, *J* = 12.1 Hz, 1H), 7.02 (d, *J* = 7.7 Hz, 1H), 5.91 (d, *J =* 8.4 Hz, 1H), 3.75 (s, 3H), 3.59 (s, 4H), 2.73 (s, 3H), 2.53 - 2.32 (m, 4H), 2.31 (s, 3H), 1.90 - 1.83 (m, 4H), 1.56 (t, *J* = 7.1 Hz, 2H), 1.41 (t, *J* = 7.1 Hz, 2H). |
| **154** | ¹H NMR (400 MHz, CDCl₃) δ 8.29 (s, 1H), 7.61 (d, *J* = 7.8 Hz, 1H), 7.58 (d, *J* = 4.7 Hz, 1H), 7.43 (d, *J* = 8.3 Hz, 1H), 7.22 (d, *J* = 8.0 Hz, 1H), 7.03 (d, *J* = 7.7 Hz, 1H), 6.77 (s, 1H), 6.37 (d, *J* = 8.3 Hz, 1H), 3.81 (s, 3H), 3.45 (s, 2H), 2.93 (d, *J =* 11.1 Hz, 2H), 2.71 (s, 3H), 2.42 (d, *J =* 12.0 Hz, 1H), 2.28 (s, 3H), 2.15 (s, 3H), 1.95 (t, *J =* 12 Hz, 2H), 1.76 (t, *J* = 11.9 Hz, 2H), 1.68 (d, *J =* 11.9 Hz, 2H), 1.61 - 1.56 (m, 2H), 1.43 - 1.37 (m, 2H). |
| **196** | 1H NMR (400 MHz, Chloroform-d) δ 8.49 (s, 1H), 7.79 (s, 1H), 7.59 (t, J = 7.9 Hz, 1H), 7.18 (d, J = 8.0 Hz, 1H), 7.03 (d, J = 7.9 Hz, 2H), 5.89 (s, 1H), 5.36 (d, J = 12.8 Hz, 1H), 3.77 (s, 3H), 3.52 (s, 4H), 2.72 (s, 3H), 2.50 (m, 4H), 2.37 (s, 3H), 1.91 (m, 4H), 1.65 - 1.59 (m, 2H), 1.43 - 1.38 (m, 2H). |
| **197** | 1H NMR (400 MHz, Chloroform-d) δ 8.50 (s, 1H), 7.82 (s, 1H), 7.60 (t, J = 7.8 Hz, 1H), 7.18 (d, J = 7.9 Hz, 1H), 7.04 (d, J = 7.7 Hz, 1H), 6.92 (d, J = 14.3 Hz, 1H), 5.88 (d, J = 8.0 Hz, 1H), 3.76 (s, 3H), 3.62 (s, 4H), 2.72 (s, 3H), 2.56 (m, 4H), 2.42 (s, 3H), 1.98 (m, 4H), 1.58 (t, J = 7.0 Hz, 2H), 1.41 (t, J = 7.0 Hz, 2H). |

### Biological Example 1. Assay for Inhibitory Activity of the Compounds of the Present Invention Against FAK Enzyme

A compound serially diluted with DMSO was mixed with a FAK recombinant protein. After the mixture was left to stand at room temperature for 10 min, a biotin-labeled TK substrate (TK) and ATP were added. After 40 min of reaction at room temperature, Sa-XL 665 and a Crytate-labeled TK antibody were added. After 1 h of incubation at room temperature, the fluorescence intensities at 615 nm and 665 nm were measured. The ratio of the fluorescence intensities at 665 nm and 615 nm was calculated. The inhibition percentage and IC₅₀ of the compound were calculated compared to the DMSO control group. The results are shown in Table 18 below.

**Table 18. The inhibitory activity of the compounds of the present invention against FAK enzyme (IC₅₀, nM)**

| **Compound** | **IC₅₀** | **Compound** | **IC₅₀** | **Compound** | **IC₅₀** | **Compound** | **IC₅₀** | **Compound** | **IC₅₀** |
|---|---|---|---|---|---|---|---|---|---|
| **1** | A | **46** | A | **91** | A | **136** | A | **181** | A |
| **2** | A | **47** | A | **92** | A | **137** | A | **182** | A |
| **3** | A | **48** | A | **93** | A | **138** | A | **183** | A |
| **4** | A | **49** | A | **94** | A | **139** | A | **184** | A |
| **5** | A | **50** | A | **95** | A | **140** | A | **185** | A |
| **6** | A | **51** | A | **96** | A | **141** | A | **186** | A |
| **7** | A | **52** | A | **97** | A | **142** | A | **187** | A |
| **8** | A | **53** | A | **98** | A | **143** | A | **188** | A |
| **9** | A | **54** | A | **99** | A | **144** | A | **189** | A |
| **10** | A | **55** | A | **100** | A | **145** | A | **190** | A |
| **11** | A | **56** | A | **101** | A | **146** | A | **191** | A |
| **12** | A | **57** | A | **102** | A | **147** | A | **192** | A |
| **13** | A | **58** | A | **103** | A | **148** | A | **193** | A |
| **14** | A | **59** | A | **104** | A | **149** | A | **194** | A |
| **15** | A | **60** | A | **105** | A | **150** | A | **195** | A |
| **16** | A | **61** | A | **106** | A | **151** | A | **196** | A |
| **17** | A | **62** | A | **107** | A | **152** | A | **197** | A |
| **18** | A | **63** | A | **108** | A | **153** | A | **198** | A |
| **19** | A | **64** | A | **109** | A | **154** | A | **199** | A |
| **20** | A | **65** | A | **110** | A | **155** | A | **200** | A |
| **21** | A | **66** | A | **111** | A | **156** | A | **201** | A |
| **22** | A | **67** | A | **112** | A | **157** | A | **202** | A |
| **23** | A | **68** | A | **113** | A | **158** | A | **203** | A |
| **24** | A | **69** | A | **114** | A | **159** | A | **204** | A |
| **25** | A | **70** | A | **115** | A | **160** | A | **205** | A |
| **26** | A | **71** | A | **116** | A | **161** | A | **206** | A |
| **27** | A | **72** | A | **117** | A | **162** | A | **207** | A |
| **28** | A | **73** | A | **118** | A | **163** | A | **208** | A |
| **29** | A | **74** | A | **119** | A | **164** | A | **209** | A |
| **30** | A | **75** | A | **120** | A | **165** | A | **210** | A |
| **31** | A | **76** | A | **121** | A | **166** | A | **211** | A |
| **32** | A | **77** | A | **122** | A | **167** | A | **212** | A |
| **33** | A | **78** | A | **123** | A | **168** | A | **213** | A |
| **34** | A | **79** | A | **124** | A | **169** | A | **214** | A |
| **35** | A | **80** | A | **125** | A | **170** | A | **215** | A |
| **36** | A | **81** | A | **126** | A | **171** | A | **216** | A |
| **37** | A | **82** | A | **127** | A | **172** | A | **217** | A |
| **38** | A | **83** | A | **128** | A | **173** | A | **218** | A |
| **39** | A | **84** | A | **129** | A | **174** | A | **BI853520** | A |
| **40** | A | **85** | A | **130** | A | **175** | A | **Compound A** | A |
| **41** | A | **86** | A | **131** | A | **176** | A | **Compound B** | A |
| **42** | A | **87** | A | **132** | A | **177** | A | | |
| **43** | A | **88** | A | **133** | A | **178** | A | | |
| **44** | A | **89** | A | **134** | A | **179** | A | | |
| **45** | A | **90** | A | **135** | A | **180** | A | | |

| | | | | | | | | | |
|---|---|---|---|---|---|---|---|---|---|
| A represents IC₅₀ < 20 nM B represents 20 nM ≤ IC₅₀ ≤ 100 nM C represents IC₅₀ > 100 nM | | | | | | | | | |

### Biological Example 2. Assay for Inhibitory Activity of the Compounds of the Present Invention Against PC3 Cell Proliferation

2D activity assay: 1200 PC3 cells were seeded in a 96-well plate. After overnight adherence culture, A compound serially diluted was added. 72 h after addition, Cell Titer-Lumi (Beyotime C0068XL) was added to measure the ATP content in the cells. The growth of the cells was evaluated, and the IC₅₀ of the compound inhibiting cell growth was calculated.

3D activity assay: PC3 cells were seeded in a 96-well plate at 1200 cells/well, and 3D culture was performed. 14 days after a compound serially diluted was added, the growth of the cells was measured. The inhibition percentage and IC₅₀ of the compound were calculated compared to the DMSO control group.

The 2D/3D inhibitory activity of the compounds of the present invention against PC3 cells is shown in Table 19 below.

**Table 19. The 2D/3D inhibitory activity of the compounds of the present invention against PC3 cells (IC₅₀, nM)**

| **Compound** | **2D** | **3D** | **Compound** | **2D** | **3D** | **Compound** | **2D** | **3D** | **Compound** | **2D** | **3D** |
|---|---|---|---|---|---|---|---|---|---|---|---|
| **1** | + | 10.79 | **57** | + | B | **113** | + | B | **169** | + | A |
| **2** | + | B | **58** | + | B | **114** | + | 1.09 | **170** | + | B |
| **3** | + | 0.45 | **59** | + | B | **115** | + | C | **171** | + | B |
| **4** | + | 201 | **60** | + | B | **116** | + | B | **172** | + | B |
| **5** | + | A | **61** | + | B | **117** | + | B | **173** | + | A |
| **6** | + | A | **62** | + | B | **118** | + | B | **174** | + | A |
| **7** | + | A | **63** | + | A | **119** | + | A | **175** | + | A |
| **8** | + | 0.96 | **64** | + | B | **120** | + | 0.46 | **176** | + | A |
| **9** | + | A | **65** | + | A | **121** | + | A | **177** | + | 0.38 |
| **10** | + | B | **66** | + | C | **122** | + | A | **178** | + | 0.38 |
| **11** | + | B | **67** | + | B | **123** | + | A | **179** | + | B |
| **12** | + | A | **68** | + | C | **124** | + | A | **180** | + | B |
| **13** | + | A | **69** | + | 8.52 | **125** | + | A | **181** | + | A |
| **14** | + | A | **70** | + | C | **126** | + | A | **182** | + | A |
| **15** | + | A | **71** | + | 5.18 | **127** | + | A | **183** | + | B |
| **16** | + | A | **72** | + | A | **128** | + | A | **184** | + | B |
| **17** | + | A | **73** | >10000 | 1.99 | **129** | + | B | **185** | + | A |
| **18** | + | B | **74** | + | B | **130** | + | B | **186** | + | A |
| **19** | + | B | **75** | + | A | **131** | + | B | **187** | + | A |
| **20** | + | A | **76** | + | A | **132** | + | B | **188** | + | B |
| **21** | + | A | **77** | >10000 | 4.88 | **133** | + | B | **189** | + | A |
| **22** | + | B | **78** | >10000 | 0.78 | **134** | + | A | **190** | + | A |
| **23** | + | B | **79** | >10000 | 0.32 | **135** | + | A | **191** | + | B |
| **24** | + | B | **80** | + | B | **136** | >10000 | 0.60 | **192** | + | A |
| **25** | + | A | **81** | + | B | **137** | >10000 | 1.40 | **193** | + | A |
| **26** | + | A | **82** | + | B | **138** | + | A | **194** | + | A |
| **27** | + | A | **83** | + | C | **139** | + | 1.70 | **195** | + | A |
| **28** | + | B | **84** | + | A | **140** | + | B | **196** | + | 0.33 |
| **29** | + | A | **85** | + | B | **141** | + | B | **197** | + | 2.42 |
| **30** | + | B | **86** | + | B | **142** | + | A | **198** | + | B |
| **31** | + | B | **87** | >10000 | 5.36 | **143** | + | A | **199** | + | B |
| **32** | + | B | **88** | >10000 | 1.78 | **144** | + | A | **200** | + | A |
| **33** | + | A | **89** | >10000 | B | **145** | + | B | **201** | + | A |
| **34** | + | A | **90** | + | B | **146** | + | B | **202** | + | A |
| **35** | + | A | **91** | + | B | **147** | + | A | **203** | + | A |
| **36** | + | A | **92** | + | B | **148** | + | A | **204** | + | A |
| **37** | + | A | **93** | + | B | **149** | + | A | **205** | + | A |
| **38** | + | B | **94** | + | A | **150** | + | A | **206** | + | A |
| **39** | + | A | **95** | + | B | **151** | + | A | **207** | + | B |
| **40** | + | A | **96** | + | A | **152** | + | B | **208** | + | C |
| **41** | + | A | **97** | + | B | **153** | + | A | **209** | + | C |
| **42** | + | A | **98** | + | B | **154** | + | B | **210** | + | C |
| **43** | + | A | **99** | + | B | **155** | + | B | **211** | + | C |
| **44** | + | B | **100** | + | B | **156** | + | B | **212** | + | C |
| **45** | + | A | **101** | + | B | **157** | + | A | **213** | + | C |
| **46** | + | B | **102** | + | B | **158** | + | B | **214** | + | C |
| **47** | + | A | **103** | + | B | **159** | + | B | **215** | + | C |
| **48** | + | B | **104** | + | B | **160** | + | A | **216** | + | B |
| **49** | + | B | **105** | + | B | **161** | + | A | **217** | + | B |
| **50** | + | B | **106** | + | B | **162** | + | A | **218** | + | C |
| **51** | + | B | **107** | + | C | **163** | + | B | **BI853520** | >10000 | 6.49 |
| **52** | + | A | **108** | + | B | **164** | + | B | **Compound A** | >10000 | 10.45 |
| **53** | + | B | **109** | + | B | **165** | + | B | **Compound B** | >10000 | 6.85 |
| **54** | + | A | **110** | + | c | **166** | + | B | | | |
| **55** | + | B | **111** | + | B | **167** | + | B | | | |
| **56** | + | C | **112** | + | B | **168** | + | A | | | |

| | | | | | | | | | | | |
|---|---|---|---|---|---|---|---|---|---|---|---|
| A represents IC₅₀ < 5 nM B represents 5 nM ≤ IC₅₀ ≤ 50 nM C represents IC₅₀ > 50 nM + represents IC₅₀ > 2000 nM | | | | | | | | | | | |

As can be seen from the data in the table above, the compounds of the present invention had substantially no inhibitory activity against PC3 2D cells, but had very strong inhibitory activity against PC3 3D cells. The activity of most of the compounds was comparable to or significantly stronger than that of the control compound BI853520.

### Biological Example 3. Assay for Inhibition of PC3 Cell Low Adsorption by the Compounds of the Present Invention

PC3 cells were seeded at 8000 cells/well. 4 h after the cells were treated with a compound, the number of adherent cells was counted. The inhibition percentage and IC₅₀ of the compound were calculated compared to the DMSO group. The results are shown in Table 20 below.

**Table 20. The inhibitory activity of the compounds of the present invention against PC3 cell low adsorption (IC₅₀, nM)**

| **Compound** | **IC₅₀** | **Compound** | **IC₅₀** | **Compound** | **IC₅₀** | **Compound** | **IC₅₀** |
|---|---|---|---|---|---|---|---|
| **BI853520** | 10.78 | **69** | 13.36 | **89** | 36.2 | **139** | 5.20 |
| **Compound A** | 16.84 | **71** | 16.45 | **91** | 36.4 | **141** | 13.92 |
| **Compound B** | 12.50 | **73** | 5.01 | **92** | 62.0 | **154** | 6.90 |
| **1** | 21.1 | **77** | 27.24 | **114** | 2.85 | **177** | 1.86 |
| **3** | 3.14 | **78** | 5.83 | **117** | 4.60 | **178** | 6.22 |
| **4** | 6.87 | **79** | 1.09 | **120** | 1.46 | **196** | 1.60 |
| **6** | 2.67 | **82** | 6.29 | **121** | 7.2 | **197** | 1.67 |
| **8** | 7.54 | **87** | 8.77 | **136** | 1.16 | | |
| **27** | 11.7 | **88** | 42.4 | **137** | 1.71 | | |

As can be seen from the data in the table above, the inhibition of PC3 cell low adsorption by some of the compounds of the present invention was significantly better than by the control compound.

### Biological Example 4. Assay for Inhibition of Y397 Phosphorylation by the Compounds of the Present Invention

PC3 cells were seeded at 8000 cells/well. 3 h after the cells were treated with a compound, the phosphorylation level at the FAK 397 site in the cells was measured by ELISA using an antibody specifically recognizing phosphorylation at the FAK 397 site. The inhibition percentage and IC₅₀ of the compound were calculated compared to the DMSO group. The results are shown in Table 21 below.

**Table 21. The inhibitory activity of the compounds of the present invention against Y397 phosphorylation (IC₅₀, nM)**

| **Compound** | **IC₅₀** | **Compound** | **IC₅₀** | **Compound** | **IC₅₀** | **Compound** | **IC₅₀** |
|---|---|---|---|---|---|---|---|
| **BI853520** | 0.80 | **78** | <0.01 | **120** | <0.01 | **139** | 0.56 |
| **4** | 0.025 | **114** | 0.39 | **136** | 0.22 | **177** | 0.02 |
| **73** | 0.028 | **117** | 0.70 | **137** | 0.08 | **196** | 0.05 |

### Biological Example 5. In Vivo Pharmacokinetic Experiment of the Compounds of the Present Invention

CD-1 female mice aged 7 to 10 weeks were intravenously administered and orally administered at doses of 2 mg/kg and 10 mg/kg, respectively. The mice were fasted for at least 12 h before the administration and given food 4 h after the administration, and they were given *ad libitum* access to water during the experiment.

On the day of the experiment, animals in the intravenous group were given the corresponding compound by single injection via the tail vein at an administration volume of 10 mL/kg; and animals in the oral group were given the corresponding compound by single intragastric injection at an administration volume of 10 mL/kg. The animals were weighed before administration, and the administration volume was calculated according to the body weight. The sample collection time points were 0.083 h, 0.167 h, 0.5 h, 1 h, 2 h, 4 h, 8 h, and 24 h. About 200 µL of whole blood was collected through the orbital venous plexus at each time point and used to prepare plasma for concentration determination by high-performance liquid chromatography-tandem mass spectrometry (LC-MS/MS). The plasma concentrations were processed using a non-compartmental model of Winnolin pharmacokinetic software, and the pharmacokinetic parameters were calculated using a linear-log trapezoidal method.

**Table 22. The results of the in vivo pharmacokinetic evaluation of the compounds**

| **Route of administration** | **Pharmacokinetic parameters** | **Compound 3** | **Compound 4** | **Compound 79*** |
|---|---|---|---|---|
| Injection (1 mg/kg) | Vdss (L/Kg) | 6.14 | 4.35 | 5.14 |
| | T_{1/2} (h) | 2.14 | 1.78 | 3.48 |
| | Cl (mL/min/Kg) | 67.14 | 34.05 | 18.88 |
| | AUC₀₋ₗₐₛₜ (h*ng/mL) | 248.25 | 489.47 | 882.68 |
| Oral administration (10 mg/kg) | Cₘₐₓ (ng/mL) | 57.25 | 360.67 | 201.33 |
| | Tₘₐₓ (h) | 4 | 4 | 4 |
| | T_{1/2} (h) | -- | -- | 3.06 |
| | AUC₀₋ₗₐₛₜ (h*ng/mL) | 190.41 | 2087.58 | 1762.53 |
| | F% | 7.94% | 42.65% | 39.99% |

| **Route of administration** | **Pharmacokinetic parameters** | **Compound 73** | **Compound 136** | **Compound 137** |
|---|---|---|---|---|
| Injection (1 mg/kg) | Vdss (L/Kg) | 5.67 | 4.15 | 3.71 |
| | T_{1/2} (h) | 1.93 | 3.31 | 3.67 |
| | Cl (mL/min/Kg) | 40.24 | 15.0 | 12.0 |
| | AUC₀₋ₗₐₛₜ (h*ng/mL) | 414.21 | 1110.75 | 1388.44 |
| Oral administration (10 mg/kg) | Cₘₐₓ (ng/mL) | 624.33 | 906.67 | 810.33 |
| | Tₘₐₓ (h) | 0.5 | 2 | 4 |
| | T_{1/2} (h) | 3.32 | 2.20 | 4.19 |
| | AU₀₋ₗₐₛₜ (h*ng/mL) | 2892.24 | 8908.0 | 12411.35 |
| | F% | 59.3% | 80.20% | 89.39% |

| **Route of administration** | **Pharmacokinetic parameters** | **Compound 117** | **Compound 120** | **Compound 177** |
|---|---|---|---|---|
| Injection (1 mg/kg) | Vdss (L/Kg) | 3.47 | 6.15 | 7.51 |
| | T_{1/2} (h) | 1.81 | 4.69 | 5.89 |
| | Cl (mL/min/Kg) | 26.05 | 19.61 | 14.98 |
| | AUC₀₋ₗₐₛₜ (h*ng/mL) | 639.86 | 849.91 | 1112.59 |
| Oral administration (10 mg/kg) | Cₘₐₓ (ng/mL) | 822.0 | 522.33 | 815.0 |
| | Tₘₐₓ (h) | 2 | 4 | 2 |
| | T_{1/2} (h) | 2.22 | 5.26 | 3.37 |
| | AUC₀₋ₗₐₛₜ (h*ng/mL) | 7849.0 | 6120.40 | 7450.02 |
| | F% | 122.7% | 72.0% | 67.0% |

| | | | | |
|---|---|---|---|---|
| *Note: The oral group dosage of compound 79 was 5 mg/kg | | | | |

### Biological Example 6. Assay for In Vivo Efficacy of the Compounds of the Present Invention

Human pancreatic cancer Mia PaCa-2 cells were cultured conventionally in 1640 medium containing 10% fetal bovine serum in a 37 °C, 5% CO₂ incubator. After being subcultured, the cells were collected when they reached the desired amount. 1 × 10⁷ Mia PaCa-2 cells were injected into the left dorsal side of each nude mouse, and the animals were randomly grouped for administration after tumors grew to 100-200 mm³. The groups were as follows: 1) a solvent control group, 8 mice; and 2) a control compound group and a test compound group, 8 mice each. The mice in the solvent control group were intragastrically administered 0.5% CMC-Na twice a day; the mice in the compound groups were intragastrically administered 0.5% MC-Tween-80 suspension once a day. On Tuesday and Thursday each week, tumor volumes and body weight of the mice were measured, and the nude mice were sacrificed on day 21 after the administration. The ability of the compounds to inhibit tumor growth was evaluated according to the tumor growth inhibition rate (TGI) = 1 - (tumor volume on day 28 in treatment group - tumor volume on day 1 in treatment group)/(administration volume on day 28 in control group - tumor volume on day 1 in control group). The toxicity of the compounds was evaluated according to the body weight of the mice.

### Miapaca-2 in vivo experiment

The groups were as follows:
1) a solvent control group; 2) a control compound BI853520 group; 3) a control compound B group; 4) a compound 4 group; 5) a compound 79 group; 6) a compound 136 group; 7) a compound 137 group; 8) a compound 117 group; and 9) a compound 178 group. The results are shown in Table 23 below.

**Table 23. The in vivo efficacy of some of the compounds of the present invention in a MiaPaCa-2 model**

| **Group** | **Compound** | **Dose** | **Administration period** | **Route of administration** | **TGI(%)** | **Change in body weight** |
|---|---|---|---|---|---|---|
| **1** | Solvent control group | -- | QD (21 days) | PO | -- | +18.9% |
| **2** | BI853520 | 25mg/kg | QD(21 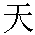 ) | PO | 73.51% | +6.48% |
| **3** | Compound B | 25mg/kg | QD(21 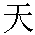 ) | PO | 75.24% | +5.05% |
| **4** | Compound 4 | 25mg/kg | QD(21 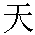 ) | PO | 69.02% | +4.69% |
| **5** | Compound 79 | 25mg/kg | QD(21 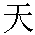 ) | PO | 89.35% | +3.72% |
| **6** | Compound 136 | 25mg/kg | QD(21 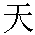 ) | PO | 89.46% | +0.76% |
| **7** | Compound 137 | 25mg/kg | QD(21 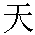 ) | PO | 87.68% | +6.08% |
| **8** | Compound 117 | 25mg/kg | QD(21 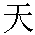 ) | PO | 62.86% | +6.28% |
| **9** | Compound 178 | 25mg/kg | QD(21 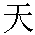 ) | PO | 82.41% | +5.41% |

As can be seen from the results of the *in vivo* experiment described above, the compounds of the present invention had significant inhibitory effects on tumor growth in the human pancreatic cancer MiaPaCa-2 model and also had significant advantages compared to the control compounds. Moreover, all compounds showed good toxicity tolerance.

### Biological Example 7. Assay for In Vivo Efficacy of the Compounds of the Present Invention

Human prostate cancer PC3 cells were cultured conventionally in 1640 medium containing 10% fetal bovine serum in a 37 °C, 5% CO₂ incubator. After being subcultured, the cells were collected when they reached the desired amount. 1 × 10⁷ PC3 cells were injected into the left dorsal side of each nude mouse, and the animals were randomly grouped for administration after tumors grew to 100-200 mm³. The groups were as follows: 1) a solvent control group, 8 mice; and 2) a control compound group and a test compound group, 8 mice each. The mice in the solvent control group were intragastrically administered 0.5% CMC-Na twice a day; the mice in the compound groups were intragastrically administered 0.5% MC-Tween-80 suspension once a day. On Tuesday and Thursday each week, tumor volumes and body weight of the mice were measured, and the nude mice were sacrificed on day 21 after the administration. The ability of the compounds to inhibit tumor growth was evaluated according to the tumor growth inhibition rate (TGI) = 1 - (tumor volume on day 28 in treatment group - tumor volume on day 1 in treatment group)/(administration volume on day 28 in control group - tumor volume on day 1 in control group). The toxicity of the compounds was evaluated according to the body weight of the mice.

### PC3 in vivo experiment

The groups were as follows:
1) a solvent control group; 2) a control compound BI853520 group; 3) a compound 4 group; 4) a compound 73 group; 5) a compound 79 group; 6) a compound 120 group; 7) a compound 136 group; and 8) a compound 137 group. The results are shown in Table 24 below.

**Table 24. The in vivo efficacy of some of the compounds of the present invention in a PC-3 model**

| **Group** | **Compound** | **Dose** | **Administration period** | **Route of administration** | **TGI(%)** | **Change in body weight** |
|---|---|---|---|---|---|---|
| **1** | Solvent control group | -- | QD (21 days) | PO | | -0.2% |
| **2** | BI853520 | 25mg/kg | QD (21 days) | PO | 74.48% | -2.3% |
| **3** | Compound 4 | 25mg/kg | QD (21 days) | PO | 61.48% | -2.0% |
| **4** | Compound 73 | 25mg/kg | QD (21 days) | PO | 74.07% | -1.4% |
| **5** | Compound 79 | 25mg/kg | QD (21 days) | PO | 88.33% | -2.9% |
| **6** | Compound 120 | 25mg/kg | QD (21 days) | PO | 90.83% | -6.3% |
| **7** | Compound 136 | 25mg/kg | QD (21 days) | PO | 76.67% | -6.6% |
| **8** | Compound 137 | 25mg/kg | QD (21 days) | PO | 76.39% | -3.7% |

As can be seen from the results of the *in vivo* experiment described above, the compounds of the present invention had significant inhibitory effects on tumor growth in the human prostate cancer PC-3 model and also had significant advantages compared to the control compound. Moreover, all compounds showed good toxicity tolerance.

### Biological Example 8. Assay for In Vivo Efficacy of the Compounds of the Present Invention

Female BALB/c mice (aged 6 weeks, 18-22 g) were provided by Vital River Laboratory Animal Technology Co., Ltd. (China) and used one week after quarantine and acclimation. All animals were kept in a room at 23±2 °C with a relative humidity of 50±5%, artificial lighting was provided from 08:00 to 20:00 every day, and the air was exchanged 13-18 times per hour. They had *ad libitum* access to standard laboratory food and water.

Mouse colon cancer MC38 cells were cultured conventionally in 1640 medium containing 10% fetal bovine serum in a 37 °C, 5% CO₂ incubator. After being subcultured, the cells were collected when they reached the desired amount. The BALB/c mice were subcutaneously injected with 1 × 10⁶ MC38 cells on the right side to form tumors. After the tumors grew to about 100 mm³, the animals were randomly divided into a solvent control group, a test compound single drug group, a test compound + PD-1 combination group, and a PD-1 single drug group (Bio xCELL). Administration was then started. Tumor volumes were measured with a caliper on day 3, day 7, day 10, day 14, day 17, and day 21 after the administration.

The ability of the compounds to inhibit tumor growth was evaluated according to the tumor growth inhibition rate (TGI) = 1 - (tumor volume on day 28 in treatment group - tumor volume on day 1 in treatment group)/(administration volume on day 28 in control group - tumor volume on day 1 in control group). The toxicity of the compounds was evaluated according to the body weight and state of the mice.

### MC38 in vivo experiment

The groups were as follows:
1) a solvent control group; 2) a control compound BI853520 group; 3) a PD-1 monoclonal antibody group; 4) a compound 79 group; 5) a compound 136 group; 6) a compound 120 group; 7) a compound 79 group + a PD-1 monoclonal antibody group; 8) a compound 136 group + a PD-1 monoclonal antibody group; 9) a compound 120 group + a PD-1 monoclonal antibody group; and 10) a control compound BI853520 + PD-1 monoclonal antibody group. The results are shown in Table 25 below.

**Table 25. The in vivo efficacy of some of the compounds of the present invention in an MC-38 model**

| **Group** | **Compound** | **Dose** | **Administration period** | **Route of administration** | **TGI(%)** | **Change in body weight** |
|---|---|---|---|---|---|---|
| **1** | Solvent control group | -- | QD (21 days) | PO | -- | +30.81% |
| **2** | BI853520 | 50mg/kg | QD (21 days) | PO | 52.64% | +18.74% |
| **3** | PD-1 monoclonal antibody | 1.5mg/kg | QW (21 days) | IV | 65.49% | +20.53% |
| **4** | Compound 79 | 50mg/kg | QD(21 days) | PO | 69.21% | +14.99% |
| **5** | Compound 136 | 50mg/kg | QD(21 days) | PO | 32.53% | +6.55% |
| **6** | Compound 120 | 50mg/kg | OD(21 davs) | PO | 66.33% | +13.2% |
| **7** | Compound 79 + PD-1 monoclonal antibody | 50mg/kg +1.5mg | QD(21 days) +QW(21 days) | PO +IV | 80.55% | +9.03% |
| **8** | Compound 136 +PD-1 monoclonal antibody | 50mg/kg +1.5mg | QD(21 days) +QW(21 days) | PO +IV | 88.89% | +6.12% |
| **9** | Compound 120 + PD-1 monoclonal antibody | 50mg/kg +1.5mg | QD(21 days) +QW(21 days) | PO +IV | 83.94% | +17.06% |
| **10** | BI853520 + PD-1 monoclonal antibody | 50mg/kg +1.5mg | QD(21 days) +QW(21 days) | PO +IV | 70.50% | +18.66% |

As can be seen from the results of the *in vivo* experiment described above, the combinations of the compounds of the present invention and the PD-1 monoclonal antibody had relatively good inhibitory effects on tumor growth in the mouse colon cancer MC-38 model, while the control compound has substantially no combination effect on the model. Moreover, all compounds showed good toxicity tolerance.

Although specific embodiments of the present invention have been described above, it will be appreciated by those skilled in the art that these embodiments are merely illustrative and that many changes or modifications can be made to these embodiments without departing from the principles and spirit of the present invention. The protection scope of the present invention is therefore defined by the appended claims.

## Claims

1. A compound of general formula (1) or an isomer thereof, a crystalline form thereof, a pharmaceutically acceptable salt thereof, a hydrate thereof, or a solvate thereof: 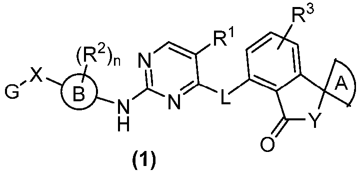 wherein in general formula (1):
ring A is (C3-C6) cycloalkyl or (3-6 membered) heterocycloalkyl, wherein the (C3-C6) cycloalkyl or (3-6 membered) heterocycloalkyl may be each independently and optionally substituted with 1, 2, 3, or 4 R^{a};
Y is -O-, -NR^{a}-, or -CR^{a}R^{b}-;
L is -CH₂-, -O-, or -S-;
ring B is selected from (3-18 membered) heterocycloalkyl, (C3-C18) cycloalkyl, (C6-C18) aryl, or (5-18 membered) heteroaryl, wherein the (3-18 membered) heterocycloalkyl, (C3-C18) cycloalkyl, (C6-C18) aryl, or (5-18 membered) heteroaryl may be each independently and optionally substituted with n R²;
X is a chemical bond, 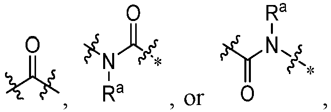 with * representing attachment to ring B;
G is -H, -D, halogen, hydroxy, amino, cyano, nitro, -OR^{a}, -NR^{a}R^{b}, -C(O)R^{a}, -CO₂R^{a}, -CONR^{a}R^{b}, (C1-C8) alkyl, (C1-C8) alkoxy, (C1-C8) haloalkyl, (C2-C8) alkenyl, (C2-C8) alkynyl, (C3-C15) cycloalkyl, (3-15 membered) heterocycloalkyl, (C6-C14) aryl, or (5-14 membered) heteroaryl, wherein the (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, or (5-14 membered) heteroaryl may be each independently and optionally substituted with 1, 2, 3, or 4 Rc;
R¹ is -H, -D, halogen, hydroxy, amino, cyano, nitro, -OR^{a}, -NR^{a}R^{b}, -C(O)R^{a}, -CO₂R^{a}, -CONR^{a}R^{b}, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, or (5-14 membered) heteroaryl, wherein the (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, or (5-14 membered) heteroaryl may be each independently and optionally substituted with 1, 2, 3, or 4 R^{c};
R² is -H, -D, halogen, hydroxy, amino, cyano, nitro, -OR^{a}, -NR^{a}R^{b}, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, or (5-14 membered) heteroaryl, wherein the (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, or (5-14 membered) heteroaryl may be each independently and optionally substituted with 1, 2, 3, or 4 R^{c}; or when 2 R² are attached to the same atom, the 2 R² may form one oxo group;
R³ is -H, -D, halogen, hydroxy, amino, cyano, nitro, -OR3^{a}, -NR^{3a}R^{3b}, -C(O)R^{3a}, -CO₂R^{3a}, - S(O)ₚR^{3a}, -S(O)ₚNR^{3a}R^{3b}, -CONR^{3a}R^{3b}, -C(=NR^{3a})-NR^{3b}R^{3c}, -NR^{3a}COR^{3b}, -NR^{3a}CONR^{3b}R^{3c}, - NR^{3a}CO₂R^{3b}, -NR^{3a}S(O)ₚNR^{3b}R^{3c}, -NR^{3a}S(O)ₚR^{3b}, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C1-C8) alkoxy, -(C1-C8) alkylene-(C3-C14) cycloalkyl, -(C1-C8) alkylene-(3-14 membered) heterocycloalkyl, -(C1-C8) alkylene-(C6-C14) aryl, or -(C1-C8) alkylene-(5-14 membered) heteroaryl;
R^{a} and R^{b} are each independently -H, -D, halogen, hydroxy, amino, cyano, nitro, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C3-C14) cycloalkyl, -(C1-C8) alkylene-(3-14 membered) heterocycloalkyl, -(C1-C8) alkylene-(C6-C14) aryl, or -(C1-C8) alkylene-(5-14 membered) heteroaryl, wherein the (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C3-C14) cycloalkyl, -(C1-C8) alkylene-(3-14 membered) heterocycloalkyl, -(C1-C8) alkylene-(C6-C14) aryl, or -(C1-C8) alkylene-(5-14 membered) heteroaryl may be each independently and optionally substituted with 1, 2, 3, or 4 R^{c}; or R^{a} and R^{b}, together with the atom to which they are attached, form one (C3-C6) cycloalkyl or (3-6 membered) heterocycloalkyl group, and the (C3-C6) cycloalkyl or (3-6 membered) heterocycloalkyl group may be each independently and optionally substituted with 1, 2, 3, or 4 R^{c};
R^{c} is -H, -D, halogen, hydroxy, amino, cyano, nitro, -OR^{xa}, -NR^{xa}R^{xb}, -(CH₂)ₘOR^{xa}, - (CH₂)ₘNR^{xa}R^{xb}, -C(O)R^{xa}, -CO₂R^{xa}, -(CH₂)ₘS (O)ₚR^{xa}, -S(O)ₚNR^{xa}R^{xb}, -(CH₂)ₘCONR^{xa}R^{xb}, - C(=NR^{xa})-NR^{xb}R^{xc}, -NR^{xa}COR^{xb}, -NR^{xa}CONR^{xb}R^{xc}, -NR^{xa}CO₂R^{xb}, -NR^{xa}S(O)ₚNR^{xb}R^{xc}, - NR^{xa}S(O)ₚR^{xb}, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C1-C8) alkoxy, -(C1-C8) alkylene-(C3-C14)cycloalkyl, -(C1-C8) alkylene-(3-14 membered) heterocycloalkyl, -(C1-C8) alkylene-(C6-C14) aryl, or -(C1-C8) alkylene-(5-14 membered) heteroaryl, wherein the (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, or (5-14 membered) heteroaryl may be each independently and optionally substituted with 1, 2, 3, or 4 R^{3c}; or when 2 R^{c} are attached to the same atom, the 2 R^{c} may form one oxo group;
R^{xa}, R^{xb}, and R^{xc} are each independently -H, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C3-C14) cycloalkyl, -(C1-C8) alkylene-(3-14 membered) heterocycloalkyl, -(C1-C8) alkylene-(C6-C14) aryl, or -(C1-C8) alkylene-(5-14 membered) heteroaryl;
R^{3a}, R^{3b}, and R^{3c} are each independently -H, -D, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C3-C14) cycloalkyl, -(C1-C8) alkylene-(3-14 membered) heterocycloalkyl, -(C1-C8) alkylene-(C6-C14) aryl, or -(C1-C8) alkylene-(5-14 membered) heteroaryl;
p is an integer of 0, 1, or 2;
m is an integer of 0, 1, 2, or 3;
n is an integer of 0, 1, 2, or 3.

2. The compound or the isomer thereof, the crystalline form thereof, the pharmaceutically acceptable salt thereof, the hydrate thereof, or the solvate thereof according to claim 1, wherein in the general formula (1), L is -CH₂- or -O-.

3. The compound or the isomer thereof, the crystalline form thereof, the pharmaceutically acceptable salt thereof, the hydrate thereof, or the solvate thereof according to claim 1 or 2, wherein in the general formula (1), ring A is (C3-C6) cycloalkyl or (3-6 membered) heterocycloalkyl, wherein the (C3-C6) cycloalkyl or (3-6 membered) heterocycloalkyl may be each independently and optionally substituted with 1, 2, 3, or 4 -H, -D, -F, -Cl, -Br, -I, -OH, - NH₂, -CN, -NO₂, -OCH₃, -OCH₂CH₃, -OCF₃, -CH₃, -CD₃, -CH₂CH₃, -CH₂F, -CHF₂, -CF₃, - (CH₂)₂OCH₃, -CH₂SO₂CH₃, -CH₂CONH₂, -CH₂CON(CH₃)₂,

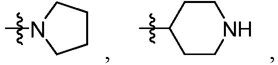

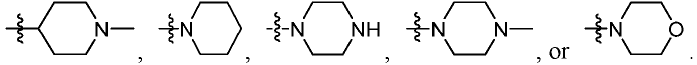

4. The compound or the isomer thereof, the crystalline form thereof, the pharmaceutically acceptable salt thereof, the hydrate thereof, or the solvate thereof according to claim 3, wherein in the general formula (1), ring A is 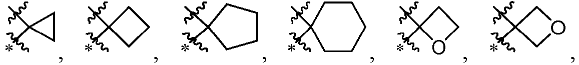 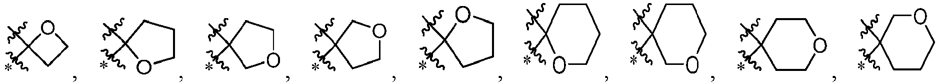 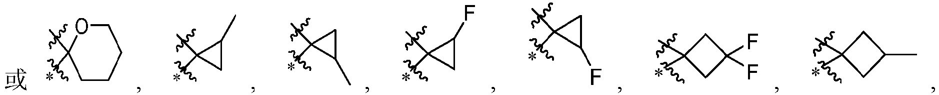 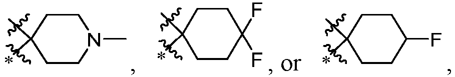 wherein * represents attachment to Y

5. The compound or the isomer thereof, the crystalline form thereof, the pharmaceutically acceptable salt thereof, the hydrate thereof, or the solvate thereof according to any one of claims 1-4, wherein in the general formula (1), ring B is selected from (C6-C12) aryl or (5-14 membered) heteroaryl, wherein the (C6-C12) aryl or (5-14 membered) heteroaryl may be optionally substituted with n R².

6. The compound or the isomer thereof, the crystalline form thereof, the pharmaceutically acceptable salt thereof, the hydrate thereof, or the solvate thereof according to claim 5, wherein in the general formula (1), ring B is 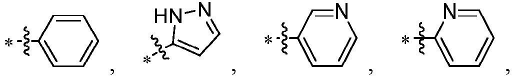 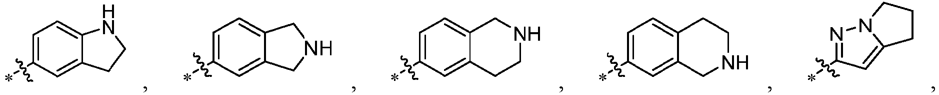 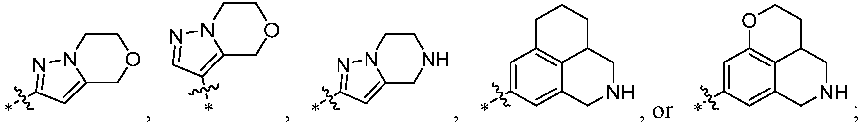 ring B is preferably 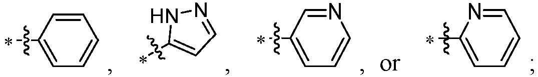 ring B is more preferably 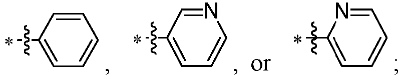 ring B is more preferably 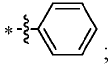 wherein "*" represents attachment to

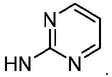

7. The compound or the isomer thereof, the crystalline form thereof, the pharmaceutically acceptable salt thereof, the hydrate thereof, or the solvate thereof according to any one of claims 1-6, wherein in general formula (1), G is -H, -D, (C1-C6) alkyl, (C1-C6) alkoxy, (C1-C6) haloalkyl, or (5-15 membered) heterocycloalkyl, wherein the (C1-C6) alkyl, (C1-C6) alkoxy, (C1-C6) haloalkyl, or (5-15 membered) heterocycloalkyl may be optionally substituted with 1, 2, 3, or 4 -H, -D, -F, -Cl, -Br, -I, -OH, -NH₂, -CN, -NO₂, -OCH₃, -NHCH₃, -N(CH₃)₂, -C(O)NH₂, -OCH₂CH₃, -OCF₃, -CH₃, -CD₃, -CH₂CH₃, -CH₂F, -CHF₂, -CF₃, -(CH₂)₂OCH₃, -CH₂SO₂CH₃, - 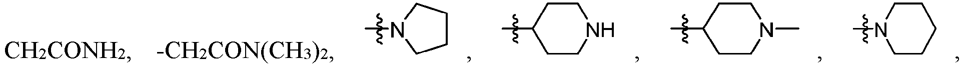 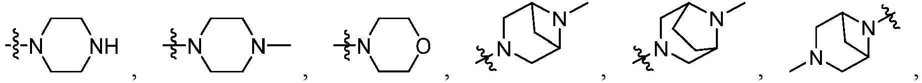 or 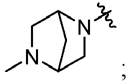 or two substituents attached to the same atom may form one oxo group.

8. The compound or the isomer thereof, the crystalline form thereof, the pharmaceutically acceptable salt thereof, the hydrate thereof, or the solvate thereof according to claim 7, wherein in the general formula (1), G is -H, (C1-C3) alkyl, or (6-12 membered) heterocycloalkyl, wherein the (C1-C3) alkyl or (6-12 membered) heterocycloalkyl may be optionally substituted with 1, 2, 3, or 4 -H, -D, -F, -CH₃, -CD₃, -CH₂CH₃, -OCH₃, -N(CH₃)₂, -(CH₂)₂OCH₃, -CH₂SO₂CH₃, - CH₂CONH₂, -CH₂CON(CH₃)₂, 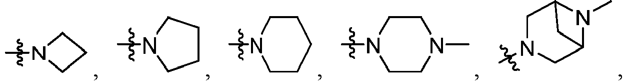 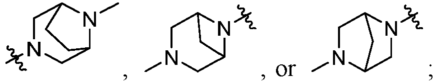 or two substituents attached to the same atom may form one oxo group.

9. The compound or the isomer thereof, the crystalline form thereof, the pharmaceutically acceptable salt thereof, the hydrate thereof, or the solvate thereof according to claim 8, wherein in the general formula (1), G is selected from -H, -CH₃, -CD₃, -CH₂CH₃, -CH₂F, -CHF₂, -CF₃, - CH₂N(CH₃)₂,

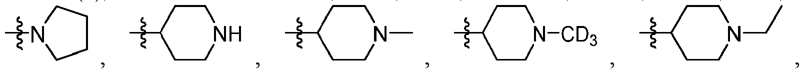

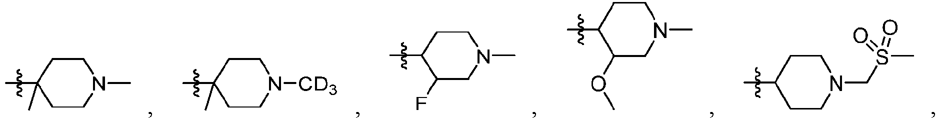

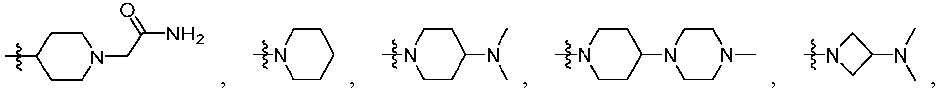

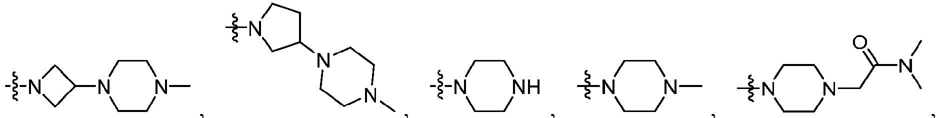

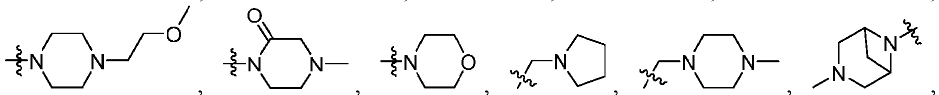

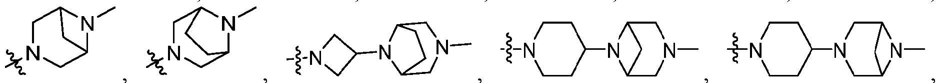

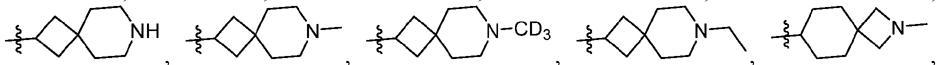

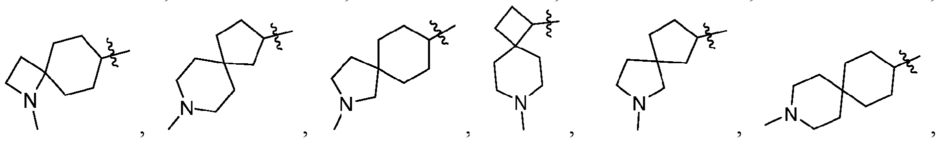

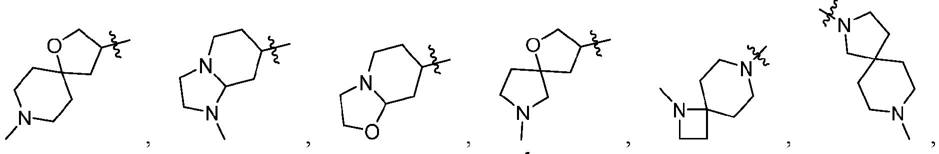

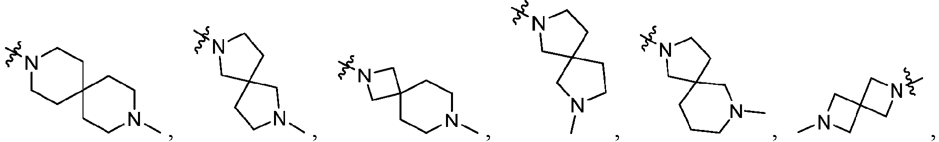

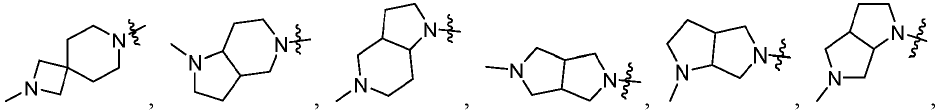

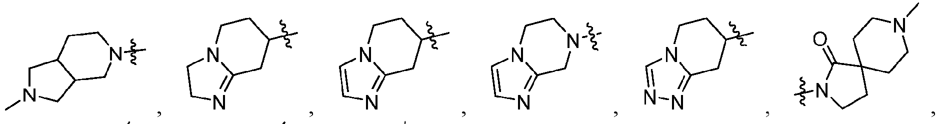

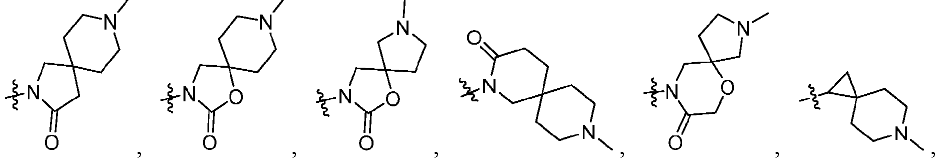

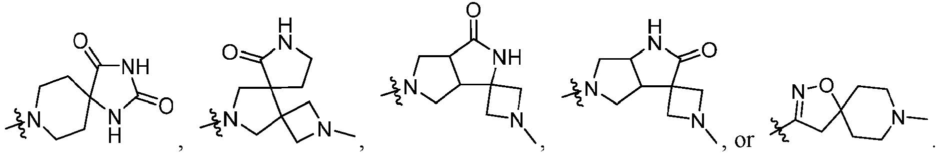

10. The compound or the isomer thereof, the crystalline form thereof, the pharmaceutically acceptable salt thereof, the hydrate thereof, or the solvate thereof according to any one of claims 1-9, wherein in the general formula (1), X is selected from a chemical bond, 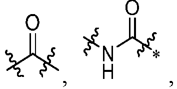 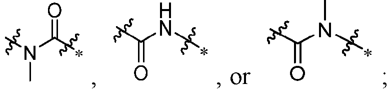 X is preferably a chemical bond, 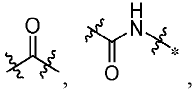 or 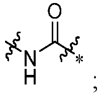 X is more preferably a chemical bond, 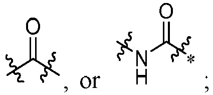 X is more preferably 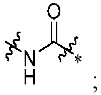 X is more preferably 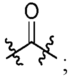 X is more preferably a chemical bond; wherein * represents attachment to ring B.

11. The compound or the isomer thereof, the crystalline form thereof, the pharmaceutically acceptable salt thereof, the hydrate thereof, or the solvate thereof according to any one of claims 1-10, wherein in the general formula (1), R¹ is selected from -H, -D, -F, -Cl, -Br, -I, -OH, -NH₂, -CN, -NO₂, -OCH₃, -NHCH₃, -N(CH₃)₂, -C(O)NH₂, -OCH₂CH₃, -OCF₃, -CH₃, -CD₃, -CH₂CH₃, -CH₂F, -CHF₂, or -CF₃; R¹ is preferably -F, -Cl, -Br, -CN, -NO₂, -CF₃, or -C(O)NH₂; R¹ is preferably -F, -Cl, -Br, -CN, or -CF₃; R¹ is more preferably -F; R¹ is more preferably -Cl; R¹ is more preferably -Br; R¹ is more preferably -CN; R¹ is more preferably -CF₃.

12. The compound or the isomer thereof, the crystalline form thereof, the pharmaceutically acceptable salt thereof, the hydrate thereof, or the solvate thereof according to any one of claims 1-11, wherein in the general formula (1), R² is -H, -D, -F, -Cl, -Br, -I, -OH, -NH₂, -CN, -NO₂, - OCF₃, -NHCH₃, -N(CH₃)₂, -NHCH₂CH₃, -N(CH₂CH₃)₂, (C1-C3) alkyl, (C1-C3) alkoxy, (C1-C3) haloalkyl, (C2-C4) alkenyl, (C2-C4) alkynyl, (C3-C6) cycloalkyl, (3-6 membered) heterocycloalkyl, (C6-C10) aryl, or (5-10 membered) heteroaryl, wherein the (C1-C3) alkyl, (C1-C3) alkoxy, (C1-C3) haloalkyl, (C2-C4) alkenyl, (C2-C4) alkynyl, (C3-C6) cycloalkyl, (3-6 membered) heterocycloalkyl, (C6-C10) aryl, or (5-10 membered) heteroaryl may be each independently and optionally substituted with 1, 2, 3, or 4 -H, -D, -F, -Cl, -Br, -I, -OH, -NH₂, - CN, -NO₂, -OCH₃, -NHCH₃, -N(CH₃)₂, -C(O)NH₂, -OCH₂CH₃, -OCF₃, -CH₃, -CD₃, -CH₂CH₃, -CH₂F, -CHF₂, or -CF₃.

13. The compound or the isomer thereof, the crystalline form thereof, the pharmaceutically acceptable salt thereof, the hydrate thereof, or the solvate thereof according to claim 12, wherein in the general formula (1), R² is selected from -H, -D, -F, -Cl, -Br, -I, -OH, -NH₂, -CN, -NO₂, - OCH₃, -NHCH₃, -N(CH₃)₂, -C(O)NH₂, -OCH₂CH₃, -OCF₃, -CH₃, -CD₃, -CH₂CH₃, -CH(CH₃)₂, -CH₂F, -CHF₂, -CF₃, 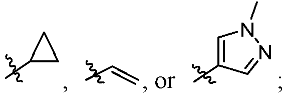 R² is preferably -H, -D, -F, -Cl, -OH, -OCH₃, -OCF₃, -CH₃, -CD₃, -CH₂CH₃, -CH(CH₃)₂, -CF₃, 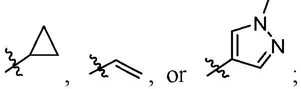 R² is more preferably -H, -F, -Cl, -OH, -OCH₃, -OCF₃, -CH₂CH₃, -CH(CH₃)₂,

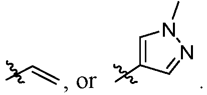

14. The compound or the isomer thereof, the crystalline form thereof, the pharmaceutically acceptable salt thereof, the hydrate thereof, or the solvate thereof according to any one of claims 1-13, wherein in the general formula (1), R³ is selected from -H, -D, -F, -Cl, -Br, -I, -OH, -NH₂, -CN, -NO₂, -OCH₃, -NHCH₃, -N(CH₃)₂, -C(O)NH₂, -OCH₂CH₃, -OCF₃, -CH₃, -CD₃, -CH₂CH₃, -CH₂F, -CHF₂, or -CF₃; R³ is preferably -H, -D, -F, -CH₃, -CD₃, or -CF₃; R³ is more preferably - H, -D, or -F; R³ is more preferably -H; R³ is more preferably -D; R³ is more preferably -F.

15. The compound or the isomer thereof, the crystalline form thereof, the pharmaceutically acceptable salt thereof, the hydrate thereof, or the solvate thereof according to any one of claims 1-14, wherein the compound has one of the following structures:

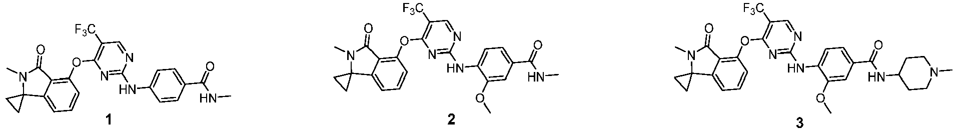

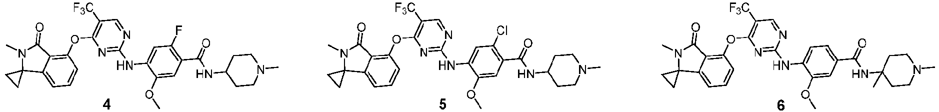

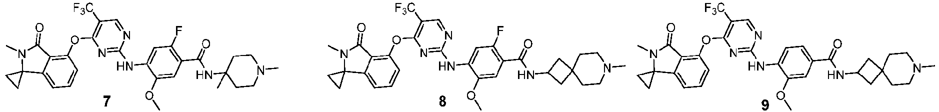

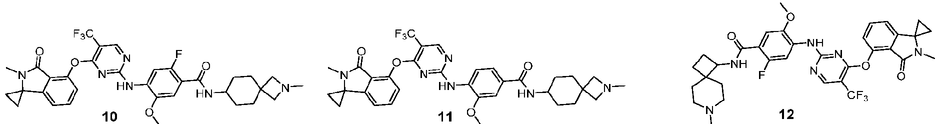

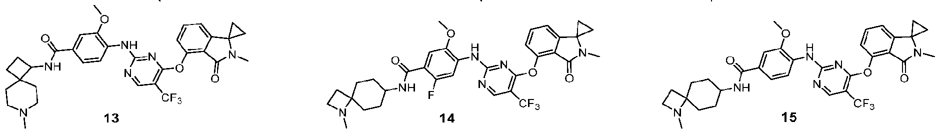

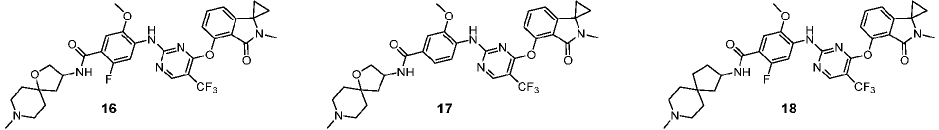

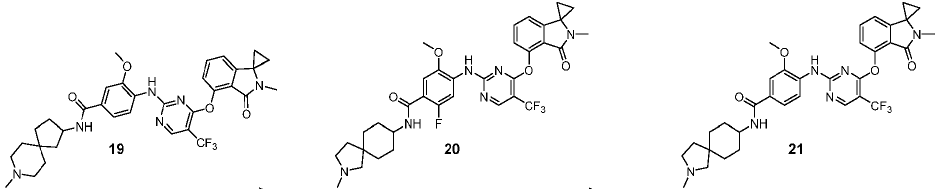

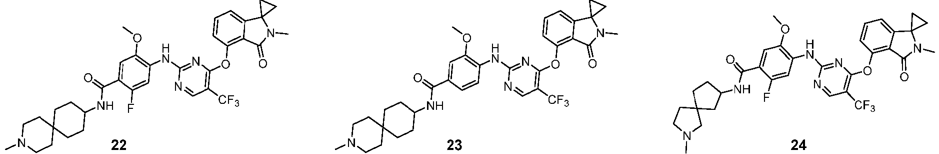

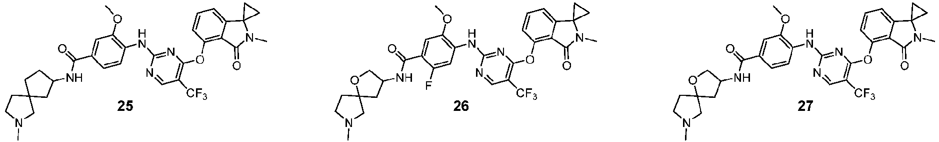

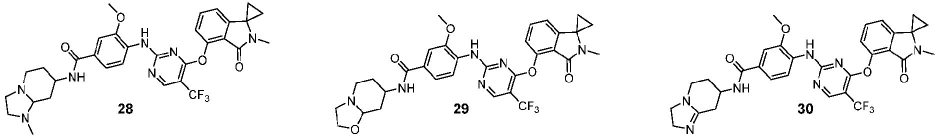

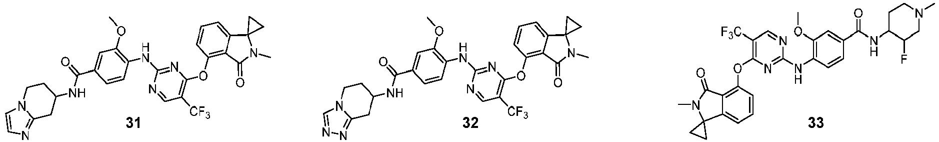

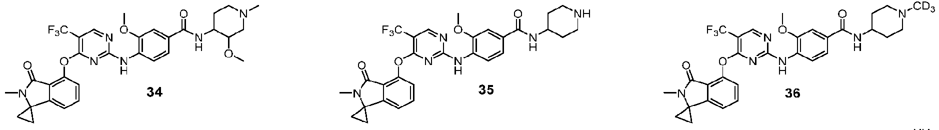

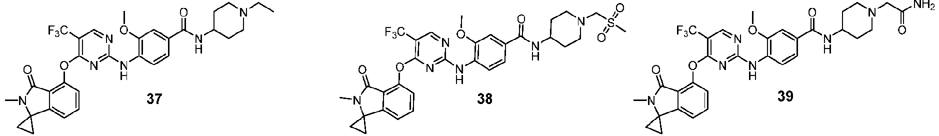

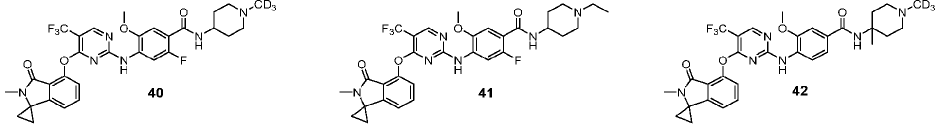

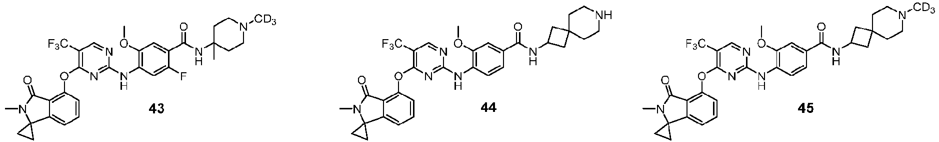

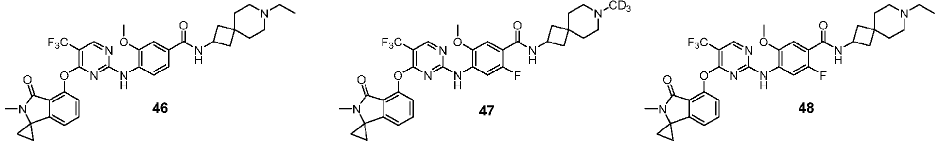

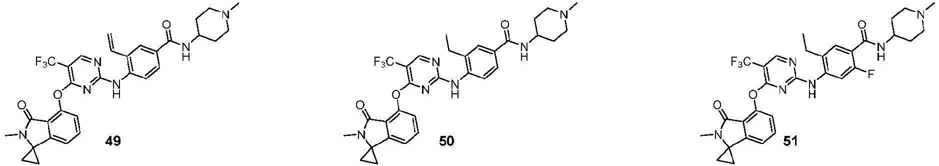

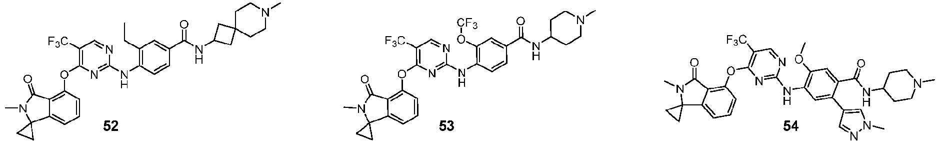

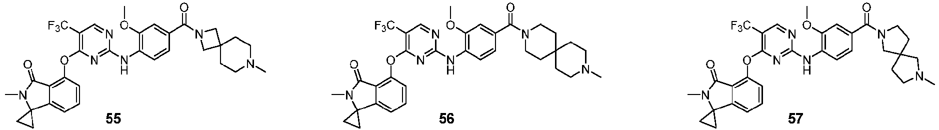

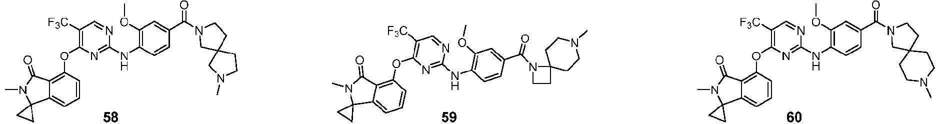

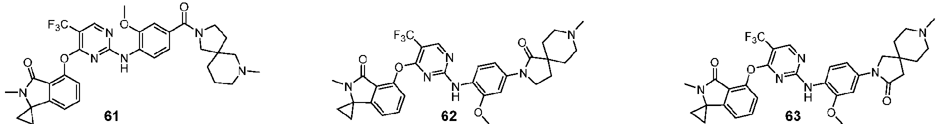

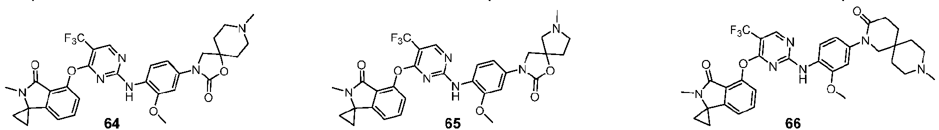

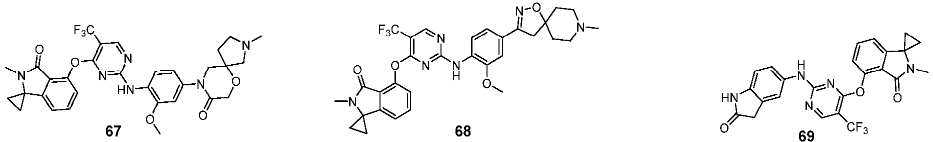

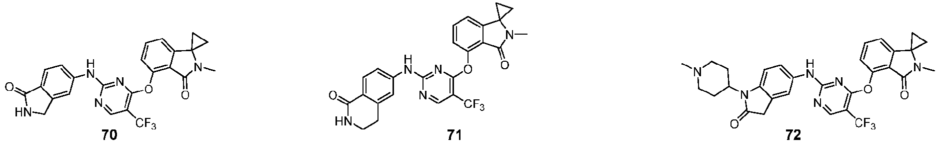

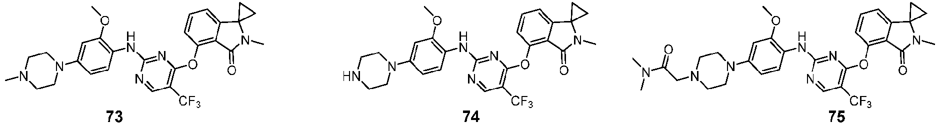

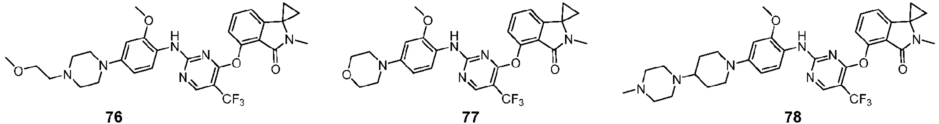

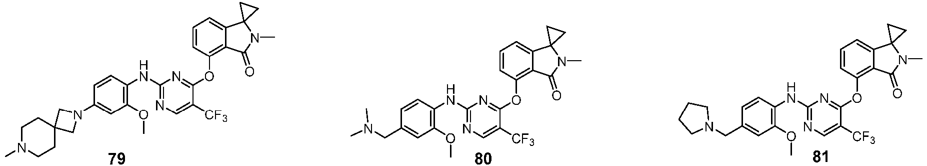

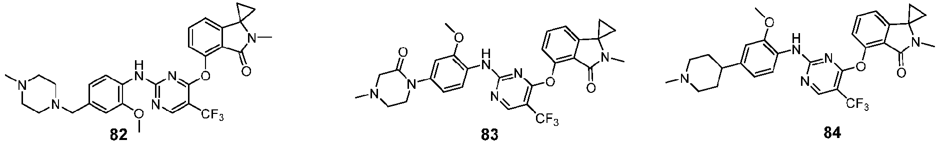

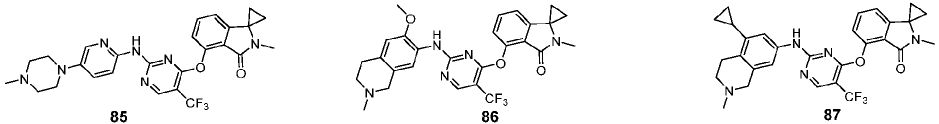

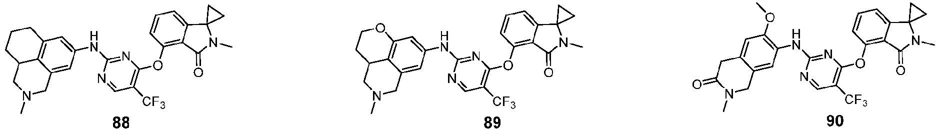

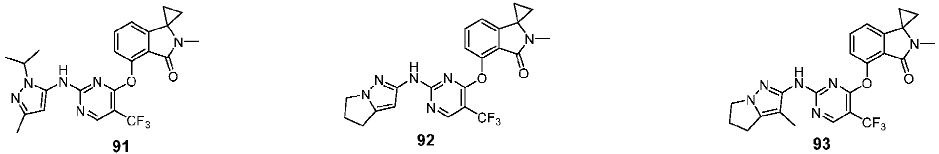

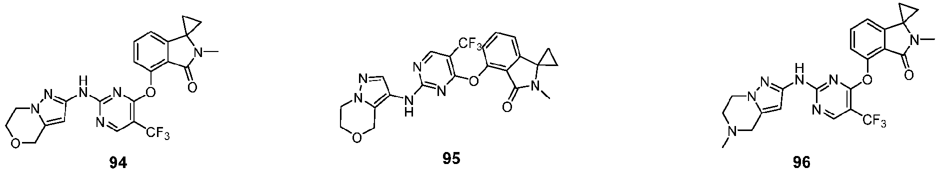

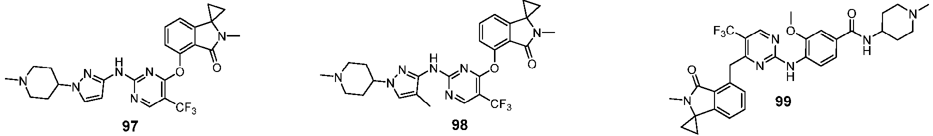

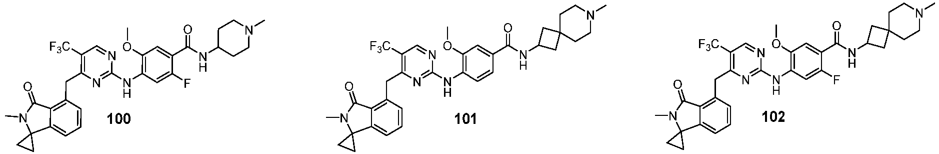

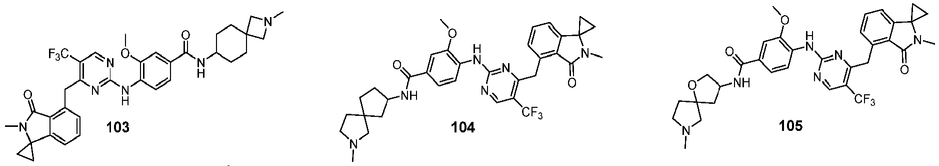

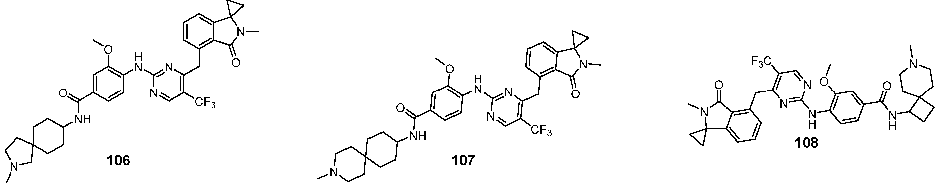

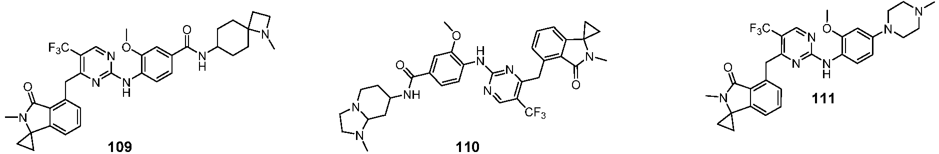

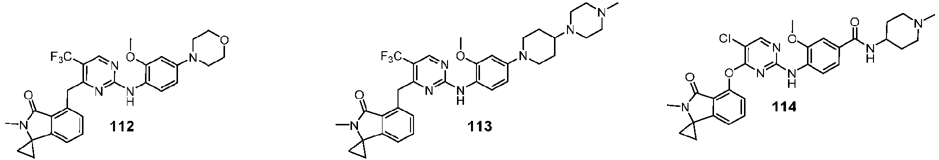

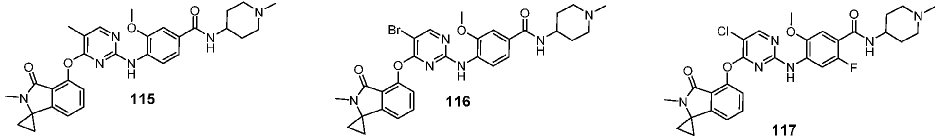

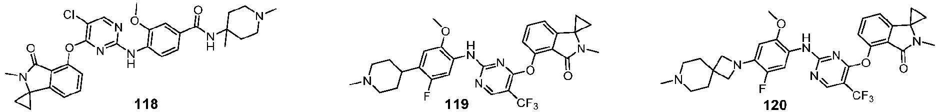

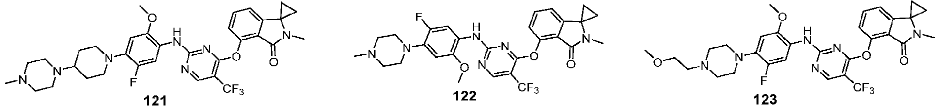

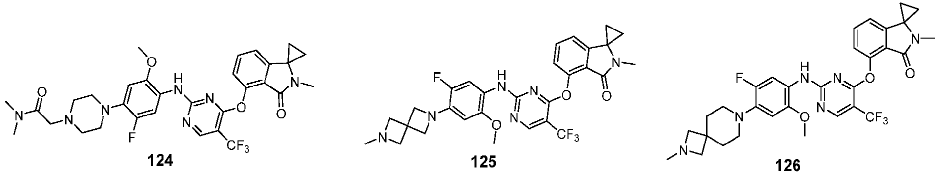

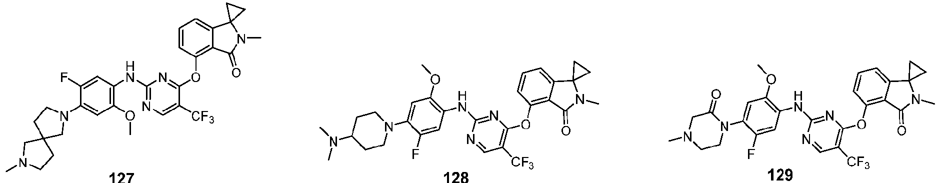

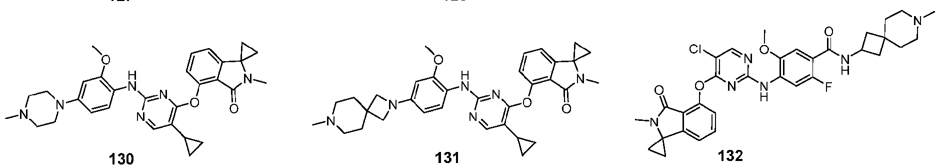

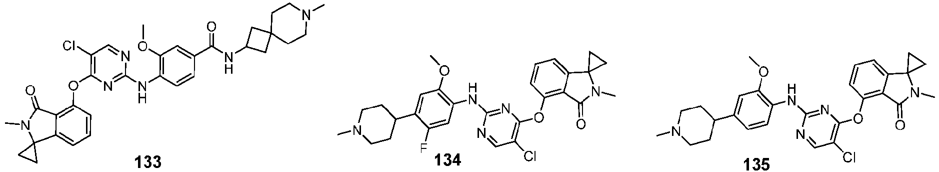

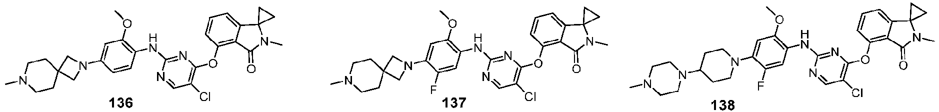

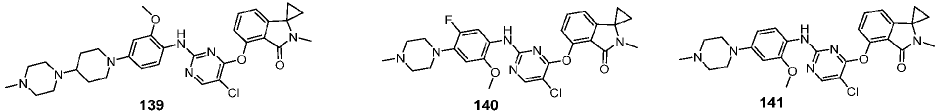

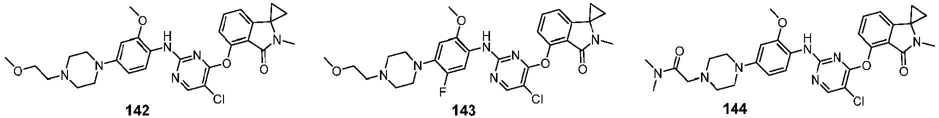

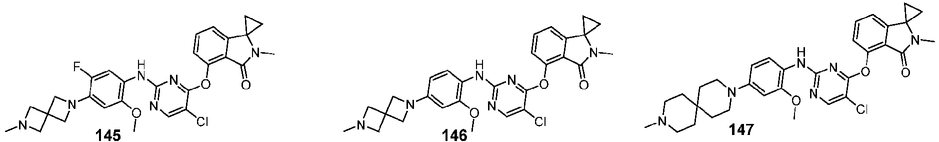

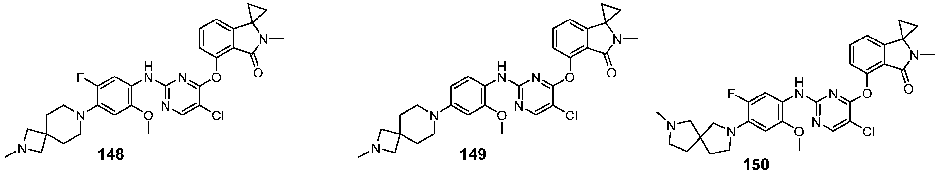

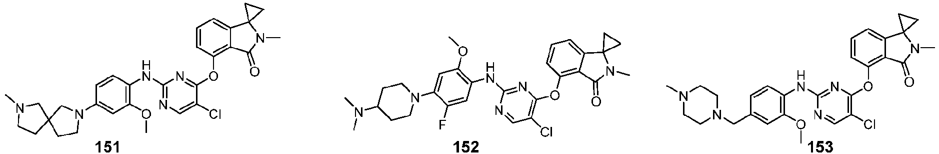

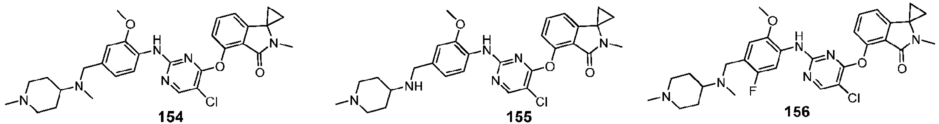

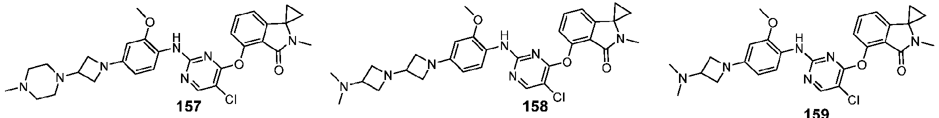

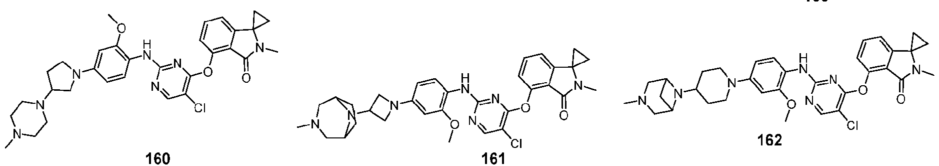

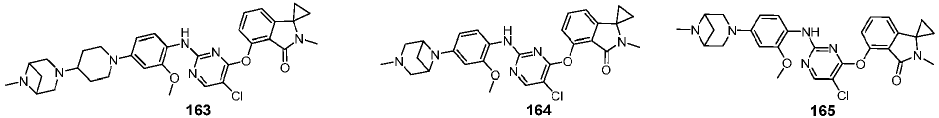

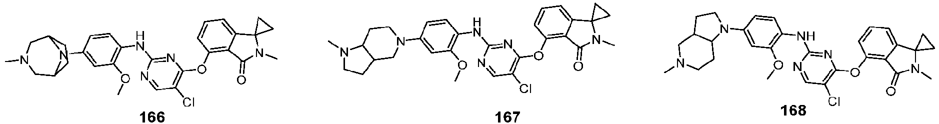

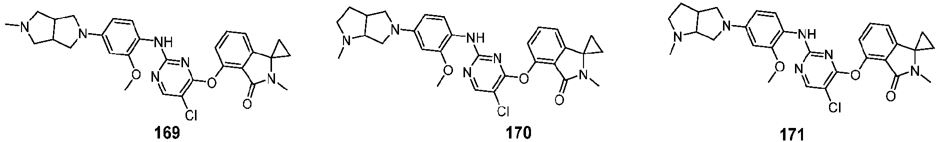

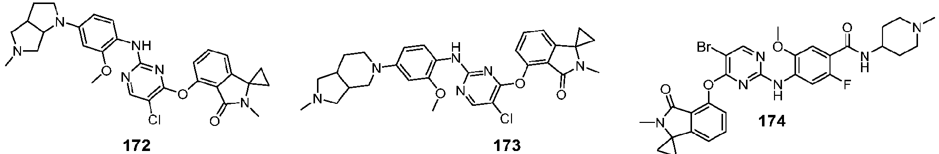

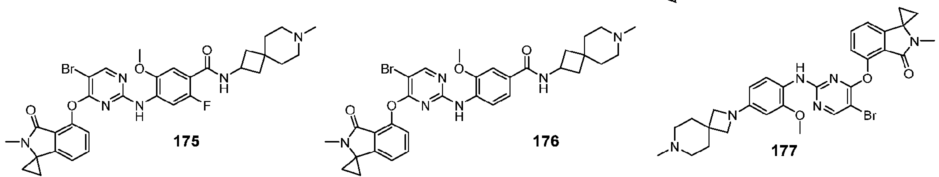

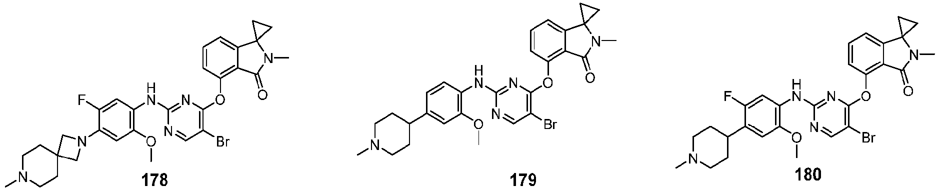

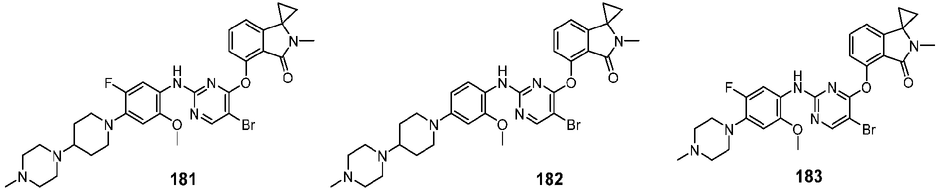

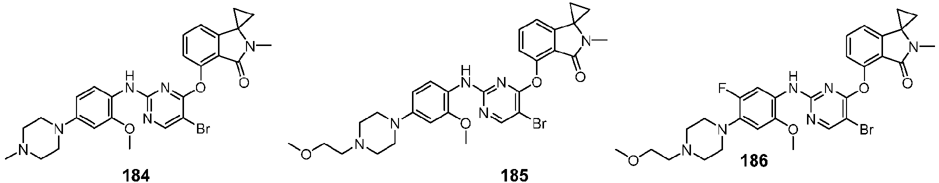

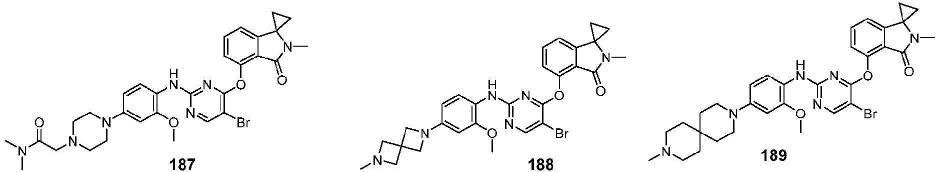

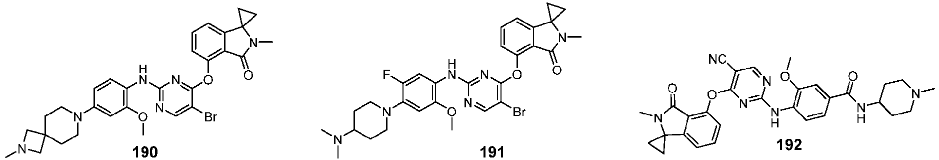

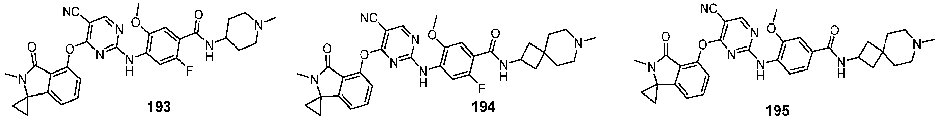

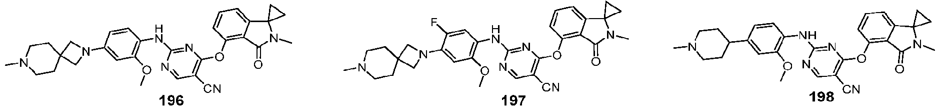

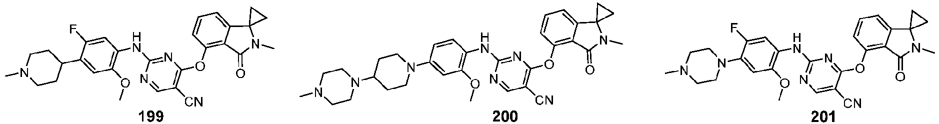

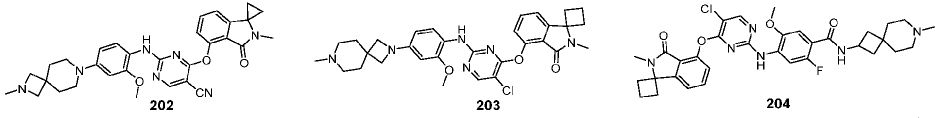

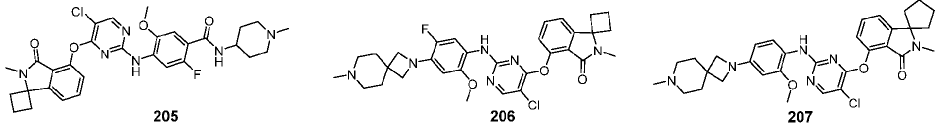

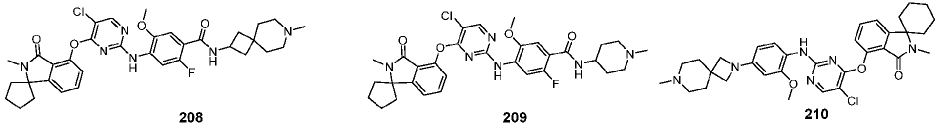

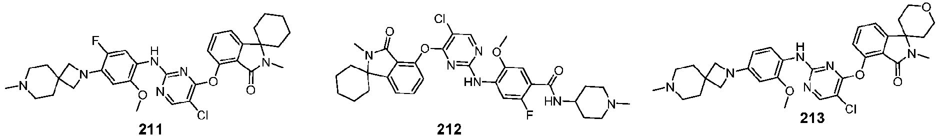

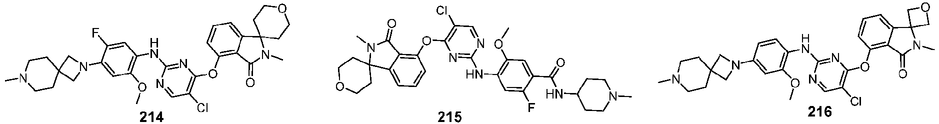

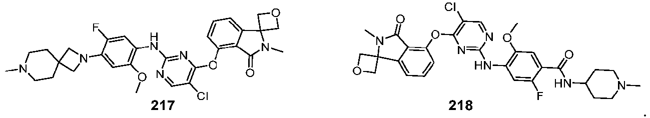

16. A pharmaceutical composition, comprising a pharmaceutically acceptable excipient or carrier, and the compound or the isomer thereof, the crystalline form thereof, the pharmaceutically acceptable salt thereof, the hydrate thereof, or the solvate thereof according to any one of claims 1-15 as an active ingredient.

17. Use of the compound or the isomer thereof, the crystalline form thereof, the pharmaceutically acceptable salt thereof, the hydrate thereof, or the solvate thereof according to any one of claims 1-15 or the pharmaceutical composition according to claim 16 in preparation of a medicament for treating a disease related to FAK kinase.
